# EUROPEAN PATENT APPLICATION

(11) **EP 3 928 781 A2**
(43) Date of publication of application: **29.12.2021**
(21) Application number: 19889969.2
(22) Date of filing: 25.11.2019
(51) Int. Cl.: A61K 35/00, A61K 36/00

(54) **SUBSTANCE FOR TREATING AND/OR PREVENTING INTOLERANCE DISEASES, AND DESIGN METHOD AND PREPARATION METHOD THEREFOR**

(30) Priority: 28.11.2018 CN 201811437559; 25.08.2019 CN 201910787180
(71) Applicant: Dong, Futian, Suzhou, Jiangsu 215123 (CN)
(72) Inventor: Dong, Futian, Suzhou, Jiangsu 215123 (CN)
(74) Representative: Bianchetti & Minoja SRL
(86) International application number: PCT/CN2019/120724
(87) International publication number: WO 2020/108445

(57) **Abstract**

The present invention discloses a substance for treating and/or preventing intolerance diseases and a design method and preparation method thereof. The stable structure corresponding to the substance related to the intolerance disease is used as the substance for treating and/or preventing the intolerance disease. The essence of disease is the imbalance of biological structure system. The imbalance of the structure of the substance in the biological structure system will cause diseases related to said substance. The present disclosure relates to the treatment and/or prevention of the intolerance disease by recognizing the stable structure corresponding to the substance by the body, adjusting the expression of its gene expression or genes by the self-adaptation, self-organization and other functions of body's structural system, and restoring the balance of the structure of the substance in the biological structure system.

## Description

### Technical Field

The invention belongs to the fields of medicines, health-care foods, food additives and the like, and particularly relates to substance for preventing or treating intolerance diseases, design method and preparation method thereof.

### Background

Intolerance refers to a complex adverse reaction disease that can cause inflammation in all tissues (including blood vessels), and manifests as symptoms and diseases of various systems throughout the body. Among the intolerance diseases, the proportion of drug intolerance is the highest, and according to statistics, at least 50% of the population have different degrees of adverse reactions to certain drugs, and the incidence of infants and children is higher than that of adults. Most patients with drug intolerance have gastrointestinal symptoms and skin reactions, but their manifestations are not limited to these, and intolerance to the same drug may present very different symptoms in different people. Drug intolerance is the main side effect of drugs, accounting for more than 90% of all side effects of drugs, including western medicine, traditional Chinese medicine, compound medicines and the like. Skin intolerance mainly refers to the phenomenon of skin erythema, prickling, molting and other adverse reactions caused by skin contact, and is mainly caused by the increasing number of skin care products, various chemical components, animal and plant extracts and some unidentified components in cosmetics. For the clinical treatment of intolerance, traditional Chinese medicine and Western medicine still remain at diet intervention or contact intervention, that is, avoiding eating and contact, and there is no fundamental solution.

According to the new medical model, namely structural information medicine, the present invention recognizes life from the perspective of structural system, and restores the homeostasis of structural system by allowing the body to recognize the information of specific structure, using the self-adaptive and self-organizing functions of the structural system of the body, to achieve the purpose of preventing and curing diseases.

### 1. The Medical Model of Traditional Chinese Medicine (TCM) and Western Medicine

The existing medical system mainly comprises Western medicine and traditional Chinese medicine, wherein the Western medicine is based on microscopic local structure, which is good at studying things, but weak in making a thorough inquiry into truth; on the contrary, the traditional Chinese medicine is mainly based on the macroscopic overall function system, which is good at the theory of the things but weak in the aspect of studying things. The advantages of the two are complementary, but the theoretical system and the research method are opposite to each other. Western medicine mainly relies on scientific experiments, using basic research methods such as anatomical physiology, biochemistry and molecular biology, and clinical trials to analyze the essence of disease through the theory of segmentation and reduction. Therefore, Western medicine focuses more on the structural materials of pathological changes, analyzes the change of specific structures, and then directly eliminate the surplus or supplement the deficiency from the level of local material structures by means of chemical drugs, operations, rays and the like, such as H2 receptor antagonists for treating hyperchlorhydria, hormone replacement therapy and the like. Such therapy has a fast onset and is effective in some acute diseases, surgical diseases and infectious diseases. However, Western medicine lacks a comprehensive understanding of the body. This type of treatment can only temporarily solve the problem of local structure. Long-term use will have adverse effects on other structures of the body and disrupt the homeostasis of the body. In fact, from 2010, one third of the diseases in the world are drug-induced diseases, which are all caused by inevitable side effects of drugs. Moreover, Western medicine has little effect on chronic degenerative diseases that have complex pathogenic factors and are affected by multiple factors, such as cardiovascular and cerebrovascular diseases, multiple sclerosis, senile dementia and the like, and is difficult to suppress the increasing morbidity and mortality of these diseases.

TCM treats life as a whole, unifying the human body with society, psychology, and environment. The core of TCM is syndrome differentiation and treatment, which reflects the objective laws of nature with humans, health and disease. Health in the meaning of TCM emphasizes the relative balance and coordination of Yin and Yang in the body in subjective and objective environments, i.e., the balance of functional systems. When this balance is broken and the order and homeostasis of the body is disturbed or destroyed, i.e., the functional system of the body is unbalanced, diseases occur. TCM diagnoses the nature of diseases by inquiring a large amount of functional system information, and then mobilizes the functional systems of the human body by methods of medicines, acupuncture, massage and the like, so as to eliminate the surplus, supplement the deficiency, strengthen the body resistance to eliminate pathogenic factors, and restore the body's homeostasis. TCM therapy has a slow onset of action, but it reduces the side effects of replacement therapy or antagonistic drugs, and is quite effective in disease prevention and treatment, health preservation, rehabilitation and the like.

In summary, Western medicine focuses more on micro structural balance, and TCM focuses more on macro functional system balance, both of which aim to correct the imbalance of the body at different levels. However, the two are completely different in the epistemology and methodology of disease and health. In order to allow these two medical models to complement each other, we need to combine the two with a more inclusive perspective and theory, and develop more effective substances for treating diseases, so as to make more outstanding contributions to human health.

### 2. Structural Information Medicine

### (1) Basic Theory of Combining Western Medicine and TCM by Structural System

After the Big Bang, positive and negative charges appeared, and the two interacted to form a simple structure of hydrogen, and then the interaction between the structures formed a more complex structure, and finally formed a macroscopic matter, including the entire biological world. Therefore, everything in the universe is unified in structure. The substance, energy and information constituting all things in the universe are also unified in the structure. Firstly, different structures form different substances, the substances are specific structures and are the basis for realizing functions. The structure and function of the living system are matched, and the specific structure has the specific function. The change of the specific structure will naturally affect the specific function of the corresponding system. Secondly, the bond energy of the interaction between structures is a relatively abstract structure, indicating the stability of the structure, and the energy is the internal driving force for the interconnection of the structure. Finally, the relationship and the order among the structures are information, the information is the most abstract structure among the three (i.e., substance, energy and information), plays a role in combining and controlling substances and energy, reflects the intrinsic rules and essence of the structures, just as the essence of organisms is determined by its abstract structure, gene. Therefore, the structure is a unity of substance, energy, and information, that is, a unity of a concrete structure and an abstract structure.

A living being is an organic system, essentially an organic structural system dominated by abstract structures. The structural system is an organic structure whole with a certain function formed by relatively stable connection ways, organizational order and temporal and spatial relations among several elements. Any element itself is also a structural system, a subsystem that constitutes the original system, and the subsystem *per se* must be composed of sub-level subsystems. The structural system of a living being is composed of structural subsystems at different levels, such as immune system, gastrointestinal system and the like, and the living being itself is a subsystem of a larger structural system, e.g., nature, and maintains its own existence and reproduction in the process of continuous exchange of substance, energy and information with nature.

DNA, as the genetic material of an organism, stores all the information during life activities, including growth, development, aging and death. In the theory of biochemical individualization, Dr. Roger Williams showed that the difference in the biochemical composition of the human body comes from the difference in genes and gene expression, that is, the difference in the spatial structure of molecules resulting from gene expression. Dr. Susumu Tonegawa, Nobel Prize winner in Physiology, showed that all diseases except trauma are related to genes. The causes of the disease mainly include two aspects: one is that the structures that constitute the body are damaged or changed by the structural environment; the other is improper gene expression. The fundamental cause of structural imbalance is the change of abstract structure, that is, the change of gene expression, such as abnormal histone acetylation, methylation, and abnormal gene methylation. If the specific structure has changed, as long as the genes related to the structure can be expressed normally, the body's metabolism will gradually replace the diseased structure with the normal structure, and the body can return to a healthy state. Therefore, the core of biological health lies in the genes and their expression.

Western medicine studies the specific structural changes, takes the disease as a model, and treats the disease based on the model. Western medicine focuses on the study of local tissues and functions in structural systems, can detect with the aid of instruments and go deep into specific and microscopic material structures. Western medicine focuses on and emphasizes the existence forms of specific human material structures and morphologies, such as anatomy, molecular biology, cytology, histology, etc., all of which are experimental ways to study and observe specific structures in structural systems from different angles, and are deficient in abstract thinking, ignoring the integrity of the body as a structural system. Therefore, it is easy to have limitations in understanding and practice. Western medicine mainly directly intervenes in specific structures, uses drugs to change and influence local structures of the body to achieve a new balance, including the study of genes, and tends to directly change genes or gene expression. TCM takes humans as a model, studies the functions of various organs of the body and the interrelationships and effects between functions under the guidance of holism. TCM does not detect specific material structures, but diagnoses diseases through functional manifestations and external information. However, the function is determined by the structure, the essence of the functional system is the structural system, and the mutual generation and restriction between the functional subsystems are essentially the mutual promotion and mutual restriction between the structural subsystems. TCM achieves overall balance by conditioning the functional order and strength of the body, and the essence of the TCM is to restore the dynamic balance of the structural system. However, TCM has great limitation due to the lack of in-depth understanding of the specific body structure. Western medicine mainly builds disease models from the perspective of microstructure, while TCM builds life models from the perspective of macroscopic functions. The microstructure of Western medicine has found the material basis for the TCM, that is, TCM syndromes have material support, namely the structural support of gene expression, and the essence is the microcosmic structural imbalance. In the structural system, based on the unification of the micro and macro, the part and the whole as well as the concrete and the abstract, the local and microcosmic concrete structure can be utilized to convert the concrete structure into abstract structural information through the recognition of the concrete structure by the body, stimulate the body's self-adaptive and self-organizing functions to adjust the genes or the expression of the genes, and regain the balance of the structural system.

As a system, the structural system has the basic characteristics of the system, such as integrity, openness, stability, and self-organization. Relative to the environment, the system is the unity of closedness and openness. The interaction between the structures in the structural system is on the premise of the interaction between the structures and the environment and on the condition of the external environment of the structures. The evolution of the same structural system depends on the external structure. The proper change and adjustment of gene expression along with changes in external structure is the basic function of organisms to adapt to the environment, maintain survival, and maintain shelf health. If this function is lacking, the organisms will generally be eliminated. The structural system of the human body has this powerful self-health function. Structural information medicine is a relatively complex, highly ordered, and autonomously regulated mechanism of action that mobilizes and regulates different levels of subsystems in the structural system, including genes, molecules, cells, tissues and organs, through the structural information of specific structures, so as to restore the structural balance of the body and achieve the purpose of preventing and treating diseases. Structural information medicine combines the theory of Western medicine that takes specific structure and function as the research object, and the theory of TCM that restores balance by adjusting the body itself.

### (2) Problems Solved by Structural Information Medicine

Living organisms cannot exist independently from the environment. The external structural environment interacts with the body's structural system through structure, namely matter, energy and information. Each individual is a unique structural system resulting from the interaction of genes and environment. Structural information medicine emphasizes the biological internal structural system and its dynamic balance with the external structural environment. It is this dynamic balance that interprets the process of life. In addition to restoring the dynamic balance of the body's own structural system and increasing the body's resistance, structural information medicine also focuses on symbiosis and co-progress. Symbiosis means that the body no longer resists and defends the external structure, but continuously internalizes the external structure information, and realizes the balance between the biological body's structural system and the external structure through the self-adaptation and self-organization functions of the organism, and realizes the transition from confrontation to peaceful coexistence and even benefit from it. Co-progress means that the structural system of a biological organism obtains the structural characteristics of other biological organisms that are beneficial to the health, survival and reproduction through structural information. The present invention is based on the established theoretical system of structural information medicine, uses the stable structure corresponding to the substance to regulate the gene or the expression of the gene through the self-adapting, self-organizing and other abilities of the organism's own structural system, so as to treat the diseases related to the substance.

### Disclosure of Invention

The object of the present invention is to address the above-mentioned drawbacks of the prior art by providing a method for designing a substance for the treatment and/or prevention of intolerance diseases.

Another object of the invention is to provide a substance designed according to this method.

The invention also aims to provide a preparation method and application of the substance.

The structural system of a biological organism is an organic structural system that combines stability and adaptability. It can adapt to the environment, maintain a dynamic balance between itself and the external environment, and then achieve better survival and reproduction. The adaptability of the biological structure system is based on the stability of the biological structure, and the stable structure is the dominant structure that plays a key role.

Biological structure system is an organic giant system formed by spiral development of core structure composed of concrete structure and abstract structure. The core structure of the biological structural system, that is, a complex in which a concrete structure and an abstract structure are unified in a nucleic acid-protein structural system, serves as a genetic information carrier and expression system, and plays a dominant role in the function of the biological structural system. What plays a dominant role in the core structure is the stable structure. At the atomic level, the abstract structure DNA and the concrete structure protein in the core structure are both biological macromolecules based on carbon elements. There are 4 electrons in the outermost layer of a carbon atom, and it is neither easy to lose electrons nor get electrons easily. Such structure is favorable for the carbon atom to form 4 covalent bonds. The covalent bond has high stability and is the strongest acting force among organic molecules. The bond energy of the covalent single bond mainly formed by carbon atoms is about 300-500 KJ/moL, and the thermal cracking temperature is 500-800°C.

The primary structure of DNA determines the double-helix secondary structure of DNA through the actions of base hydrogen bond, base stacking force and the like, namely, the stable primary structure determines the adaptive secondary structure of the DNA, and the core of genetic information is in the stable structure. DNA transfers genetic information to RNA by transcription and finally RNA expresses genetic information into protein by translation. This is the law followed by all cellular organisms. The establishment of the central law clarifies the information transmission path of genetic information from DNA to RNA to protein. From the base sequence in the primary structure of DNA to the base sequence in the primary structure of RNA and then to the amino acid sequence in the primary structure of protein, the genetic information carrier maps the information one-to-one to the functional information carrier amino acids through base complementary pairing and triplet codons. Therefore, this information transmission path is based on the stable structure of these biological macromolecules, and is transferred from the stable structure of the abstract structure to the stable structure of the concrete structure.

The covalent backbone in the primary structure of a protein contains hundreds of single bonds which can rotate freely, but the rotational freedom of each single bond is limited by factors such as the rigid planar nature of the peptide bonds in the primary structure, the number and position of hydrophilic/hydrophobic amino acids and acidic/basic amino acids, and the like, and finally, a three-dimensional structure which is most thermodynamically stable is formed. Therefore, in fact, the control component of the final structure of the protein, that is, the amino acid sequence, is formed very early. These stable structures that dominate the protein conformation form a framework, from which the protein assembles into its final form during folding of the polypeptide chains. The amino acid sequence of a protein contains all the information that determines its three-dimensional structure, and the structure of a protein determines the function thereof. Therefore, consistent with the genetic information, the nature of the functional information is that the stable structure determines the adaptive structure.

From the system perspective, the stability of a biological structural system is the self-stabilizing ability of an organic structural system formed by structural interaction. Each structure has structures that promote it and structures that inhibit it in the structural system, they form a dynamic balance in the interaction, that is, the structure is not lacked or excessive, and can be self-adjusted within a certain range according to environmental changes, so that the original ordered state is maintained and restored, namely, the order of the structure is maintained in an unbalanced state, wherein the interaction between the stable structures plays a dominant role. For example, in the recognition of structural information, the innate immune system in animals and plants can recognize the structural features of pathogens through pattern recognition receptors (PRRs). These features are stable molecular structures (e.g., cell walls composed of LPS in all bacteria) that are not easily mutated and are common to different types of pathogens. Take food allergies as an example, even if food containing allergens is steamed at high temperature, it will still cause allergies. The immune recognition recognizes the stable structure of allergens. Taking the neurotransmitter opioid peptide as another example, opioid peptides with different stereo conformations have different selectivity on cell membrane receptors, and endomorphin-1 is a biological active peptide with the highest affinity and selectivity on |_i-receptors known at present. The adaptive structure is the molecular basis for the binding of signal molecules to receptors, and in view of the change in its stable structure, amino acid, the difference between endomorphin-1 and other opioid peptides is mainly in three amino acids. Through the change of amino acid residues, the proper spatial layout is formed, and the spatial conformation is changed. This plays a role in correct positioning for this type of transmitter to bind to the receptor. The interaction of the stable structures can cause the obvious change of the adaptive structure, and plays a great role in information recognition. During the process of information transmission, receptors on cell membranes need to bring information into cell nuclei, which mainly depends on protein modification. The basis of protein modification is the chemical reactivity of amino acid residues in a primary structure. By adjusting the stable structure of the protein under the action of enzymes, the high order structure of the protein is more adapted to the functional requirements under specific time and space conditions. The modification has the characteristics of reversibility and versatility, such as phosphorylation and dephosphorylation of proteins. For the information transmitted to the nucleus, the cell will eventually make corresponding feedback through changes in gene expression, and continue to transmit instructions through the signal pathway. Therefore, the interaction or structural adjustment of the stable structure determines the change of the adaptive structure, which may, of course, also counteract the stable structure.

Among the biological structural systems, no structure is "isolated". The interaction, mutual restriction and interdependence between the structures and the sub-structure systems constitute the organic structural system of living beings. All organisms are composed of cells, and cells are the sub-structural systems of this large structural system of the body. Depending on the complexity of biological organisms, it is composed of hundreds, tens of thousands, or even hundreds of millions of cells that perform various specific functions. Various cells have different functions but cooperate with each other, thus forming an orderly and controllable cell society. The maintenance of this kind of sociality depends not only on the material metabolism and energy metabolism of the cell, but also on the communication and signal regulation between cells, so as to coordinate the behavior of the cell, such as cell growth, division, differentiation, apoptosis and other various physiological functions, and realize the complete life activity process of multicellular organisms. The essence of intercellular communication and signal regulation is also the interaction between structures, which determines the behavior and fate of cells, including structural and functional differentiation, location, survival, and death. The morphological structure, life activities and position of cells in the body are all regulated and controlled by the body, local tissues, surrounding cells and extracellular signal molecules, such as nerve cells, immune cells and endocrine cells, which participate in and maintain the homeostatic balance of the body through social connection. The interaction of structures in a biological structure system allows cells to have the ability to distinguish itself from foreign objects, and to distinguish between the same species and heterogeneous cells. The phenomenon of mutual recognition and identification between cells and the recognition of molecules of self and foreign substances is called cell recognition. Cells adhere to each other or establish a stable connection with certain morphological structure on the basis of recognition, thereby influencing and interacting with each other. Many vital activities are related to the recognition ability of cells. Cell recognition is also a very important link in the development and differentiation process of cells, which form different types of tissues through recognition and adhesion. Body immunity is actually a recognition phenomenon of cells. White blood cells in the blood can recognize invading bacteria and phagocytose them, but never phagocytose their own normal cells in the blood. This is the recognition of cells between different species. When the allogeneic tissue is transplanted clinically, the body will reject the transplanted allogeneic tissue, which is caused by the cell recognition between allogeneic cells. In addition, many physiological and pathological processes, such as blood coagulation, inflammatory reaction, thrombosis, infection by pathogenic microorganisms, and tumor cell metastasis, are related to the complex mutual recognition and adhesion between the same species or different species and the induced effects thereof. Structure recognition is based on interactions between structures that underlie the self-adaptive and self-organizing organic structural system formed by organisms.

The openness of the structural system is not only the openness of specific structures, but also the openness between relationships. The self-adaptive and self-organizing capabilities of the structural system are mainly reflected in the stability of the system under external environmental stimuli. The structural system of the body realizes self-adjustment and self-organization through the transmission and processing of information, and continuously overcomes the uncertainty of the structural system so as to keep the structural system in dynamic balance. The exchange between the structural system and its environment is not only the exchange of matter, but also the exchange of energy or information. To maintain homeostasis in a constantly changing environment, organisms need cells to have the ability to perceive these changes and respond. The ability of cells to receive and transmit information and execute instructions is the basis for organisms to respond to changes in internal and external environments. Proteins are the effectors of various physiological activities in cells (e.g., catalyzing chemical reactions and regulating gene expression), and changes in the state of the protein itself may also change its functional state. Organisms use protein molecules that are possessed by cells to build a signaling system. Because the shape of the protein molecule can be changed, the functions closely related to the shape of the protein molecule will change accordingly. The change in the state of a protein will change the state of downstream molecules by interacting with downstream protein molecules, and downstream molecules can change the state of further downstream molecules. Such successive changes of the state of each level are the way of information transmission in the cell. Furthermore, proteins are molecules with biological functions, which make the final response to signals after state changes and become effector molecules. The effector molecules at the end of the information transmission chain are mostly transcription factors, and respond to signals by regulating the expression of genes.

Taking the immune system as an example, any external structure entering the body will be recognized and evaluated by the immune system. For example, when bacteria or viruses enter the human body, immune cells interact with foreign structures to recognize their structural information, then secrete different cytokines according to the difference in structural information to transmit the information to B lymphocytes, regulate the gene expression of the B cells to synthesize IgG antibody, which binds to the antigen structure to form a complex and is eventually cleared by macrophages, so that the invasion of foreign matters is resisted. When parasitic pathogens invade, immune cells interact with foreign structures, and the recognized structural information activates B cells and regulates gene expression to synthesize IgE antibodies. These antibodies bind to mast cells and basophils, causing them to release toxic granular proteins and other substances, leading to the death of parasites.

During the immune response, the recognized structure determines the body's specific feedback. Through the interaction with the structure, the concrete structure is converted into the abstract structure, i.e., structural information. According to the recognition of the structural information, the body mobilizes multiple cells in the immune system to act synergistically, regulate gene expression, release different active substances to adapt to foreign objects, and realize a new stable and harmonious structural system. At the same time, the immune system interacts with other systems to exchange materials, energy, and information, so as to realize the coordination and cooperation between different subsystems in the structural system. It can be seen that there are structural information recognition and self-regulation in the structural system of the body, which restore the balance of the structural system, and even establish a new structural balance mechanism with the external structure.

A disease is an abnormal process of vital activities that occurs due to a disorder of homeostasis under certain etiology, and triggers a series of changes in metabolism, function, and structure, manifested as abnormal symptoms, signs and behaviors. The disease is an abnormal life process caused by self-stabilization regulation disorder after the organism is damaged under certain conditions. From a structural point of view, diseases of living beings are caused by imbalance in the structural systems of living beings, including: (1) structural imbalance within a biological structural system, (2) structural imbalance between a biological structural system and an external structure, i.e., the structural system of a living being lacks substances that inhibit the external structure. Therefore, substances related to diseases include: substance that causes the disease, substance of the organism itself and related to the disease, and substance that inhibits structure that causes the disease. The substance that inhibits the structure that causes the disease is a substance that can establish a structural balance with the structure that causes the disease. Disease is an extremely complex process. In many cases, from health to disease is a process from quantitative change to qualitative change. Some diseases involve the structural imbalance of multiple systems in the biological structure system. The "syndrome" of TCM reflects the essence of the disease, and the "syndrome" is differentiated and treated. The "syndrome" of TCM is neither a disease or a disorder as described by Western medicine, nor a generalization of a single factor or linear causal relationship of the disease, it refers to the understanding and generalization of the body's response state on various physiological and pathological associations such as the etiology, disease location, pathogenesis, pathological nature, and struggle between vital qi and pathogenic factors of a disease at certain stages. It focuses on the contradiction between vital qi and pathogenic factors of the human body. Therefore, strengthening the body resistance to eliminate pathogens and eliminating pathogens to calm the body resistance, which focus on both the body resistance and pathogens, regulate Yin and Yang of the human body, restore the relative balance of Yin and Yang, and coordinate the steady state of the body resistance and pathogens, are the basic rules of TCM.

Western medicine studies the relationship between the structure and the function from a concrete and micro perspective, thereby studying health and diseases; TCM studies the multi-dimensional and two-way regulation relationship of generation, restriction, control and transformation among human body functional systems from an abstract and macro perspective, thereby studying health and diseases. From a structural point of view, the essence of living things is a dynamic balanced, coordinated and orderly, self-adaptive, self-organizing organic structural system formed by the interaction between body structures. Structural information medicine integrates the basic theories of TCM and Western medicine with structure, recognizes life from the perspective of structural systems, and restores the dynamic balance of structural systems by allowing the body to recognize the information of specific structures and using the self-adaptation and self-organization functions of the body's structural system, so as to achieve the purpose of preventing and curing diseases.

Different lesions of the same organ can cause different diseases, and the symptoms of the same disease in different individuals are not exactly the same. In addition, the same disease may involve lesions of a plurality of different organs or tissues in a biological structural system, and the structures of the lesions of different individuals having the same disease are not identical. Therefore, the present invention combines the concrete structure of Western medicine research and the macroscopic theory established by TCM, treats and/or prevents diseases from the perspective of structural information. For the disease of the organism, only the substances related to the disease and the specific diseased cells, tissues or organs where the disease-related substances are located need to be known. There is no need to explore the mechanism through which the disease is caused, the specific symptoms of the disease, or the system where the disease-related substances are located. Whether in accordance with Western medicine or TCM, from a systematic point of view, people are the same, and even humans, pigs, cows, sheep, and primates are basically the same. Therefore, when the specific structure of the lesion cannot be determined or it is not easy to separate the structure of the lesion, treating and/or preventing the disease with the stable structure corresponding to the cells, tissues or organs containing the lesion structure (that is, the unbalanced structure) will be more versatile and easier to obtain and prepare. Therefore, the method for designing substances for the treatment and/or prevention of diseases disclosed in the present invention uses the stable structure corresponding to the substance related to diseases as an external structure to act on the structural system of the organism, and the structural system of the organism enable the organism to establish the balance with the structure of the external substance or restore the structural imbalance in the biological structural system according to structural self-adaptation and self-organization, thereby eliminating, relieving the diseases or preventing the diseases. For example, the stable structure corresponding to the lung of pig is used to treat and/or prevent asthma or allergic rhinitis, which is a disease on the nose that interacts with the structure of the lung, that is, a disease related to the lung. The method for designing substances for the treatment and/or prevention of diseases of the present invention mainly includes supporting vital qi with evil qi (pathogenic factor), and strengthening vital qi with vital qi.

The imbalance of the biological structure system includes the imbalance between the biological structure system and the structure other than itself. In the present invention, the biological structure system itself is called "vital qi", and the external structure that causes the imbalance of the biological structure system is called "external evil qi (exogenous pathogenic factor)". "External evil qi" is relative to living beings and includes substances that are harmful to healthy organisms, such as pathogenic bacteria, viruses, and the like, as well as substances that are not harmful to healthy organisms themselves, but for some organisms, may cause disease when these organisms come into contact with the substance. For example, a substance is harmless to a healthy living being, and even beneficial, i.e., the structure of the substance is balanced with the structure of the living being. However, if the body's structure is unbalanced, the balance between the structure of the substance and the structure of the organism is broken, diseases will occur, and different imbalances can cause different diseases, such as allergy, intolerance to the substance, such as allergy and intolerance to milk. Due to the functions of self-adaptation, self-organization, self-stabilization and the like of the biological structure system, under the action of the external structure, a structure that interacts with the external structure in an inhibitory or promoting manner will be produced, so that the balance between the biological structure system and the external structure is established. This is a way of supporting "vital qi" with "external evil qi".

The pathological changes of the internal structure of the biological structural system are often imbalance of structural interaction. For example, for a certain structure in the biological structural system, the lack of expression of genes of the structure that inhibits it or the overexpression of genes of the structure that promotes it, will break the balance between the structures. For example, tumor suppressor genes and proto-oncogenes restrict each other and maintain the relative stability of positive and negative regulatory signals. When such genes are mutated, deleted or inactivated, the balance of interactions between structures is broken, which can cause malignant transformation of cells and lead to tumors. In the present invention, the unbalanced structure in the biological structural system is called "internal evil qi (endogenous pathogenic factor)", and the biological structure system itself is called "vital qi". According to the present invention, the stable structure corresponding to the unbalanced substance inside the organism is used as an external structure to act on the biological structural system, and due to the functions of self-adaptation, self-organization, self-stabilization and the like of the biological structure system, the biological structure system can regulate the gene expression of the structure that interacts with the external structure according to the structural information in said external structure, so as to achieve a balance with the external structure, restore the balance of the unbalanced internal structure of the biological structure system, and cure diseases. This is a way of supporting "vital qi" with "internal evil qi".

Every normal structure in a biological structural system has a structure that interacts with it, i.e., a balanced structure, and the present invention refers to both the balanced structure and the biological structural system as "vital qi". The stable structure corresponding to the normal non-pathological substance in the system is used as an external structure to act on the biological structure system, and due to the functions of self-adaptation, self-organization, self-stabilization and the like of the biological structure system, the biological structure system can regulate the gene expression of the structure that interacts with the external structure to a normal level according to the structural information of each structure in said external structure, so the unbalanced structure naturally restores the balance. According to the present invention, the stable structure corresponding to the non-pathological substance is used as the substance for the treatment and/or prevention of diseases caused by the pathological changes of the same or similar substance as said non-pathological substance. This is a way of strengthening "vital qi" with "vital qi".

The balance of the biological structural system is dynamic, especially the balance between the biological structural system and the structure of the environment. For example, if a disease-causing substance does not cause disease in a certain organism, then the structural system of said organism and the structure of the disease-causing substance can reach a balance when interacting, in other words, in the structure of the organism related to the disease, there is a structure that interacts with the structure of the disease-causing substance to avoid the occurrence of the disease. Said structure does not exist in isolation in the biological structure system, and there are structures that interact with this structure in the biological system, thus forming a balance. The stable structure corresponding to the substance related to the disease in a certain organism is used as an external structure to act on a biological structure system in need of treatment said disease, and due to the functions of self-adaptation, self-organization, self-stabilization and the like of the biological structure system, the biological structure system can regulate the gene or gene expression of the structure that interacts with the external structure according to the structural information of each structure in the external structure, so as to achieve a balance between the biological structure system and the structure of the disease-causing substance. This is also is also a way of strengthening "vital qi" with "vital qi".

The invention is characterized in that:
The present invention is based on the established theoretical system of structural information medicine, uses the stable structure corresponding to the substance to act on the biological structural system, regulates the gene or the expression of the gene through the self-adapting, self-organizing and other abilities of the organism's own structural system, so as to treat the diseases related to the substance.

In view of this, the present invention provides substances for the treatment and/or prevention of intolerance diseases, as well as design methods and preparation methods of said substances. The specific embodiments are as follows:
A method for designing substances for the treatment and/or prevention of intolerance diseases, wherein the stable structure corresponding to the substance related to the intolerance disease is used as the substance for treating and/or preventing the intolerance disease.

Preferably, the stable structure corresponding to the substance refers to the structure of said substance that exists in a solid state at a high temperature of 500 °C or higher.

Preferably, the stable structure corresponding to the substance related to the intolerance disease is selected from one or more of the following:
(1) the stable structure corresponding to the substance that causes intolerance in the organism;
(2) the stable structure corresponding to the substance of the organism itself and related to intolerance disease;
(3) the stable structure corresponding to the substance that inhibits the structure that causes the intolerance disease;
(4) the stable structure corresponding to the substance containing a substance of any one of (1) to (3) above.

The substance for treating and/or preventing the intolerance disease designed according to the above-mentioned method.

The preparation method of the above-mentioned substance for treating and/or preventing the intolerance disease, wherein the stable structure corresponding to the substance related to the intolerance disease is prepared by a high-temperature carbonization method.

Use of the above-mentioned substance for treating and/or preventing the intolerance disease in the preparation of medicines, health products, foods, and food additives.

A method for designing substances for the treatment and/or prevention of food intolerance diseases, wherein the stable structure corresponding to the substance related to the food intolerance disease is used as the substance for treating and/or preventing the food intolerance disease.

Preferably, the stable structure corresponding to the substance refers to the structure of said substance that exists in a solid state at a high temperature of 500 °C or higher.

Preferably, the stable structure corresponding to the substance related to the food intolerance disease is selected from one or more of the following:
(1) the stable structure corresponding to the substance that causes food intolerance in the organism;
(2) the stable structure corresponding to the substance of the organism itself and related to food intolerance disease;
(3) the stable structure corresponding to the substance that inhibits the structure that causes the food intolerance disease;
(4) the stable structure corresponding to the substance containing a substance of any one of (1) to (3) above.

The substance for treating and/or preventing the food intolerance disease designed according to the above-mentioned method.

The preparation method of the above-mentioned substance for treating and/or preventing the food intolerance disease, wherein the stable structure corresponding to the substance related to the food intolerance disease is prepared by a high-temperature carbonization method.

Use of the above-mentioned substance for treating and/or preventing the food intolerance disease in the preparation of medicines, health products, foods, and food additives.

A method for designing substances for the treatment and/or prevention of barley intolerance disease, wherein the stable structure corresponding to the substance related to barley intolerance disease is used as the substance for treating and/or preventing barley intolerance disease.

Preferably, the stable structure corresponding to the substance refers to the structure of said substance that exists in a solid state at a high temperature of 500 °C or higher.

Preferably, the stable structure corresponding to the substance related to barley intolerance disease is selected from one or more of the following:
(1) the stable structure corresponding to the substance that causes barley intolerance in the organism;
(2) the stable structure corresponding to the substance of the organism itself and related to barley intolerance disease;
(3) the stable structure corresponding to the substance that inhibits the structure that causes barley intolerance disease;
(4) the stable structure corresponding to the substance containing a substance of any one of (1) to (3) above.

The substance for treating and/or preventing barley intolerance disease designed according to the above-mentioned method.

The preparation method of the above-mentioned substance for treating and/or preventing barley intolerance disease, wherein the stable structure corresponding to the substance related to barley intolerance disease is prepared by a high-temperature carbonization method.

Use of the above-mentioned substance for treating and/or preventing barley intolerance disease in the preparation of medicines, health products, foods, and food additives.

A method for designing substances for the treatment and/or prevention of buckwheat intolerance disease, wherein the stable structure corresponding to the substance related to buckwheat intolerance disease is used as the substance for treating and/or preventing buckwheat intolerance disease.

Preferably, the stable structure corresponding to the substance refers to the structure of said substance that exists in a solid state at a high temperature of 500 °C or higher.

Preferably, the stable structure corresponding to the substance related to buckwheat intolerance disease is selected from one or more of the following:
(1) the stable structure corresponding to the substance that causes buckwheat intolerance in the organism;
(2) the stable structure corresponding to the substance of the organism itself and related to buckwheat intolerance disease;
(3) the stable structure corresponding to the substance that inhibits the structure that causes buckwheat intolerance disease;
(4) the stable structure corresponding to the substance containing a substance of any one of (1) to (3) above.

The substance for treating and/or preventing buckwheat intolerance disease designed according to the above-mentioned method.

The preparation method of the above-mentioned substance for treating and/or preventing buckwheat intolerance disease, wherein the stable structure corresponding to the substance related to buckwheat intolerance disease is prepared by a high-temperature carbonization method.

Use of the above-mentioned substance for treating and/or preventing buckwheat intolerance disease in the preparation of medicines, health products, foods, and food additives.

A method for designing substances for the treatment and/or prevention of corn intolerance disease, wherein the stable structure corresponding to the substance related to corn intolerance disease is used as the substance for treating and/or preventing corn intolerance disease.

Preferably, the stable structure corresponding to the substance refers to the structure of said substance that exists in a solid state at a high temperature of 500 °C or higher.

Preferably, the stable structure corresponding to the substance related to corn intolerance disease is selected from one or more of the following:
(1) the stable structure corresponding to the substance that causes corn intolerance in the organism;
(2) the stable structure corresponding to the substance of the organism itself and related to corn intolerance disease;
(3) the stable structure corresponding to the substance that inhibits the structure that causes corn intolerance disease;
(4) the stable structure corresponding to the substance containing a substance of any one of (1) to (3) above.

The substance for treating and/or preventing corn intolerance disease designed according to the above-mentioned method.

The preparation method of the above-mentioned substance for treating and/or preventing corn intolerance disease, wherein the stable structure corresponding to the substance related to corn intolerance disease is prepared by a high-temperature carbonization method.

Use of the above-mentioned substance for treating and/or preventing corn intolerance disease in the preparation of medicines, health products, foods, and food additives.

A method for designing substances for the treatment and/or prevention of malt intolerance disease, wherein the stable structure corresponding to the substance related to malt intolerance disease is used as the substance for treating and/or preventing malt intolerance disease.

Preferably, the stable structure corresponding to the substance refers to the structure of said substance that exists in a solid state at a high temperature of 500 °C or higher.

Preferably, the stable structure corresponding to the substance related to malt intolerance disease is selected from one or more of the following:
(1) the stable structure corresponding to the substance that causes malt intolerance in the organism;
(2) the stable structure corresponding to the substance of the organism itself and related to malt intolerance disease;
(3) the stable structure corresponding to the substance that inhibits the structure that causes malt intolerance disease;
(4) the stable structure corresponding to the substance containing a substance of any one of (1) to (3) above.

The substance for treating and/or preventing malt intolerance disease designed according to the above-mentioned method.

The preparation method of the above-mentioned substance for treating and/or preventing malt intolerance disease, wherein the stable structure corresponding to the substance related to malt intolerance disease is prepared by a high-temperature carbonization method.

Use of the above-mentioned substance for treating and/or preventing malt intolerance disease in the preparation of medicines, health products, foods, and food additives.

A method for designing substances for the treatment and/or prevention of oat intolerance disease, wherein the stable structure corresponding to the substance related to oat intolerance disease is used as the substance for treating and/or preventing oat intolerance disease.

Preferably, the stable structure corresponding to the substance refers to the structure of said substance that exists in a solid state at a high temperature of 500 °C or higher.

Preferably, the stable structure corresponding to the substance related to oat intolerance disease is selected from one or more of the following:
(1) the stable structure corresponding to the substance that causes oat intolerance in the organism;
(2) the stable structure corresponding to the substance of the organism itself and related to oat intolerance disease;
(3) the stable structure corresponding to the substance that inhibits the structure that causes oat intolerance disease;
(4) the stable structure corresponding to the substance containing a substance of any one of (1) to (3) above.

The substance for treating and/or preventing oat intolerance disease designed according to the above-mentioned method.

The preparation method of the above-mentioned substance for treating and/or preventing oat intolerance disease, wherein the stable structure corresponding to the substance related to oat intolerance disease is prepared by a high-temperature carbonization method.

Use of the above-mentioned substance for treating and/or preventing oat intolerance disease in the preparation of medicines, health products, foods, and food additives.

A method for designing substances for the treatment and/or prevention of rye intolerance disease, wherein the stable structure corresponding to the substance related to rye intolerance disease is used as the substance for treating and/or preventing rye intolerance disease.

Preferably, the stable structure corresponding to the substance refers to the structure of said substance that exists in a solid state at a high temperature of 500 °C or higher.

Preferably, the stable structure corresponding to the substance related to rye intolerance disease is selected from one or more of the following:
(1) the stable structure corresponding to the substance that causes rye intolerance in the organism;
(2) the stable structure corresponding to the substance of the organism itself and related to rye intolerance disease;
(3) the stable structure corresponding to the substance that inhibits the structure that causes rye intolerance disease;
(4) the stable structure corresponding to the substance containing a substance of any one of (1) to (3) above.

The substance for treating and/or preventing rye intolerance disease designed according to the above-mentioned method.

The preparation method of the above-mentioned substance for treating and/or preventing rye intolerance disease, wherein the stable structure corresponding to the substance related to rye intolerance disease is prepared by a high-temperature carbonization method.

Use of the above-mentioned substance for treating and/or preventing rye intolerance disease in the preparation of medicines, health products, foods, and food additives.

A method for designing substances for the treatment and/or prevention of rice intolerance disease, wherein the stable structure corresponding to the substance related to rice intolerance disease is used as the substance for treating and/or preventing rice intolerance disease.

Preferably, the stable structure corresponding to the substance refers to the structure of said substance that exists in a solid state at a high temperature of 500 °C or higher.

Preferably, the stable structure corresponding to the substance related to rice intolerance disease is selected from one or more of the following:
(1) the stable structure corresponding to the substance that causes rice intolerance in the organism;
(2) the stable structure corresponding to the substance of the organism itself and related to rice intolerance disease;
(3) the stable structure corresponding to the substance that inhibits the structure that causes rice intolerance disease;
(4) the stable structure corresponding to the substance containing a substance of any one of (1) to (3) above.

The substance for treating and/or preventing rice intolerance disease designed according to the above-mentioned method.

The preparation method of the above-mentioned substance for treating and/or preventing rice intolerance disease, wherein the stable structure corresponding to the substance related to rice intolerance disease is prepared by a high-temperature carbonization method.

Use of the above-mentioned substance for treating and/or preventing rice intolerance disease in the preparation of medicines, health products, foods, and food additives.

A method for designing substances for the treatment and/or prevention of wheat intolerance disease, wherein the stable structure corresponding to the substance related to wheat intolerance disease is used as the substance for treating and/or preventing wheat intolerance disease.

Preferably, the stable structure corresponding to the substance refers to the structure of said substance that exists in a solid state at a high temperature of 500 °C or higher.

Preferably, the stable structure corresponding to the substance related to wheat intolerance disease is selected from one or more of the following:
(1) the stable structure corresponding to the substance that causes wheat intolerance in the organism;
(2) the stable structure corresponding to the substance of the organism itself and related to wheat intolerance disease;
(3) the stable structure corresponding to the substance that inhibits the structure that causes wheat intolerance disease;
(4) the stable structure corresponding to the substance containing a substance of any one of (1) to (3) above.

The substance for treating and/or preventing wheat intolerance disease designed according to the above-mentioned method.

The preparation method of the above-mentioned substance for treating and/or preventing wheat intolerance disease, wherein the stable structure corresponding to the substance related to wheat intolerance disease is prepared by a high-temperature carbonization method.

Use of the above-mentioned substance for treating and/or preventing wheat intolerance disease in the preparation of medicines, health products, foods, and food additives.

A method for designing substances for the treatment and/or prevention of gluten intolerance disease, wherein the stable structure corresponding to the substance related to gluten intolerance disease is used as the substance for treating and/or preventing gluten intolerance disease.

Preferably, the stable structure corresponding to the substance refers to the structure of said substance that exists in a solid state at a high temperature of 500 °C or higher.

Preferably, the stable structure corresponding to the substance related to gluten intolerance disease is selected from one or more of the following:
(1) the stable structure corresponding to the substance that causes gluten intolerance in the organism;
(2) the stable structure corresponding to the substance of the organism itself and related to gluten intolerance disease;
(3) the stable structure corresponding to the substance that inhibits the structure that causes gluten intolerance disease;
(4) the stable structure corresponding to the substance containing a substance of any one of (1) to (3) above.

The substance for treating and/or preventing gluten intolerance disease designed according to the above-mentioned method.

The preparation method of the above-mentioned substance for treating and/or preventing gluten intolerance disease, wherein the stable structure corresponding to the substance related to gluten intolerance disease is prepared by a high-temperature carbonization method.

Use of the above-mentioned substance for treating and/or preventing gluten intolerance disease in the preparation of medicines, health products, foods, and food additives.

A method for designing substances for the treatment and/or prevention of apple intolerance disease, wherein the stable structure corresponding to the substance related to apple intolerance disease is used as the substance for treating and/or preventing apple intolerance disease.

Preferably, the stable structure corresponding to the substance refers to the structure of said substance that exists in a solid state at a high temperature of 500 °C or higher.

Preferably, the stable structure corresponding to the substance related to apple intolerance disease is selected from one or more of the following:
(1) the stable structure corresponding to the substance that causes apple intolerance in the organism;
(2) the stable structure corresponding to the substance of the organism itself and related to apple intolerance disease;
(3) the stable structure corresponding to the substance that inhibits the structure that causes apple intolerance disease;
(4) the stable structure corresponding to the substance containing a substance of any one of (1) to (3) above.

The substance for treating and/or preventing apple intolerance disease designed according to the above-mentioned method.

The preparation method of the above-mentioned substance for treating and/or preventing apple intolerance disease, wherein the stable structure corresponding to the substance related to apple intolerance disease is prepared by a high-temperature carbonization method.

Use of the above-mentioned substance for treating and/or preventing apple intolerance disease in the preparation of medicines, health products, foods, and food additives.

A method for designing substances for the treatment and/or prevention of pear intolerance disease, wherein the stable structure corresponding to the substance related to pear intolerance disease is used as the substance for treating and/or preventing pear intolerance disease.

Preferably, the stable structure corresponding to the substance refers to the structure of said substance that exists in a solid state at a high temperature of 500 °C or higher.

Preferably, the stable structure corresponding to the substance related to pear intolerance disease is selected from one or more of the following:
(1) the stable structure corresponding to the substance that causes pear intolerance in the organism;
(2) the stable structure corresponding to the substance of the organism itself and related to pear intolerance disease;
(3) the stable structure corresponding to the substance that inhibits the structure that causes pear intolerance disease;
(4) the stable structure corresponding to the substance containing a substance of any one of (1) to (3) above.

The substance for treating and/or preventing pear intolerance disease designed according to the above-mentioned method.

The preparation method of the above-mentioned substance for treating and/or preventing pear intolerance disease, wherein the stable structure corresponding to the substance related to pear intolerance disease is prepared by a high-temperature carbonization method.

Use of the above-mentioned substance for treating and/or preventing pear intolerance disease in the preparation of medicines, health products, foods, and food additives.

A method for designing substances for the treatment and/or prevention of banana intolerance disease, wherein the stable structure corresponding to the substance related to banana intolerance disease is used as the substance for treating and/or preventing banana intolerance disease.

Preferably, the stable structure corresponding to the substance refers to the structure of said substance that exists in a solid state at a high temperature of 500 °C or higher.

Preferably, the stable structure corresponding to the substance related to banana intolerance disease is selected from one or more of the following:
(1) the stable structure corresponding to the substance that causes banana intolerance in the organism;
(2) the stable structure corresponding to the substance of the organism itself and related to banana intolerance disease;
(3) the stable structure corresponding to the substance that inhibits the structure that causes banana intolerance disease;
(4) the stable structure corresponding to the substance containing a substance of any one of (1) to (3) above.

The substance for treating and/or preventing banana intolerance disease designed according to the above-mentioned method.

The preparation method of the above-mentioned substance for treating and/or preventing banana intolerance disease, wherein the stable structure corresponding to the substance related to banana intolerance disease is prepared by a high-temperature carbonization method.

Use of the above-mentioned substance for treating and/or preventing banana intolerance disease in the preparation of medicines, health products, foods, and food additives.

A method for designing substances for the treatment and/or prevention of orange intolerance disease, wherein the stable structure corresponding to the substance related to orange intolerance disease is used as the substance for treating and/or preventing orange intolerance disease.

Preferably, the stable structure corresponding to the substance refers to the structure of said substance that exists in a solid state at a high temperature of 500 °C or higher.

Preferably, the stable structure corresponding to the substance related to orange intolerance disease is selected from one or more of the following:
(1) the stable structure corresponding to the substance that causes orange intolerance in the organism;
(2) the stable structure corresponding to the substance of the organism itself and related to orange intolerance disease;
(3) the stable structure corresponding to the substance that inhibits the structure that causes orange intolerance disease;
(4) the stable structure corresponding to the substance containing a substance of any one of (1) to (3) above.

The substance for treating and/or preventing orange intolerance disease designed according to the above-mentioned method.

The preparation method of the above-mentioned substance for treating and/or preventing orange intolerance disease, wherein the stable structure corresponding to the substance related to orange intolerance disease is prepared by a high-temperature carbonization method.

Use of the above-mentioned substance for treating and/or preventing orange intolerance disease in the preparation of medicines, health products, foods, and food additives.

A method for designing substances for the treatment and/or prevention of cantaloupe intolerance disease, wherein the stable structure corresponding to the substance related to cantaloupe intolerance disease is used as the substance for treating and/or preventing cantaloupe intolerance disease.

Preferably, the stable structure corresponding to the substance refers to the structure of said substance that exists in a solid state at a high temperature of 500 °C or higher.

Preferably, the stable structure corresponding to the substance related to cantaloupe intolerance disease is selected from one or more of the following:
(1) the stable structure corresponding to the substance that causes cantaloupe intolerance in the organism;
(2) the stable structure corresponding to the substance of the organism itself and related to cantaloupe intolerance disease;
(3) the stable structure corresponding to the substance that inhibits the structure that causes cantaloupe intolerance disease;
(4) the stable structure corresponding to the substance containing a substance of any one of (1) to (3) above.

The substance for treating and/or preventing cantaloupe intolerance disease designed according to the above-mentioned method.

The preparation method of the above-mentioned substance for treating and/or preventing cantaloupe intolerance disease, wherein the stable structure corresponding to the substance related to cantaloupe intolerance disease is prepared by a high-temperature carbonization method.

Use of the above-mentioned substance for treating and/or preventing cantaloupe intolerance disease in the preparation of medicines, health products, foods, and food additives.

A method for designing substances for the treatment and/or prevention of cinnamon intolerance disease, wherein the stable structure corresponding to the substance related to cinnamon intolerance disease is used as the substance for treating and/or preventing cinnamon intolerance disease.

Preferably, the stable structure corresponding to the substance refers to the structure of said substance that exists in a solid state at a high temperature of 500 °C or higher.

Preferably, the stable structure corresponding to the substance related to cinnamon intolerance disease is selected from one or more of the following:
(1) the stable structure corresponding to the substance that causes cinnamon intolerance in the organism;
(2) the stable structure corresponding to the substance of the organism itself and related to cinnamon intolerance disease;
(3) the stable structure corresponding to the substance that inhibits the structure that causes cinnamon intolerance disease;
(4) the stable structure corresponding to the substance containing a substance of any one of (1) to (3) above.

The substance for treating and/or preventing cinnamon intolerance disease designed according to the above-mentioned method.

The preparation method of the above-mentioned substance for treating and/or preventing cinnamon intolerance disease, wherein the stable structure corresponding to the substance related to cinnamon intolerance disease is prepared by a high-temperature carbonization method.

Use of the above-mentioned substance for treating and/or preventing cinnamon intolerance disease in the preparation of medicines, health products, foods, and food additives.

A method for designing substances for the treatment and/or prevention of grape intolerance disease, wherein the stable structure corresponding to the substance related to grape intolerance disease is used as the substance for treating and/or preventing grape intolerance disease.

Preferably, the stable structure corresponding to the substance refers to the structure of said substance that exists in a solid state at a high temperature of 500 °C or higher.

Preferably, the stable structure corresponding to the substance related to grape intolerance disease is selected from one or more of the following:
(1) the stable structure corresponding to the substance that causes grape intolerance in the organism;
(2) the stable structure corresponding to the substance of the organism itself and related to grape intolerance disease;
(3) the stable structure corresponding to the substance that inhibits the structure that causes grape intolerance disease;
(4) the stable structure corresponding to the substance containing a substance of any one of (1) to (3) above.

The substance for treating and/or preventing grape intolerance disease designed according to the above-mentioned method.

The preparation method of the above-mentioned substance for treating and/or preventing grape intolerance disease, wherein the stable structure corresponding to the substance related to grape intolerance disease is prepared by a high-temperature carbonization method.

Use of the above-mentioned substance for treating and/or preventing grape intolerance disease in the preparation of medicines, health products, foods, and food additives.

A method for designing substances for the treatment and/or prevention of pomelo intolerance disease, wherein the stable structure corresponding to the substance related to pomelo intolerance disease is used as the substance for treating and/or preventing pomelo intolerance disease.

Preferably, the stable structure corresponding to the substance refers to the structure of said substance that exists in a solid state at a high temperature of 500 °C or higher.

Preferably, the stable structure corresponding to the substance related to pomelo intolerance disease is selected from one or more of the following:
(1) the stable structure corresponding to the substance that causes pomelo intolerance in the organism;
(2) the stable structure corresponding to the substance of the organism itself and related to pomelo intolerance disease;
(3) the stable structure corresponding to the substance that inhibits the structure that causes pomelo intolerance disease;
(4) the stable structure corresponding to the substance containing a substance of any one of (1) to (3) above.

The substance for treating and/or preventing pomelo intolerance disease designed according to the above-mentioned method.

The preparation method of the above-mentioned substance for treating and/or preventing pomelo intolerance disease, wherein the stable structure corresponding to the substance related to pomelo intolerance disease is prepared by a high-temperature carbonization method.

Use of the above-mentioned substance for treating and/or preventing pomelo intolerance disease in the preparation of medicines, health products, foods, and food additives.

A method for designing substances for the treatment and/or prevention of lemon intolerance disease, wherein the stable structure corresponding to the substance related to lemon intolerance disease is used as the substance for treating and/or preventing lemon intolerance disease.

Preferably, the stable structure corresponding to the substance refers to the structure of said substance that exists in a solid state at a high temperature of 500 °C or higher.

Preferably, the stable structure corresponding to the substance related to lemon intolerance disease is selected from one or more of the following:
(1) the stable structure corresponding to the substance that causes lemon intolerance in the organism;
(2) the stable structure corresponding to the substance of the organism itself and related to lemon intolerance disease;
(3) the stable structure corresponding to the substance that inhibits the structure that causes lemon intolerance disease;
(4) the stable structure corresponding to the substance containing a substance of any one of (1) to (3) above.

The substance for treating and/or preventing lemon intolerance disease designed according to the above-mentioned method.

The preparation method of the above-mentioned substance for treating and/or preventing lemon intolerance disease, wherein the stable structure corresponding to the substance related to lemon intolerance disease is prepared by a high-temperature carbonization method.

Use of the above-mentioned substance for treating and/or preventing lemon intolerance disease in the preparation of medicines, health products, foods, and food additives.

A method for designing substances for the treatment and/or prevention of olive intolerance disease, wherein the stable structure corresponding to the substance related to olive intolerance disease is used as the substance for treating and/or preventing olive intolerance disease.

Preferably, the stable structure corresponding to the substance refers to the structure of said substance that exists in a solid state at a high temperature of 500 °C or higher.

Preferably, the stable structure corresponding to the substance related to olive intolerance disease is selected from one or more of the following:
(1) the stable structure corresponding to the substance that causes olive intolerance in the organism;
(2) the stable structure corresponding to the substance of the organism itself and related to olive intolerance disease;
(3) the stable structure corresponding to the substance that inhibits the structure that causes olive intolerance disease;
(4) the stable structure corresponding to the substance containing a substance of any one of (1) to (3) above.

The substance for treating and/or preventing olive intolerance disease designed according to the above-mentioned method.

The preparation method of the above-mentioned substance for treating and/or preventing olive intolerance disease, wherein the stable structure corresponding to the substance related to olive intolerance disease is prepared by a high-temperature carbonization method.

Use of the above-mentioned substance for treating and/or preventing olive intolerance disease in the preparation of medicines, health products, foods, and food additives.

A method for designing substances for the treatment and/or prevention of tangerine intolerance disease, wherein the stable structure corresponding to the substance related to tangerine intolerance disease is used as the substance for treating and/or preventing tangerine intolerance disease.

Preferably, the stable structure corresponding to the substance refers to the structure of said substance that exists in a solid state at a high temperature of 500 °C or higher.

Preferably, the stable structure corresponding to the substance related to tangerine intolerance disease is selected from one or more of the following:
(1) the stable structure corresponding to the substance that causes tangerine intolerance in the organism;
(2) the stable structure corresponding to the substance of the organism itself and related to tangerine intolerance disease;
(3) the stable structure corresponding to the substance that inhibits the structure that causes tangerine intolerance disease;
(4) the stable structure corresponding to the substance containing a substance of any one of (1) to (3) above.

The substance for treating and/or preventing tangerine intolerance disease designed according to the above-mentioned method.

The preparation method of the above-mentioned substance for treating and/or preventing tangerine intolerance disease, wherein the stable structure corresponding to the substance related to tangerine intolerance disease is prepared by a high-temperature carbonization method.

Use of the above-mentioned substance for treating and/or preventing tangerine intolerance disease in the preparation of medicines, health products, foods, and food additives.

A method for designing substances for the treatment and/or prevention of peach intolerance disease, wherein the stable structure corresponding to the substance related to peach intolerance disease is used as the substance for treating and/or preventing peach intolerance disease.

Preferably, the stable structure corresponding to the substance refers to the structure of said substance that exists in a solid state at a high temperature of 500 °C or higher.

Preferably, the stable structure corresponding to the substance related to peach intolerance disease is selected from one or more of the following:
(1) the stable structure corresponding to the substance that causes peach intolerance in the organism;
(2) the stable structure corresponding to the substance of the organism itself and related to peach intolerance disease;
(3) the stable structure corresponding to the substance that inhibits the structure that causes peach intolerance disease;
(4) the stable structure corresponding to the substance containing a substance of any one of (1) to (3) above.

The substance for treating and/or preventing peach intolerance disease designed according to the above-mentioned method.

The preparation method of the above-mentioned substance for treating and/or preventing peach intolerance disease, wherein the stable structure corresponding to the substance related to peach intolerance disease is prepared by a high-temperature carbonization method.

Use of the above-mentioned substance for treating and/or preventing peach intolerance disease in the preparation of medicines, health products, foods, and food additives.

A method for designing substances for the treatment and/or prevention of pineapple intolerance disease, wherein the stable structure corresponding to the substance related to pineapple intolerance disease is used as the substance for treating and/or preventing pineapple intolerance disease.

Preferably, the stable structure corresponding to the substance refers to the structure of said substance that exists in a solid state at a high temperature of 500 °C or higher.

Preferably, the stable structure corresponding to the substance related to pineapple intolerance disease is selected from one or more of the following:
(1) the stable structure corresponding to the substance that causes pineapple intolerance in the organism;
(2) the stable structure corresponding to the substance of the organism itself and related to pineapple intolerance disease;
(3) the stable structure corresponding to the substance that inhibits the structure that causes pineapple intolerance disease;
(4) the stable structure corresponding to the substance containing a substance of any one of (1) to (3) above.

The substance for treating and/or preventing pineapple intolerance disease designed according to the above-mentioned method.

The preparation method of the above-mentioned substance for treating and/or preventing pineapple intolerance disease, wherein the stable structure corresponding to the substance related to pineapple intolerance disease is prepared by a high-temperature carbonization method.

Use of the above-mentioned substance for treating and/or preventing pineapple intolerance disease in the preparation of medicines, health products, foods, and food additives.

A method for designing substances for the treatment and/or prevention of strawberry intolerance disease, wherein the stable structure corresponding to the substance related to strawberry intolerance disease is used as the substance for treating and/or preventing strawberry intolerance disease.

Preferably, the stable structure corresponding to the substance refers to the structure of said substance that exists in a solid state at a high temperature of 500 °C or higher.

Preferably, the stable structure corresponding to the substance related to strawberry intolerance disease is selected from one or more of the following:
(1) the stable structure corresponding to the substance that causes strawberry intolerance in the organism;
(2) the stable structure corresponding to the substance of the organism itself and related to strawberry intolerance disease;
(3) the stable structure corresponding to the substance that inhibits the structure that causes strawberry intolerance disease;
(4) the stable structure corresponding to the substance containing a substance of any one of (1) to (3) above.

The substance for treating and/or preventing strawberry intolerance disease designed according to the above-mentioned method.

The preparation method of the above-mentioned substance for treating and/or preventing strawberry intolerance disease, wherein the stable structure corresponding to the substance related to strawberry intolerance disease is prepared by a high-temperature carbonization method.

Use of the above-mentioned substance for treating and/or preventing strawberry intolerance disease in the preparation of medicines, health products, foods, and food additives.

A method for designing substances for the treatment and/or prevention of beef intolerance disease, wherein the stable structure corresponding to the substance related to beef intolerance disease is used as the substance for treating and/or preventing beef intolerance disease.

Preferably, the stable structure corresponding to the substance refers to the structure of said substance that exists in a solid state at a high temperature of 500 °C or higher.

Preferably, the stable structure corresponding to the substance related to beef intolerance disease is selected from one or more of the following:
(1) the stable structure corresponding to the substance that causes beef intolerance in the organism;
(2) the stable structure corresponding to the substance of the organism itself and related to beef intolerance disease;
(3) the stable structure corresponding to the substance that inhibits the structure that causes beef intolerance disease;
(4) the stable structure corresponding to the substance containing a substance of any one of (1) to (3) above.

The substance for treating and/or preventing beef intolerance disease designed according to the above-mentioned method.

The preparation method of the above-mentioned substance for treating and/or preventing beef intolerance disease, wherein the stable structure corresponding to the substance related to beef intolerance disease is prepared by a high-temperature carbonization method.

Use of the above-mentioned substance for treating and/or preventing beef intolerance disease in the preparation of medicines, health products, foods, and food additives.

A method for designing substances for the treatment and/or prevention of chicken intolerance disease, wherein the stable structure corresponding to the substance related to chicken intolerance disease is used as the substance for treating and/or preventing chicken intolerance disease.

Preferably, the stable structure corresponding to the substance refers to the structure of said substance that exists in a solid state at a high temperature of 500 °C or higher.

Preferably, the stable structure corresponding to the substance related to chicken intolerance disease is selected from one or more of the following:
(1) the stable structure corresponding to the substance that causes chicken intolerance in the organism;
(2) the stable structure corresponding to the substance of the organism itself and related to chicken intolerance disease;
(3) the stable structure corresponding to the substance that inhibits the structure that causes chicken intolerance disease;
(4) the stable structure corresponding to the substance containing a substance of any one of (1) to (3) above.

The substance for treating and/or preventing chicken intolerance disease designed according to the above-mentioned method.

The preparation method of the above-mentioned substance for treating and/or preventing chicken intolerance disease, wherein the stable structure corresponding to the substance related to chicken intolerance disease is prepared by a high-temperature carbonization method.

Use of the above-mentioned substance for treating and/or preventing chicken intolerance disease in the preparation of medicines, health products, foods, and food additives.

A method for designing substances for the treatment and/or prevention of egg intolerance disease, wherein the stable structure corresponding to the substance related to egg intolerance disease is used as the substance for treating and/or preventing egg intolerance disease.

Preferably, the stable structure corresponding to the substance refers to the structure of said substance that exists in a solid state at a high temperature of 500 °C or higher.

Preferably, the stable structure corresponding to the substance related to egg intolerance disease is selected from one or more of the following:
(1) the stable structure corresponding to the substance that causes egg intolerance in the organism;
(2) the stable structure corresponding to the substance of the organism itself and related to egg intolerance disease;
(3) the stable structure corresponding to the substance that inhibits the structure that causes egg intolerance disease;
(4) the stable structure corresponding to the substance containing a substance of any one of (1) to (3) above.

The substance for treating and/or preventing egg intolerance disease designed according to the above-mentioned method.

The preparation method of the above-mentioned substance for treating and/or preventing egg intolerance disease, wherein the stable structure corresponding to the substance related to egg intolerance disease is prepared by a high-temperature carbonization method.

Use of the above-mentioned substance for treating and/or preventing egg intolerance disease in the preparation of medicines, health products, foods, and food additives.

A method for designing substances for the treatment and/or prevention of pork intolerance disease, wherein the stable structure corresponding to the substance related to pork intolerance disease is used as the substance for treating and/or preventing pork intolerance disease.

Preferably, the stable structure corresponding to the substance refers to the structure of said substance that exists in a solid state at a high temperature of 500 °C or higher.

Preferably, the stable structure corresponding to the substance related to pork intolerance disease is selected from one or more of the following:
(1) the stable structure corresponding to the substance that causes pork intolerance in the organism;
(2) the stable structure corresponding to the substance of the organism itself and related to pork intolerance disease;
(3) the stable structure corresponding to the substance that inhibits the structure that causes pork intolerance disease;
(4) the stable structure corresponding to the substance containing a substance of any one of (1) to (3) above.

The substance for treating and/or preventing pork intolerance disease designed according to the above-mentioned method.

The preparation method of the above-mentioned substance for treating and/or preventing pork intolerance disease, wherein the stable structure corresponding to the substance related to pork intolerance disease is prepared by a high-temperature carbonization method.

Use of the above-mentioned substance for treating and/or preventing pork intolerance disease in the preparation of medicines, health products, foods, and food additives.

A method for designing substances for the treatment and/or prevention of turkey intolerance disease, wherein the stable structure corresponding to the substance related to turkey intolerance disease is used as the substance for treating and/or preventing turkey intolerance disease.

Preferably, the stable structure corresponding to the substance refers to the structure of said substance that exists in a solid state at a high temperature of 500 °C or higher.

Preferably, the stable structure corresponding to the substance related to turkey intolerance disease is selected from one or more of the following:
(1) the stable structure corresponding to the substance that causes turkey intolerance in the organism;
(2) the stable structure corresponding to the substance of the organism itself and related to turkey intolerance disease;
(3) the stable structure corresponding to the substance that inhibits the structure that causes turkey intolerance disease;
(4) the stable structure corresponding to the substance containing a substance of any one of (1) to (3) above.

The substance for treating and/or preventing turkey intolerance disease designed according to the above-mentioned method.

The preparation method of the above-mentioned substance for treating and/or preventing turkey intolerance disease, wherein the stable structure corresponding to the substance related to turkey intolerance disease is prepared by a high-temperature carbonization method.

Use of the above-mentioned substance for treating and/or preventing turkey intolerance disease in the preparation of medicines, health products, foods, and food additives.

A method for designing substances for the treatment and/or prevention of cabbage intolerance disease, wherein the stable structure corresponding to the substance related to cabbage intolerance disease is used as the substance for treating and/or preventing cabbage intolerance disease.

Preferably, the stable structure corresponding to the substance refers to the structure of said substance that exists in a solid state at a high temperature of 500 °C or higher.

Preferably, the stable structure corresponding to the substance related to cabbage intolerance disease is selected from one or more of the following:
(1) the stable structure corresponding to the substance that causes cabbage intolerance in the organism;
(2) the stable structure corresponding to the substance of the organism itself and related to cabbage intolerance disease;
(3) the stable structure corresponding to the substance that inhibits the structure that causes cabbage intolerance disease;
(4) the stable structure corresponding to the substance containing a substance of any one of (1) to (3) above.

The substance for treating and/or preventing cabbage intolerance disease designed according to the above-mentioned method.

The preparation method of the above-mentioned substance for treating and/or preventing cabbage intolerance disease, wherein the stable structure corresponding to the substance related to cabbage intolerance disease is prepared by a high-temperature carbonization method.

Use of the above-mentioned substance for treating and/or preventing cabbage intolerance disease in the preparation of medicines, health products, foods, and food additives.

A method for designing substances for the treatment and/or prevention of crown daisy intolerance disease, wherein the stable structure corresponding to the substance related to crown daisy intolerance disease is used as the substance for treating and/or preventing crown daisy intolerance disease.

Preferably, the stable structure corresponding to the substance refers to the structure of said substance that exists in a solid state at a high temperature of 500 °C or higher.

Preferably, the stable structure corresponding to the substance related to crown daisy intolerance disease is selected from one or more of the following:
(1) the stable structure corresponding to the substance that causes crown daisy intolerance in the organism;
(2) the stable structure corresponding to the substance of the organism itself and related to crown daisy intolerance disease;
(3) the stable structure corresponding to the substance that inhibits the structure that causes crown daisy intolerance disease;
(4) the stable structure corresponding to the substance containing a substance of any one of (1) to (3) above.

The substance for treating and/or preventing crown daisy intolerance disease designed according to the above-mentioned method.

The preparation method of the above-mentioned substance for treating and/or preventing crown daisy intolerance disease, wherein the stable structure corresponding to the substance related to crown daisy intolerance disease is prepared by a high-temperature carbonization method.

Use of the above-mentioned substance for treating and/or preventing crown daisy intolerance disease in the preparation of medicines, health products, foods, and food additives.

A method for designing substances for the treatment and/or prevention of carrot intolerance disease, wherein the stable structure corresponding to the substance related to carrot intolerance disease is used as the substance for treating and/or preventing carrot intolerance disease.

Preferably, the stable structure corresponding to the substance refers to the structure of said substance that exists in a solid state at a high temperature of 500 °C or higher.

Preferably, the stable structure corresponding to the substance related to carrot intolerance disease is selected from one or more of the following:
(1) the stable structure corresponding to the substance that causes carrot intolerance in the organism;
(2) the stable structure corresponding to the substance of the organism itself and related to carrot intolerance disease;
(3) the stable structure corresponding to the substance that inhibits the structure that causes carrot intolerance disease;
(4) the stable structure corresponding to the substance containing a substance of any one of (1) to (3) above.

The substance for treating and/or preventing carrot intolerance disease designed according to the above-mentioned method.

The preparation method of the above-mentioned substance for treating and/or preventing carrot intolerance disease, wherein the stable structure corresponding to the substance related to carrot intolerance disease is prepared by a high-temperature carbonization method.

Use of the above-mentioned substance for treating and/or preventing carrot intolerance disease in the preparation of medicines, health products, foods, and food additives.

A method for designing substances for the treatment and/or prevention of cucumber intolerance disease, wherein the stable structure corresponding to the substance related to cucumber intolerance disease is used as the substance for treating and/or preventing cucumber intolerance disease.

Preferably, the stable structure corresponding to the substance refers to the structure of said substance that exists in a solid state at a high temperature of 500 °C or higher.

Preferably, the stable structure corresponding to the substance related to cucumber intolerance disease is selected from one or more of the following:
(1) the stable structure corresponding to the substance that causes cucumber intolerance in the organism;
(2) the stable structure corresponding to the substance of the organism itself and related to cucumber intolerance disease;
(3) the stable structure corresponding to the substance that inhibits the structure that causes cucumber intolerance disease;
(4) the stable structure corresponding to the substance containing a substance of any one of (1) to (3) above.

The substance for treating and/or preventing cucumber intolerance disease designed according to the above-mentioned method.

The preparation method of the above-mentioned substance for treating and/or preventing cucumber intolerance disease, wherein the stable structure corresponding to the substance related to cucumber intolerance disease is prepared by a high-temperature carbonization method.

Use of the above-mentioned substance for treating and/or preventing cucumber intolerance disease in the preparation of medicines, health products, foods, and food additives.

A method for designing substances for the treatment and/or prevention of eggplant intolerance disease, wherein the stable structure corresponding to the substance related to eggplant intolerance disease is used as the substance for treating and/or preventing eggplant intolerance disease.

Preferably, the stable structure corresponding to the substance refers to the structure of said substance that exists in a solid state at a high temperature of 500 °C or higher.

Preferably, the stable structure corresponding to the substance related to eggplant intolerance disease is selected from one or more of the following:
(1) the stable structure corresponding to the substance that causes eggplant intolerance in the organism;
(2) the stable structure corresponding to the substance of the organism itself and related to eggplant intolerance disease;
(3) the stable structure corresponding to the substance that inhibits the structure that causes eggplant intolerance disease;
(4) the stable structure corresponding to the substance containing a substance of any one of (1) to (3) above.

The substance for treating and/or preventing eggplant intolerance disease designed according to the above-mentioned method.

The preparation method of the above-mentioned substance for treating and/or preventing eggplant intolerance disease, wherein the stable structure corresponding to the substance related to eggplant intolerance disease is prepared by a high-temperature carbonization method.

Use of the above-mentioned substance for treating and/or preventing eggplant intolerance disease in the preparation of medicines, health products, foods, and food additives.

A method for designing substances for the treatment and/or prevention of cauliflower intolerance disease, wherein the stable structure corresponding to the substance related to cauliflower intolerance disease is used as the substance for treating and/or preventing cauliflower intolerance disease.

Preferably, the stable structure corresponding to the substance refers to the structure of said substance that exists in a solid state at a high temperature of 500 °C or higher.

Preferably, the stable structure corresponding to the substance related to cauliflower intolerance disease is selected from one or more of the following:
(1) the stable structure corresponding to the substance that causes cauliflower intolerance in the organism;
(2) the stable structure corresponding to the substance of the organism itself and related to cauliflower intolerance disease;
(3) the stable structure corresponding to the substance that inhibits the structure that causes cauliflower intolerance disease;
(4) the stable structure corresponding to the substance containing a substance of any one of (1) to (3) above.

The substance for treating and/or preventing cauliflower intolerance disease designed according to the above-mentioned method.

The preparation method of the above-mentioned substance for treating and/or preventing cauliflower intolerance disease, wherein the stable structure corresponding to the substance related to cauliflower intolerance disease is prepared by a high-temperature carbonization method.

Use of the above-mentioned substance for treating and/or preventing cauliflower intolerance disease in the preparation of medicines, health products, foods, and food additives.

A method for designing substances for the treatment and/or prevention of celery intolerance disease, wherein the stable structure corresponding to the substance related to celery intolerance disease is used as the substance for treating and/or preventing celery intolerance disease.

Preferably, the stable structure corresponding to the substance refers to the structure of said substance that exists in a solid state at a high temperature of 500 °C or higher.

Preferably, the stable structure corresponding to the substance related to celery intolerance disease is selected from one or more of the following:
(1) the stable structure corresponding to the substance that causes celery intolerance in the organism;
(2) the stable structure corresponding to the substance of the organism itself and related to celery intolerance disease;
(3) the stable structure corresponding to the substance that inhibits the structure that causes celery intolerance disease;
(4) the stable structure corresponding to the substance containing a substance of any one of (1) to (3) above.

The substance for treating and/or preventing celery intolerance disease designed according to the above-mentioned method.

The preparation method of the above-mentioned substance for treating and/or preventing celery intolerance disease, wherein the stable structure corresponding to the substance related to celery intolerance disease is prepared by a high-temperature carbonization method.

Use of the above-mentioned substance for treating and/or preventing celery intolerance disease in the preparation of medicines, health products, foods, and food additives.

A method for designing substances for the treatment and/or prevention of peas intolerance disease, wherein the stable structure corresponding to the substance related to peas intolerance disease is used as the substance for treating and/or preventing peas intolerance disease.

Preferably, the stable structure corresponding to the substance refers to the structure of said substance that exists in a solid state at a high temperature of 500 °C or higher.

Preferably, the stable structure corresponding to the substance related to peas intolerance disease is selected from one or more of the following:
(1) the stable structure corresponding to the substance that causes peas intolerance in the organism;
(2) the stable structure corresponding to the substance of the organism itself and related to peas intolerance disease;
(3) the stable structure corresponding to the substance that inhibits the structure that causes peas intolerance disease;
(4) the stable structure corresponding to the substance containing a substance of any one of (1) to (3) above.

The substance for treating and/or preventing peas intolerance disease designed according to the above-mentioned method.

The preparation method of the above-mentioned substance for treating and/or preventing peas intolerance disease, wherein the stable structure corresponding to the substance related to peas intolerance disease is prepared by a high-temperature carbonization method.

Use of the above-mentioned substance for treating and/or preventing peas intolerance disease in the preparation of medicines, health products, foods, and food additives.

A method for designing substances for the treatment and/or prevention of green pepper intolerance disease, wherein the stable structure corresponding to the substance related to green pepper intolerance disease is used as the substance for treating and/or preventing green pepper intolerance disease.

Preferably, the stable structure corresponding to the substance refers to the structure of said substance that exists in a solid state at a high temperature of 500 °C or higher.

Preferably, the stable structure corresponding to the substance related to green pepper intolerance disease is selected from one or more of the following:
(1) the stable structure corresponding to the substance that causes green pepper intolerance in the organism;
(2) the stable structure corresponding to the substance of the organism itself and related to green pepper intolerance disease;
(3) the stable structure corresponding to the substance that inhibits the structure that causes green pepper intolerance disease;
(4) the stable structure corresponding to the substance containing a substance of any one of (1) to (3) above.

The substance for treating and/or preventing green pepper intolerance disease designed according to the above-mentioned method.

The preparation method of the above-mentioned substance for treating and/or preventing green pepper intolerance disease, wherein the stable structure corresponding to the substance related to green pepper intolerance disease is prepared by a high-temperature carbonization method.

Use of the above-mentioned substance for treating and/or preventing green pepper intolerance disease in the preparation of medicines, health products, foods, and food additives.

A method for designing substances for the treatment and/or prevention of garlic intolerance disease, wherein the stable structure corresponding to the substance related to garlic intolerance disease is used as the substance for treating and/or preventing garlic intolerance disease.

Preferably, the stable structure corresponding to the substance refers to the structure of said substance that exists in a solid state at a high temperature of 500 °C or higher.

Preferably, the stable structure corresponding to the substance related to garlic intolerance disease is selected from one or more of the following:
(1) the stable structure corresponding to the substance that causes garlic intolerance in the organism;
(2) the stable structure corresponding to the substance of the organism itself and related to garlic intolerance disease;
(3) the stable structure corresponding to the substance that inhibits the structure that causes garlic intolerance disease;
(4) the stable structure corresponding to the substance containing a substance of any one of (1) to (3) above.

The substance for treating and/or preventing garlic intolerance disease designed according to the above-mentioned method.

The preparation method of the above-mentioned substance for treating and/or preventing garlic intolerance disease, wherein the stable structure corresponding to the substance related to garlic intolerance disease is prepared by a high-temperature carbonization method.

Use of the above-mentioned substance for treating and/or preventing garlic intolerance disease in the preparation of medicines, health products, foods, and food additives.

A method for designing substances for the treatment and/or prevention of red pepper intolerance disease, wherein the stable structure corresponding to the substance related to red pepper intolerance disease is used as the substance for treating and/or preventing red pepper intolerance disease.

Preferably, the stable structure corresponding to the substance refers to the structure of said substance that exists in a solid state at a high temperature of 500 °C or higher.

Preferably, the stable structure corresponding to the substance related to red pepper intolerance disease is selected from one or more of the following:
(1) the stable structure corresponding to the substance that causes red pepper intolerance in the organism;
(2) the stable structure corresponding to the substance of the organism itself and related to red pepper intolerance disease;
(3) the stable structure corresponding to the substance that inhibits the structure that causes red pepper intolerance disease;
(4) the stable structure corresponding to the substance containing a substance of any one of (1) to (3) above.

The substance for treating and/or preventing red pepper intolerance disease designed according to the above-mentioned method.

The preparation method of the above-mentioned substance for treating and/or preventing red pepper intolerance disease, wherein the stable structure corresponding to the substance related to red pepper intolerance disease is prepared by a high-temperature carbonization method.

Use of the above-mentioned substance for treating and/or preventing red pepper intolerance disease in the preparation of medicines, health products, foods, and food additives.

A method for designing substances for the treatment and/or prevention of lettuce intolerance disease, wherein the stable structure corresponding to the substance related to lettuce intolerance disease is used as the substance for treating and/or preventing lettuce intolerance disease.

Preferably, the stable structure corresponding to the substance refers to the structure of said substance that exists in a solid state at a high temperature of 500 °C or higher.

Preferably, the stable structure corresponding to the substance related to lettuce intolerance disease is selected from one or more of the following:
(1) the stable structure corresponding to the substance that causes lettuce intolerance in the organism;
(2) the stable structure corresponding to the substance of the organism itself and related to lettuce intolerance disease;
(3) the stable structure corresponding to the substance that inhibits the structure that causes lettuce intolerance disease;
(4) the stable structure corresponding to the substance containing a substance of any one of (1) to (3) above.

The substance for treating and/or preventing lettuce intolerance disease designed according to the above-mentioned method.

The preparation method of the above-mentioned substance for treating and/or preventing lettuce intolerance disease, wherein the stable structure corresponding to the substance related to lettuce intolerance disease is prepared by a high-temperature carbonization method.

Use of the above-mentioned substance for treating and/or preventing lettuce intolerance disease in the preparation of medicines, health products, foods, and food additives.

A method for designing substances for the treatment and/or prevention of shepherd's purse seed intolerance disease, wherein the stable structure corresponding to the substance related to shepherd's purse seed intolerance disease is used as the substance for treating and/or preventing shepherd's purse seed intolerance disease.

Preferably, the stable structure corresponding to the substance refers to the structure of said substance that exists in a solid state at a high temperature of 500 °C or higher.

Preferably, the stable structure corresponding to the substance related to shepherd's purse seed intolerance disease is selected from one or more of the following:
(1) the stable structure corresponding to the substance that causes shepherd's purse seed intolerance in the organism;
(2) the stable structure corresponding to the substance of the organism itself and related to shepherd's purse seed intolerance disease;
(3) the stable structure corresponding to the substance that inhibits the structure that causes shepherd's purse seed intolerance disease;
(4) the stable structure corresponding to the substance containing a substance of any one of (1) to (3) above.

The substance for treating and/or preventing shepherd's purse seed intolerance disease designed according to the above-mentioned method.

The preparation method of the above-mentioned substance for treating and/or preventing shepherd's purse seed intolerance disease, wherein the stable structure corresponding to the substance related to shepherd's purse seed intolerance disease is prepared by a high-temperature carbonization method.

Use of the above-mentioned substance for treating and/or preventing shepherd's purse seed intolerance disease in the preparation of medicines, health products, foods, and food additives.

A method for designing substances for the treatment and/or prevention of onion intolerance disease, wherein the stable structure corresponding to the substance related to onion intolerance disease is used as the substance for treating and/or preventing onion intolerance disease.

Preferably, the stable structure corresponding to the substance refers to the structure of said substance that exists in a solid state at a high temperature of 500 °C or higher.

Preferably, the stable structure corresponding to the substance related to onion intolerance disease is selected from one or more of the following:
(1) the stable structure corresponding to the substance that causes onion intolerance in the organism;
(2) the stable structure corresponding to the substance of the organism itself and related to onion intolerance disease;
(3) the stable structure corresponding to the substance that inhibits the structure that causes onion intolerance disease;
(4) the stable structure corresponding to the substance containing a substance of any one of (1) to (3) above.

The substance for treating and/or preventing onion intolerance disease designed according to the above-mentioned method.

The preparation method of the above-mentioned substance for treating and/or preventing onion intolerance disease, wherein the stable structure corresponding to the substance related to onion intolerance disease is prepared by a high-temperature carbonization method.

Use of the above-mentioned substance for treating and/or preventing onion intolerance disease in the preparation of medicines, health products, foods, and food additives.

A method for designing substances for the treatment and/or prevention of parsley intolerance disease, wherein the stable structure corresponding to the substance related to parsley intolerance disease is used as the substance for treating and/or preventing parsley intolerance disease.

Preferably, the stable structure corresponding to the substance refers to the structure of said substance that exists in a solid state at a high temperature of 500 °C or higher.

Preferably, the stable structure corresponding to the substance related to parsley intolerance disease is selected from one or more of the following:
(1) the stable structure corresponding to the substance that causes parsley intolerance in the organism;
(2) the stable structure corresponding to the substance of the organism itself and related to parsley intolerance disease;
(3) the stable structure corresponding to the substance that inhibits the structure that causes parsley intolerance disease;
(4) the stable structure corresponding to the substance containing a substance of any one of (1) to (3) above.

The substance for treating and/or preventing parsley intolerance disease designed according to the above-mentioned method.

The preparation method of the above-mentioned substance for treating and/or preventing parsley intolerance disease, wherein the stable structure corresponding to the substance related to parsley intolerance disease is prepared by a high-temperature carbonization method.

Use of the above-mentioned substance for treating and/or preventing parsley intolerance disease in the preparation of medicines, health products, foods, and food additives.

A method for designing substances for the treatment and/or prevention of okra intolerance disease, wherein the stable structure corresponding to the substance related to okra intolerance disease is used as the substance for treating and/or preventing okra intolerance disease.

Preferably, the stable structure corresponding to the substance refers to the structure of said substance that exists in a solid state at a high temperature of 500 °C or higher.

Preferably, the stable structure corresponding to the substance related to okra intolerance disease is selected from one or more of the following:
(1) the stable structure corresponding to the substance that causes okra intolerance in the organism;
(2) the stable structure corresponding to the substance of the organism itself and related to okra intolerance disease;
(3) the stable structure corresponding to the substance that inhibits the structure that causes okra intolerance disease;
(4) the stable structure corresponding to the substance containing a substance of any one of (1) to (3) above.

The substance for treating and/or preventing okra intolerance disease designed according to the above-mentioned method.

The preparation method of the above-mentioned substance for treating and/or preventing okra intolerance disease, wherein the stable structure corresponding to the substance related to okra intolerance disease is prepared by a high-temperature carbonization method.

Use of the above-mentioned substance for treating and/or preventing okra intolerance disease in the preparation of medicines, health products, foods, and food additives.

A method for designing substances for the treatment and/or prevention of potato intolerance disease, wherein the stable structure corresponding to the substance related to potato intolerance disease is used as the substance for treating and/or preventing potato intolerance disease.

Preferably, the stable structure corresponding to the substance refers to the structure of said substance that exists in a solid state at a high temperature of 500 °C or higher.

Preferably, the stable structure corresponding to the substance related to potato intolerance disease is selected from one or more of the following:
(1) the stable structure corresponding to the substance that causes potato intolerance in the organism;
(2) the stable structure corresponding to the substance of the organism itself and related to potato intolerance disease;
(3) the stable structure corresponding to the substance that inhibits the structure that causes potato intolerance disease;
(4) the stable structure corresponding to the substance containing a substance of any one of (1) to (3) above.

The substance for treating and/or preventing potato intolerance disease designed according to the above-mentioned method.

The preparation method of the above-mentioned substance for treating and/or preventing potato intolerance disease, wherein the stable structure corresponding to the substance related to potato intolerance disease is prepared by a high-temperature carbonization method.

Use of the above-mentioned substance for treating and/or preventing potato intolerance disease in the preparation of medicines, health products, foods, and food additives.

A method for designing substances for the treatment and/or prevention of mushroom intolerance disease, wherein the stable structure corresponding to the substance related to mushroom intolerance disease is used as the substance for treating and/or preventing mushroom intolerance disease.

Preferably, the stable structure corresponding to the substance refers to the structure of said substance that exists in a solid state at a high temperature of 500 °C or higher.

Preferably, the stable structure corresponding to the substance related to mushroom intolerance disease is selected from one or more of the following:
(1) the stable structure corresponding to the substance that causes mushroom intolerance in the organism;
(2) the stable structure corresponding to the substance of the organism itself and related to mushroom intolerance disease;
(3) the stable structure corresponding to the substance that inhibits the structure that causes mushroom intolerance disease;
(4) the stable structure corresponding to the substance containing a substance of any one of (1) to (3) above.

The substance for treating and/or preventing mushroom intolerance disease designed according to the above-mentioned method.

The preparation method of the above-mentioned substance for treating and/or preventing mushroom intolerance disease, wherein the stable structure corresponding to the substance related to mushroom intolerance disease is prepared by a high-temperature carbonization method.

Use of the above-mentioned substance for treating and/or preventing mushroom intolerance disease in the preparation of medicines, health products, foods, and food additives.

A method for designing substances for the treatment and/or prevention of safflower seed intolerance disease, wherein the stable structure corresponding to the substance related to safflower seed intolerance disease is used as the substance for treating and/or preventing safflower seed intolerance disease.

Preferably, the stable structure corresponding to the substance refers to the structure of said substance that exists in a solid state at a high temperature of 500 °C or higher.

Preferably, the stable structure corresponding to the substance related to safflower seed intolerance disease is selected from one or more of the following:
(1) the stable structure corresponding to the substance that causes safflower seed intolerance in the organism;
(2) the stable structure corresponding to the substance of the organism itself and related to safflower seed intolerance disease;
(3) the stable structure corresponding to the substance that inhibits the structure that causes safflower seed intolerance disease;
(4) the stable structure corresponding to the substance containing a substance of any one of (1) to (3) above.

The substance for treating and/or preventing safflower seed intolerance disease designed according to the above-mentioned method.

The preparation method of the above-mentioned substance for treating and/or preventing safflower seed intolerance disease, wherein the stable structure corresponding to the substance related to safflower seed intolerance disease is prepared by a high-temperature carbonization method.

Use of the above-mentioned substance for treating and/or preventing safflower seed intolerance disease in the preparation of medicines, health products, foods, and food additives.

A method for designing substances for the treatment and/or prevention of spinach intolerance disease, wherein the stable structure corresponding to the substance related to spinach intolerance disease is used as the substance for treating and/or preventing spinach intolerance disease.

Preferably, the stable structure corresponding to the substance refers to the structure of said substance that exists in a solid state at a high temperature of 500 °C or higher.

Preferably, the stable structure corresponding to the substance related to spinach intolerance disease is selected from one or more of the following:
(1) the stable structure corresponding to the substance that causes spinach intolerance in the organism;
(2) the stable structure corresponding to the substance of the organism itself and related to spinach intolerance disease;
(3) the stable structure corresponding to the substance that inhibits the structure that causes spinach intolerance disease;
(4) the stable structure corresponding to the substance containing a substance of any one of (1) to (3) above.

The substance for treating and/or preventing spinach intolerance disease designed according to the above-mentioned method.

The preparation method of the above-mentioned substance for treating and/or preventing spinach intolerance disease, wherein the stable structure corresponding to the substance related to spinach intolerance disease is prepared by a high-temperature carbonization method.

Use of the above-mentioned substance for treating and/or preventing spinach intolerance disease in the preparation of medicines, health products, foods, and food additives.

A method for designing substances for the treatment and/or prevention of pumpkin intolerance disease, wherein the stable structure corresponding to the substance related to pumpkin intolerance disease is used as the substance for treating and/or preventing pumpkin intolerance disease.

Preferably, the stable structure corresponding to the substance refers to the structure of said substance that exists in a solid state at a high temperature of 500 °C or higher.

Preferably, the stable structure corresponding to the substance related to pumpkin intolerance disease is selected from one or more of the following:
(1) the stable structure corresponding to the substance that causes pumpkin intolerance in the organism;
(2) the stable structure corresponding to the substance of the organism itself and related to pumpkin intolerance disease;
(3) the stable structure corresponding to the substance that inhibits the structure that causes pumpkin intolerance disease;
(4) the stable structure corresponding to the substance containing a substance of any one of (1) to (3) above.

The substance for treating and/or preventing pumpkin intolerance disease designed according to the above-mentioned method.

The preparation method of the above-mentioned substance for treating and/or preventing pumpkin intolerance disease, wherein the stable structure corresponding to the substance related to pumpkin intolerance disease is prepared by a high-temperature carbonization method.

Use of the above-mentioned substance for treating and/or preventing pumpkin intolerance disease in the preparation of medicines, health products, foods, and food additives.

A method for designing substances for the treatment and/or prevention of sweet potato intolerance disease, wherein the stable structure corresponding to the substance related to sweet potato intolerance disease is used as the substance for treating and/or preventing sweet potato intolerance disease.

Preferably, the stable structure corresponding to the substance refers to the structure of said substance that exists in a solid state at a high temperature of 500 °C or higher.

Preferably, the stable structure corresponding to the substance related to sweet potato intolerance disease is selected from one or more of the following:
(1) the stable structure corresponding to the substance that causes sweet potato intolerance in the organism;
(2) the stable structure corresponding to the substance of the organism itself and related to sweet potato intolerance disease;
(3) the stable structure corresponding to the substance that inhibits the structure that causes sweet potato intolerance disease;
(4) the stable structure corresponding to the substance containing a substance of any one of (1) to (3) above.

The substance for treating and/or preventing sweet potato intolerance disease designed according to the above-mentioned method.

The preparation method of the above-mentioned substance for treating and/or preventing sweet potato intolerance disease, wherein the stable structure corresponding to the substance related to sweet potato intolerance disease is prepared by a high-temperature carbonization method.

Use of the above-mentioned substance for treating and/or preventing sweet potato intolerance disease in the preparation of medicines, health products, foods, and food additives.

A method for designing substances for the treatment and/or prevention of tamato intolerance disease, wherein the stable structure corresponding to the substance related to tamato intolerance disease is used as the substance for treating and/or preventing tamato intolerance disease.

Preferably, the stable structure corresponding to the substance refers to the structure of said substance that exists in a solid state at a high temperature of 500 °C or higher.

Preferably, the stable structure corresponding to the substance related to tamato intolerance disease is selected from one or more of the following:
(1) the stable structure corresponding to the substance that causes tamato intolerance in the organism;
(2) the stable structure corresponding to the substance of the organism itself and related to tamato intolerance disease;
(3) the stable structure corresponding to the substance that inhibits the structure that causes tamato intolerance disease;
(4) the stable structure corresponding to the substance containing a substance of any one of (1) to (3) above.

The substance for treating and/or preventing tamato intolerance disease designed according to the above-mentioned method.

The preparation method of the above-mentioned substance for treating and/or preventing tamato intolerance disease, wherein the stable structure corresponding to the substance related to tamato intolerance disease is prepared by a high-temperature carbonization method.

Use of the above-mentioned substance for treating and/or preventing tamato intolerance disease in the preparation of medicines, health products, foods, and food additives.

A method for designing substances for the treatment and/or prevention of butter intolerance disease, wherein the stable structure corresponding to the substance related to butter intolerance disease is used as the substance for treating and/or preventing butter intolerance disease.

Preferably, the stable structure corresponding to the substance refers to the structure of said substance that exists in a solid state at a high temperature of 500 °C or higher.

Preferably, the stable structure corresponding to the substance related to butter intolerance disease is selected from one or more of the following:
(1) the stable structure corresponding to the substance that causes butter intolerance in the organism;
(2) the stable structure corresponding to the substance of the organism itself and related to butter intolerance disease;
(3) the stable structure corresponding to the substance that inhibits the structure that causes butter intolerance disease;
(4) the stable structure corresponding to the substance containing a substance of any one of (1) to (3) above.

The substance for treating and/or preventing butter intolerance disease designed according to the above-mentioned method.

The preparation method of the above-mentioned substance for treating and/or preventing butter intolerance disease, wherein the stable structure corresponding to the substance related to butter intolerance disease is prepared by a high-temperature carbonization method.

Use of the above-mentioned substance for treating and/or preventing butter intolerance disease in the preparation of medicines, health products, foods, and food additives.

A method for designing substances for the treatment and/or prevention of cheddar cheese intolerance disease, wherein the stable structure corresponding to the substance related to cheddar cheese intolerance disease is used as the substance for treating and/or preventing cheddar cheese intolerance disease.

Preferably, the stable structure corresponding to the substance refers to the structure of said substance that exists in a solid state at a high temperature of 500 °C or higher.

Preferably, the stable structure corresponding to the substance related to cheddar cheese intolerance disease is selected from one or more of the following:
(1) the stable structure corresponding to the substance that causes cheddar cheese intolerance in the organism;
(2) the stable structure corresponding to the substance of the organism itself and related to cheddar cheese intolerance disease;
(3) the stable structure corresponding to the substance that inhibits the structure that causes cheddar cheese intolerance disease;
(4) the stable structure corresponding to the substance containing a substance of any one of (1) to (3) above.

The substance for treating and/or preventing cheddar cheese intolerance disease designed according to the above-mentioned method.

The preparation method of the above-mentioned substance for treating and/or preventing cheddar cheese intolerance disease, wherein the stable structure corresponding to the substance related to cheddar cheese intolerance disease is prepared by a high-temperature carbonization method.

Use of the above-mentioned substance for treating and/or preventing cheddar cheese intolerance disease in the preparation of medicines, health products, foods, and food additives.

A method for designing substances for the treatment and/or prevention of cottage cheese intolerance disease, wherein the stable structure corresponding to the substance related to cottage cheese intolerance disease is used as the substance for treating and/or preventing cottage cheese intolerance disease.

Preferably, the stable structure corresponding to the substance refers to the structure of said substance that exists in a solid state at a high temperature of 500 °C or higher.

Preferably, the stable structure corresponding to the substance related to cottage cheese intolerance disease is selected from one or more of the following:
(1) the stable structure corresponding to the substance that causes cottage cheese intolerance in the organism;
(2) the stable structure corresponding to the substance of the organism itself and related to cottage cheese intolerance disease;
(3) the stable structure corresponding to the substance that inhibits the structure that causes cottage cheese intolerance disease;
(4) the stable structure corresponding to the substance containing a substance of any one of (1) to (3) above.

The substance for treating and/or preventing cottage cheese intolerance disease designed according to the above-mentioned method.

The preparation method of the above-mentioned substance for treating and/or preventing cottage cheese intolerance disease, wherein the stable structure corresponding to the substance related to cottage cheese intolerance disease is prepared by a high-temperature carbonization method.

Use of the above-mentioned substance for treating and/or preventing cottage cheese intolerance disease in the preparation of medicines, health products, foods, and food additives.

A method for designing substances for the treatment and/or prevention of milk intolerance disease, wherein the stable structure corresponding to the substance related to milk intolerance disease is used as the substance for treating and/or preventing milk intolerance disease.

Preferably, the stable structure corresponding to the substance refers to the structure of said substance that exists in a solid state at a high temperature of 500 °C or higher.

Preferably, the stable structure corresponding to the substance related to milk intolerance disease is selected from one or more of the following:
(1) the stable structure corresponding to the substance that causes milk intolerance in the organism;
(2) the stable structure corresponding to the substance of the organism itself and related to milk intolerance disease;
(3) the stable structure corresponding to the substance that inhibits the structure that causes milk intolerance disease;
(4) the stable structure corresponding to the substance containing a substance of any one of (1) to (3) above.

The substance for treating and/or preventing milk intolerance disease designed according to the above-mentioned method.

The preparation method of the above-mentioned substance for treating and/or preventing milk intolerance disease, wherein the stable structure corresponding to the substance related to milk intolerance disease is prepared by a high-temperature carbonization method.

Use of the above-mentioned substance for treating and/or preventing milk intolerance disease in the preparation of medicines, health products, foods, and food additives.

A method for designing substances for the treatment and/or prevention of ewe's milk intolerance disease, wherein the stable structure corresponding to the substance related to ewe's milk intolerance disease is used as the substance for treating and/or preventing ewe's milk intolerance disease.

Preferably, the stable structure corresponding to the substance refers to the structure of said substance that exists in a solid state at a high temperature of 500 °C or higher.

Preferably, the stable structure corresponding to the substance related to ewe's milk intolerance disease is selected from one or more of the following:
(1) the stable structure corresponding to the substance that causes ewe's milk intolerance in the organism;
(2) the stable structure corresponding to the substance of the organism itself and related to ewe's milk intolerance disease;
(3) the stable structure corresponding to the substance that inhibits the structure that causes ewe's milk intolerance disease;
(4) the stable structure corresponding to the substance containing a substance of any one of (1) to (3) above.

The substance for treating and/or preventing ewe's milk intolerance disease designed according to the above-mentioned method.

The preparation method of the above-mentioned substance for treating and/or preventing ewe's milk intolerance disease, wherein the stable structure corresponding to the substance related to ewe's milk intolerance disease is prepared by a high-temperature carbonization method.

Use of the above-mentioned substance for treating and/or preventing ewe's milk intolerance disease in the preparation of medicines, health products, foods, and food additives.

A method for designing substances for the treatment and/or prevention of Swiss cheese intolerance disease, wherein the stable structure corresponding to the substance related to Swiss cheese intolerance disease is used as the substance for treating and/or preventing Swiss cheese intolerance disease.

Preferably, the stable structure corresponding to the substance refers to the structure of said substance that exists in a solid state at a high temperature of 500 °C or higher.

Preferably, the stable structure corresponding to the substance related to Swiss cheese intolerance disease is selected from one or more of the following:
(1) the stable structure corresponding to the substance that causes Swiss cheese intolerance in the organism;
(2) the stable structure corresponding to the substance of the organism itself and related to Swiss cheese intolerance disease;
(3) the stable structure corresponding to the substance that inhibits the structure that causes Swiss cheese intolerance disease;
(4) the stable structure corresponding to the substance containing a substance of any one of (1) to (3) above.

The substance for treating and/or preventing Swiss cheese intolerance disease designed according to the above-mentioned method.

The preparation method of the above-mentioned substance for treating and/or preventing Swiss cheese intolerance disease, wherein the stable structure corresponding to the substance related to Swiss cheese intolerance disease is prepared by a high-temperature carbonization method.

Use of the above-mentioned substance for treating and/or preventing Swiss cheese intolerance disease in the preparation of medicines, health products, foods, and food additives.

A method for designing substances for the treatment and/or prevention of yogurt intolerance disease, wherein the stable structure corresponding to the substance related to yogurt intolerance disease is used as the substance for treating and/or preventing yogurt intolerance disease.

Preferably, the stable structure corresponding to the substance refers to the structure of said substance that exists in a solid state at a high temperature of 500 °C or higher.

Preferably, the stable structure corresponding to the substance related to yogurt intolerance disease is selected from one or more of the following:
(1) the stable structure corresponding to the substance that causes yogurt intolerance in the organism;
(2) the stable structure corresponding to the substance of the organism itself and related to yogurt intolerance disease;
(3) the stable structure corresponding to the substance that inhibits the structure that causes yogurt intolerance disease;
(4) the stable structure corresponding to the substance containing a substance of any one of (1) to (3) above.

The substance for treating and/or preventing yogurt intolerance disease designed according to the above-mentioned method.

The preparation method of the above-mentioned substance for treating and/or preventing yogurt intolerance disease, wherein the stable structure corresponding to the substance related to yogurt intolerance disease is prepared by a high-temperature carbonization method.

Use of the above-mentioned substance for treating and/or preventing yogurt intolerance disease in the preparation of medicines, health products, foods, and food additives.

A method for designing substances for the treatment and/or prevention of America cheese intolerance disease, wherein the stable structure corresponding to the substance related to America cheese intolerance disease is used as the substance for treating and/or preventing America cheese intolerance disease.

Preferably, the stable structure corresponding to the substance refers to the structure of said substance that exists in a solid state at a high temperature of 500 °C or higher.

Preferably, the stable structure corresponding to the substance related to America cheese intolerance disease is selected from one or more of the following:
(1) the stable structure corresponding to the substance that causes America cheese intolerance in the organism;
(2) the stable structure corresponding to the substance of the organism itself and related to America cheese intolerance disease;
(3) the stable structure corresponding to the substance that inhibits the structure that causes America cheese intolerance disease;
(4) the stable structure corresponding to the substance containing a substance of any one of (1) to (3) above.

The substance for treating and/or preventing America cheese intolerance disease designed according to the above-mentioned method.

The preparation method of the above-mentioned substance for treating and/or preventing America cheese intolerance disease, wherein the stable structure corresponding to the substance related to America cheese intolerance disease is prepared by a high-temperature carbonization method.

Use of the above-mentioned substance for treating and/or preventing America cheese intolerance disease in the preparation of medicines, health products, foods, and food additives.

A method for designing substances for the treatment and/or prevention of honey intolerance disease, wherein the stable structure corresponding to the substance related to honey intolerance disease is used as the substance for treating and/or preventing honey intolerance disease.

Preferably, the stable structure corresponding to the substance refers to the structure of said substance that exists in a solid state at a high temperature of 500 °C or higher.

Preferably, the stable structure corresponding to the substance related to honey intolerance disease is selected from one or more of the following:
(1) the stable structure corresponding to the substance that causes honey intolerance in the organism;
(2) the stable structure corresponding to the substance of the organism itself and related to honey intolerance disease;
(3) the stable structure corresponding to the substance that inhibits the structure that causes honey intolerance disease;
(4) the stable structure corresponding to the substance containing a substance of any one of (1) to (3) above.

The substance for treating and/or preventing honey intolerance disease designed according to the above-mentioned method.

The preparation method of the above-mentioned substance for treating and/or preventing honey intolerance disease, wherein the stable structure corresponding to the substance related to honey intolerance disease is prepared by a high-temperature carbonization method.

Use of the above-mentioned substance for treating and/or preventing honey intolerance disease in the preparation of medicines, health products, foods, and food additives.

A method for designing substances for the treatment and/or prevention of tobacco intolerance disease, wherein the stable structure corresponding to the substance related to tobacco intolerance disease is used as the substance for treating and/or preventing tobacco intolerance disease.

Preferably, the stable structure corresponding to the substance refers to the structure of said substance that exists in a solid state at a high temperature of 500 °C or higher.

Preferably, the stable structure corresponding to the substance related to tobacco intolerance disease is selected from one or more of the following:
(1) the stable structure corresponding to the substance that causes tobacco intolerance in the organism;
(2) the stable structure corresponding to the substance of the organism itself and related to tobacco intolerance disease;
(3) the stable structure corresponding to the substance that inhibits the structure that causes tobacco intolerance disease;
(4) the stable structure corresponding to the substance containing a substance of any one of (1) to (3) above.

The substance for treating and/or preventing tobacco intolerance disease designed according to the above-mentioned method.

The preparation method of the above-mentioned substance for treating and/or preventing tobacco intolerance disease, wherein the stable structure corresponding to the substance related to tobacco intolerance disease is prepared by a high-temperature carbonization method.

Use of the above-mentioned substance for treating and/or preventing tobacco intolerance disease in the preparation of medicines, health products, foods, and food additives.

A method for designing substances for the treatment and/or prevention of cashew intolerance disease, wherein the stable structure corresponding to the substance related to cashew intolerance disease is used as the substance for treating and/or preventing cashew intolerance disease.

Preferably, the stable structure corresponding to the substance refers to the structure of said substance that exists in a solid state at a high temperature of 500 °C or higher.

Preferably, the stable structure corresponding to the substance related to cashew intolerance disease is selected from one or more of the following:
(1) the stable structure corresponding to the substance that causes cashew intolerance in the organism;
(2) the stable structure corresponding to the substance of the organism itself and related to cashew intolerance disease;
(3) the stable structure corresponding to the substance that inhibits the structure that causes cashew intolerance disease;
(4) the stable structure corresponding to the substance containing a substance of any one of (1) to (3) above.

The substance for treating and/or preventing cashew intolerance disease designed according to the above-mentioned method.

The preparation method of the above-mentioned substance for treating and/or preventing cashew intolerance disease, wherein the stable structure corresponding to the substance related to cashew intolerance disease is prepared by a high-temperature carbonization method.

Use of the above-mentioned substance for treating and/or preventing cashew intolerance disease in the preparation of medicines, health products, foods, and food additives.

A method for designing substances for the treatment and/or prevention of peanut intolerance disease, wherein the stable structure corresponding to the substance related to peanut intolerance disease is used as the substance for treating and/or preventing peanut intolerance disease.

Preferably, the stable structure corresponding to the substance refers to the structure of said substance that exists in a solid state at a high temperature of 500 °C or higher.

Preferably, the stable structure corresponding to the substance related to peanut intolerance disease is selected from one or more of the following:
(1) the stable structure corresponding to the substance that causes peanut intolerance in the organism;
(2) the stable structure corresponding to the substance of the organism itself and related to peanut intolerance disease;
(3) the stable structure corresponding to the substance that inhibits the structure that causes peanut intolerance disease;
(4) the stable structure corresponding to the substance containing a substance of any one of (1) to (3) above.

The substance for treating and/or preventing peanut intolerance disease designed according to the above-mentioned method.

The preparation method of the above-mentioned substance for treating and/or preventing peanut intolerance disease, wherein the stable structure corresponding to the substance related to peanut intolerance disease is prepared by a high-temperature carbonization method.

Use of the above-mentioned substance for treating and/or preventing peanut intolerance disease in the preparation of medicines, health products, foods, and food additives.

A method for designing substances for the treatment and/or prevention of sesame intolerance disease, wherein the stable structure corresponding to the substance related to sesame intolerance disease is used as the substance for treating and/or preventing sesame intolerance disease.

Preferably, the stable structure corresponding to the substance refers to the structure of said substance that exists in a solid state at a high temperature of 500 °C or higher.

Preferably, the stable structure corresponding to the substance related to sesame intolerance disease is selected from one or more of the following:
(1) the stable structure corresponding to the substance that causes sesame intolerance in the organism;
(2) the stable structure corresponding to the substance of the organism itself and related to sesame intolerance disease;
(3) the stable structure corresponding to the substance that inhibits the structure that causes sesame intolerance disease;
(4) the stable structure corresponding to the substance containing a substance of any one of (1) to (3) above.

The substance for treating and/or preventing sesame intolerance disease designed according to the above-mentioned method.

The preparation method of the above-mentioned substance for treating and/or preventing sesame intolerance disease, wherein the stable structure corresponding to the substance related to sesame intolerance disease is prepared by a high-temperature carbonization method.

Use of the above-mentioned substance for treating and/or preventing sesame intolerance disease in the preparation of medicines, health products, foods, and food additives.

A method for designing substances for the treatment and/or prevention of soybean intolerance disease, wherein the stable structure corresponding to the substance related to soybean intolerance disease is used as the substance for treating and/or preventing soybean intolerance disease.

Preferably, the stable structure corresponding to the substance refers to the structure of said substance that exists in a solid state at a high temperature of 500 °C or higher.

Preferably, the stable structure corresponding to the substance related to soybean intolerance disease is selected from one or more of the following:
(1) the stable structure corresponding to the substance that causes soybean intolerance in the organism;
(2) the stable structure corresponding to the substance of the organism itself and related to soybean intolerance disease;
(3) the stable structure corresponding to the substance that inhibits the structure that causes soybean intolerance disease;
(4) the stable structure corresponding to the substance containing a substance of any one of (1) to (3) above.

The substance for treating and/or preventing soybean intolerance disease designed according to the above-mentioned method.

The preparation method of the above-mentioned substance for treating and/or preventing soybean intolerance disease, wherein the stable structure corresponding to the substance related to soybean intolerance disease is prepared by a high-temperature carbonization method.

Use of the above-mentioned substance for treating and/or preventing soybean intolerance disease in the preparation of medicines, health products, foods, and food additives.

A method for designing substances for the treatment and/or prevention of sunflower seed intolerance disease, wherein the stable structure corresponding to the substance related to sunflower seed intolerance disease is used as the substance for treating and/or preventing sunflower seed intolerance disease.

Preferably, the stable structure corresponding to the substance refers to the structure of said substance that exists in a solid state at a high temperature of 500 °C or higher.

Preferably, the stable structure corresponding to the substance related to sunflower seed intolerance disease is selected from one or more of the following:
(1) the stable structure corresponding to the substance that causes sunflower seed intolerance in the organism;
(2) the stable structure corresponding to the substance of the organism itself and related to sunflower seed intolerance disease;
(3) the stable structure corresponding to the substance that inhibits the structure that causes sunflower seed intolerance disease;
(4) the stable structure corresponding to the substance containing a substance of any one of (1) to (3) above.

The substance for treating and/or preventing sunflower seed intolerance disease designed according to the above-mentioned method.

The preparation method of the above-mentioned substance for treating and/or preventing sunflower seed intolerance disease, wherein the stable structure corresponding to the substance related to sunflower seed intolerance disease is prepared by a high-temperature carbonization method.

Use of the above-mentioned substance for treating and/or preventing sunflower seed intolerance disease in the preparation of medicines, health products, foods, and food additives.

A method for designing substances for the treatment and/or prevention of crab intolerance disease, wherein the stable structure corresponding to the substance related to crab intolerance disease is used as the substance for treating and/or preventing crab intolerance disease.

Preferably, the stable structure corresponding to the substance refers to the structure of said substance that exists in a solid state at a high temperature of 500 °C or higher.

Preferably, the stable structure corresponding to the substance related to crab intolerance disease is selected from one or more of the following:
(1) the stable structure corresponding to the substance that causes crab intolerance in the organism;
(2) the stable structure corresponding to the substance of the organism itself and related to crab intolerance disease;
(3) the stable structure corresponding to the substance that inhibits the structure that causes crab intolerance disease;
(4) the stable structure corresponding to the substance containing a substance of any one of (1) to (3) above.

The substance for treating and/or preventing crab intolerance disease designed according to the above-mentioned method.

The preparation method of the above-mentioned substance for treating and/or preventing crab intolerance disease, wherein the stable structure corresponding to the substance related to crab intolerance disease is prepared by a high-temperature carbonization method.

Use of the above-mentioned substance for treating and/or preventing crab intolerance disease in the preparation of medicines, health products, foods, and food additives.

A method for designing substances for the treatment and/or prevention of oyster intolerance disease, wherein the stable structure corresponding to the substance related to oyster intolerance disease is used as the substance for treating and/or preventing oyster intolerance disease.

Preferably, the stable structure corresponding to the substance refers to the structure of said substance that exists in a solid state at a high temperature of 500 °C or higher.

Preferably, the stable structure corresponding to the substance related to oyster intolerance disease is selected from one or more of the following:
(1) the stable structure corresponding to the substance that causes oyster intolerance in the organism;
(2) the stable structure corresponding to the substance of the organism itself and related to oyster intolerance disease;
(3) the stable structure corresponding to the substance that inhibits the structure that causes oyster intolerance disease;
(4) the stable structure corresponding to the substance containing a substance of any one of (1) to (3) above.

The substance for treating and/or preventing oyster intolerance disease designed according to the above-mentioned method.

The preparation method of the above-mentioned substance for treating and/or preventing oyster intolerance disease, wherein the stable structure corresponding to the substance related to oyster intolerance disease is prepared by a high-temperature carbonization method.

Use of the above-mentioned substance for treating and/or preventing oyster intolerance disease in the preparation of medicines, health products, foods, and food additives.

A method for designing substances for the treatment and/or prevention of scallop intolerance disease, wherein the stable structure corresponding to the substance related to scallop intolerance disease is used as the substance for treating and/or preventing scallop intolerance disease.

Preferably, the stable structure corresponding to the substance refers to the structure of said substance that exists in a solid state at a high temperature of 500 °C or higher.

Preferably, the stable structure corresponding to the substance related to scallop intolerance disease is selected from one or more of the following:
(1) the stable structure corresponding to the substance that causes scallop intolerance in the organism;
(2) the stable structure corresponding to the substance of the organism itself and related to scallop intolerance disease;
(3) the stable structure corresponding to the substance that inhibits the structure that causes scallop intolerance disease;
(4) the stable structure corresponding to the substance containing a substance of any one of (1) to (3) above.

The substance for treating and/or preventing scallop intolerance disease designed according to the above-mentioned method.

The preparation method of the above-mentioned substance for treating and/or preventing scallop intolerance disease, wherein the stable structure corresponding to the substance related to scallop intolerance disease is prepared by a high-temperature carbonization method.

Use of the above-mentioned substance for treating and/or preventing scallop intolerance disease in the preparation of medicines, health products, foods, and food additives.

A method for designing substances for the treatment and/or prevention of shrimp intolerance disease, wherein the stable structure corresponding to the substance related to shrimp intolerance disease is used as the substance for treating and/or preventing shrimp intolerance disease.

Preferably, the stable structure corresponding to the substance refers to the structure of said substance that exists in a solid state at a high temperature of 500 °C or higher.

Preferably, the stable structure corresponding to the substance related to shrimp intolerance disease is selected from one or more of the following:
(1) the stable structure corresponding to the substance that causes shrimp intolerance in the organism;
(2) the stable structure corresponding to the substance of the organism itself and related to shrimp intolerance disease;
(3) the stable structure corresponding to the substance that inhibits the structure that causes shrimp intolerance disease;
(4) the stable structure corresponding to the substance containing a substance of any one of (1) to (3) above.

The substance for treating and/or preventing shrimp intolerance disease designed according to the above-mentioned method.

The preparation method of the above-mentioned substance for treating and/or preventing shrimp intolerance disease, wherein the stable structure corresponding to the substance related to shrimp intolerance disease is prepared by a high-temperature carbonization method.

Use of the above-mentioned substance for treating and/or preventing shrimp intolerance disease in the preparation of medicines, health products, foods, and food additives.

A method for designing substances for the treatment and/or prevention of brewer's yeast intolerance disease, wherein the stable structure corresponding to the substance related to brewer's yeast intolerance disease is used as the substance for treating and/or preventing brewer's yeast intolerance disease.

Preferably, the stable structure corresponding to the substance refers to the structure of said substance that exists in a solid state at a high temperature of 500 °C or higher.

Preferably, the stable structure corresponding to the substance related to brewer's yeast intolerance disease is selected from one or more of the following:
(1) the stable structure corresponding to the substance that causes brewer's yeast intolerance in the organism;
(2) the stable structure corresponding to the substance of the organism itself and related to brewer's yeast intolerance disease;
(3) the stable structure corresponding to the substance that inhibits the structure that causes brewer's yeast intolerance disease;
(4) the stable structure corresponding to the substance containing a substance of any one of (1) to (3) above.

The substance for treating and/or preventing brewer's yeast intolerance disease designed according to the above-mentioned method.

The preparation method of the above-mentioned substance for treating and/or preventing brewer's yeast intolerance disease, wherein the stable structure corresponding to the substance related to brewer's yeast intolerance disease is prepared by a high-temperature carbonization method.

Use of the above-mentioned substance for treating and/or preventing brewer's yeast intolerance disease in the preparation of medicines, health products, foods, and food additives.

A method for designing substances for the treatment and/or prevention of black tea intolerance disease, wherein the stable structure corresponding to the substance related to black tea intolerance disease is used as the substance for treating and/or preventing black tea intolerance disease.

Preferably, the stable structure corresponding to the substance refers to the structure of said substance that exists in a solid state at a high temperature of 500 °C or higher.

Preferably, the stable structure corresponding to the substance related to black tea intolerance disease is selected from one or more of the following:
(1) the stable structure corresponding to the substance that causes black tea intolerance in the organism;
(2) the stable structure corresponding to the substance of the organism itself and related to black tea intolerance disease;
(3) the stable structure corresponding to the substance that inhibits the structure that causes black tea intolerance disease;
(4) the stable structure corresponding to the substance containing a substance of any one of (1) to (3) above.

The substance for treating and/or preventing black tea intolerance disease designed according to the above-mentioned method.

The preparation method of the above-mentioned substance for treating and/or preventing black tea intolerance disease, wherein the stable structure corresponding to the substance related to black tea intolerance disease is prepared by a high-temperature carbonization method.

Use of the above-mentioned substance for treating and/or preventing black tea intolerance disease in the preparation of medicines, health products, foods, and food additives.

A method for designing substances for the treatment and/or prevention of broad bean intolerance disease, wherein the stable structure corresponding to the substance related to broad bean intolerance disease is used as the substance for treating and/or preventing broad bean intolerance disease.

Preferably, the stable structure corresponding to the substance refers to the structure of said substance that exists in a solid state at a high temperature of 500 °C or higher.

Preferably, the stable structure corresponding to the substance related to broad bean intolerance disease is selected from one or more of the following:
(1) the stable structure corresponding to the substance that causes broad bean intolerance in the organism;
(2) the stable structure corresponding to the substance of the organism itself and related to broad bean intolerance disease;
(3) the stable structure corresponding to the substance that inhibits the structure that causes broad bean intolerance disease;
(4) the stable structure corresponding to the substance containing a substance of any one of (1) to (3) above.

The substance for treating and/or preventing broad bean intolerance disease designed according to the above-mentioned method.

The preparation method of the above-mentioned substance for treating and/or preventing broad bean intolerance disease, wherein the stable structure corresponding to the substance related to broad bean intolerance disease is prepared by a high-temperature carbonization method.

Use of the above-mentioned substance for treating and/or preventing broad bean intolerance disease in the preparation of medicines, health products, foods, and food additives.

A method for designing substances for the treatment and/or prevention of green bean intolerance disease, wherein the stable structure corresponding to the substance related to green bean intolerance disease is used as the substance for treating and/or preventing green bean intolerance disease.

Preferably, the stable structure corresponding to the substance refers to the structure of said substance that exists in a solid state at a high temperature of 500 °C or higher.

Preferably, the stable structure corresponding to the substance related to green bean intolerance disease is selected from one or more of the following:
(1) the stable structure corresponding to the substance that causes green bean intolerance in the organism;
(2) the stable structure corresponding to the substance of the organism itself and related to green bean intolerance disease;
(3) the stable structure corresponding to the substance that inhibits the structure that causes green bean intolerance disease;
(4) the stable structure corresponding to the substance containing a substance of any one of (1) to (3) above.

The substance for treating and/or preventing green bean intolerance disease designed according to the above-mentioned method.

The preparation method of the above-mentioned substance for treating and/or preventing green bean intolerance disease, wherein the stable structure corresponding to the substance related to green bean intolerance disease is prepared by a high-temperature carbonization method.

Use of the above-mentioned substance for treating and/or preventing green bean intolerance disease in the preparation of medicines, health products, foods, and food additives.

A method for designing substances for the treatment and/or prevention of black walnut intolerance disease, wherein the stable structure corresponding to the substance related to black walnut intolerance disease is used as the substance for treating and/or preventing black walnut intolerance disease.

Preferably, the stable structure corresponding to the substance refers to the structure of said substance that exists in a solid state at a high temperature of 500 °C or higher.

Preferably, the stable structure corresponding to the substance related to black walnut intolerance disease is selected from one or more of the following:
(1) the stable structure corresponding to the substance that causes black walnut intolerance in the organism;
(2) the stable structure corresponding to the substance of the organism itself and related to black walnut intolerance disease;
(3) the stable structure corresponding to the substance that inhibits the structure that causes black walnut intolerance disease;
(4) the stable structure corresponding to the substance containing a substance of any one of (1) to (3) above.

The substance for treating and/or preventing black walnut intolerance disease designed according to the above-mentioned method.

The preparation method of the above-mentioned substance for treating and/or preventing black walnut intolerance disease, wherein the stable structure corresponding to the substance related to black walnut intolerance disease is prepared by a high-temperature carbonization method.

Use of the above-mentioned substance for treating and/or preventing black walnut intolerance disease in the preparation of medicines, health products, foods, and food additives.

A method for designing substances for the treatment and/or prevention of almond intolerance disease, wherein the stable structure corresponding to the substance related to almond intolerance disease is used as the substance for treating and/or preventing almond intolerance disease.

Preferably, the stable structure corresponding to the substance refers to the structure of said substance that exists in a solid state at a high temperature of 500 °C or higher.

Preferably, the stable structure corresponding to the substance related to almond intolerance disease is selected from one or more of the following:
(1) the stable structure corresponding to the substance that causes almond intolerance in the organism;
(2) the stable structure corresponding to the substance of the organism itself and related to almond intolerance disease;
(3) the stable structure corresponding to the substance that inhibits the structure that causes almond intolerance disease;
(4) the stable structure corresponding to the substance containing a substance of any one of (1) to (3) above.

The substance for treating and/or preventing almond intolerance disease designed according to the above-mentioned method.

The preparation method of the above-mentioned substance for treating and/or preventing almond intolerance disease, wherein the stable structure corresponding to the substance related to almond intolerance disease is prepared by a high-temperature carbonization method.

Use of the above-mentioned substance for treating and/or preventing almond intolerance disease in the preparation of medicines, health products, foods, and food additives.

A method for designing substances for the treatment and/or prevention of cod intolerance disease, wherein the stable structure corresponding to the substance related to cod intolerance disease is used as the substance for treating and/or preventing cod intolerance disease.

Preferably, the stable structure corresponding to the substance refers to the structure of said substance that exists in a solid state at a high temperature of 500 °C or higher.

Preferably, the stable structure corresponding to the substance related to cod intolerance disease is selected from one or more of the following:
(1) the stable structure corresponding to the substance that causes cod intolerance in the organism;
(2) the stable structure corresponding to the substance of the organism itself and related to cod intolerance disease;
(3) the stable structure corresponding to the substance that inhibits the structure that causes cod intolerance disease;
(4) the stable structure corresponding to the substance containing a substance of any one of (1) to (3) above.

The substance for treating and/or preventing cod intolerance disease designed according to the above-mentioned method.

The preparation method of the above-mentioned substance for treating and/or preventing cod intolerance disease, wherein the stable structure corresponding to the substance related to cod intolerance disease is prepared by a high-temperature carbonization method.

Use of the above-mentioned substance for treating and/or preventing cod intolerance disease in the preparation of medicines, health products, foods, and food additives.

A method for designing substances for the treatment and/or prevention of halibut intolerance disease, wherein the stable structure corresponding to the substance related to halibut intolerance disease is used as the substance for treating and/or preventing halibut intolerance disease.

Preferably, the stable structure corresponding to the substance refers to the structure of said substance that exists in a solid state at a high temperature of 500 °C or higher.

Preferably, the stable structure corresponding to the substance related to halibut intolerance disease is selected from one or more of the following:
(1) the stable structure corresponding to the substance that causes halibut intolerance in the organism;
(2) the stable structure corresponding to the substance of the organism itself and related to halibut intolerance disease;
(3) the stable structure corresponding to the substance that inhibits the structure that causes halibut intolerance disease;
(4) the stable structure corresponding to the substance containing a substance of any one of (1) to (3) above.

The substance for treating and/or preventing halibut intolerance disease designed according to the above-mentioned method.

The preparation method of the above-mentioned substance for treating and/or preventing halibut intolerance disease, wherein the stable structure corresponding to the substance related to halibut intolerance disease is prepared by a high-temperature carbonization method.

Use of the above-mentioned substance for treating and/or preventing halibut intolerance disease in the preparation of medicines, health products, foods, and food additives.

A method for designing substances for the treatment and/or prevention of lobster intolerance disease, wherein the stable structure corresponding to the substance related to lobster intolerance disease is used as the substance for treating and/or preventing lobster intolerance disease.

Preferably, the stable structure corresponding to the substance refers to the structure of said substance that exists in a solid state at a high temperature of 500 °C or higher.

Preferably, the stable structure corresponding to the substance related to lobster intolerance disease is selected from one or more of the following:
(1) the stable structure corresponding to the substance that causes lobster intolerance in the organism;
(2) the stable structure corresponding to the substance of the organism itself and related to lobster intolerance disease;
(3) the stable structure corresponding to the substance that inhibits the structure that causes lobster intolerance disease;
(4) the stable structure corresponding to the substance containing a substance of any one of (1) to (3) above.

The substance for treating and/or preventing lobster intolerance disease designed according to the above-mentioned method.

The preparation method of the above-mentioned substance for treating and/or preventing lobster intolerance disease, wherein the stable structure corresponding to the substance related to lobster intolerance disease is prepared by a high-temperature carbonization method.

Use of the above-mentioned substance for treating and/or preventing lobster intolerance disease in the preparation of medicines, health products, foods, and food additives.

A method for designing substances for the treatment and/or prevention of salmon intolerance disease, wherein the stable structure corresponding to the substance related to salmon intolerance disease is used as the substance for treating and/or preventing salmon intolerance disease.

Preferably, the stable structure corresponding to the substance refers to the structure of said substance that exists in a solid state at a high temperature of 500 °C or higher.

Preferably, the stable structure corresponding to the substance related to salmon intolerance disease is selected from one or more of the following:
(1) the stable structure corresponding to the substance that causes salmon intolerance in the organism;
(2) the stable structure corresponding to the substance of the organism itself and related to salmon intolerance disease;
(3) the stable structure corresponding to the substance that inhibits the structure that causes salmon intolerance disease;
(4) the stable structure corresponding to the substance containing a substance of any one of (1) to (3) above.

The substance for treating and/or preventing salmon intolerance disease designed according to the above-mentioned method.

The preparation method of the above-mentioned substance for treating and/or preventing salmon intolerance disease, wherein the stable structure corresponding to the substance related to salmon intolerance disease is prepared by a high-temperature carbonization method.

Use of the above-mentioned substance for treating and/or preventing salmon intolerance disease in the preparation of medicines, health products, foods, and food additives.

A method for designing substances for the treatment and/or prevention of sardine intolerance disease, wherein the stable structure corresponding to the substance related to sardine intolerance disease is used as the substance for treating and/or preventing sardine intolerance disease.

Preferably, the stable structure corresponding to the substance refers to the structure of said substance that exists in a solid state at a high temperature of 500 °C or higher.

Preferably, the stable structure corresponding to the substance related to sardine intolerance disease is selected from one or more of the following:
(1) the stable structure corresponding to the substance that causes sardine intolerance in the organism;
(2) the stable structure corresponding to the substance of the organism itself and related to sardine intolerance disease;
(3) the stable structure corresponding to the substance that inhibits the structure that causes sardine intolerance disease;
(4) the stable structure corresponding to the substance containing a substance of any one of (1) to (3) above.

The substance for treating and/or preventing sardine intolerance disease designed according to the above-mentioned method.

The preparation method of the above-mentioned substance for treating and/or preventing sardine intolerance disease, wherein the stable structure corresponding to the substance related to sardine intolerance disease is prepared by a high-temperature carbonization method.

Use of the above-mentioned substance for treating and/or preventing sardine intolerance disease in the preparation of medicines, health products, foods, and food additives.

A method for designing substances for the treatment and/or prevention of carp intolerance disease, wherein the stable structure corresponding to the substance related to carp intolerance disease is used as the substance for treating and/or preventing carp intolerance disease.

Preferably, the stable structure corresponding to the substance refers to the structure of said substance that exists in a solid state at a high temperature of 500 °C or higher.

Preferably, the stable structure corresponding to the substance related to carp intolerance disease is selected from one or more of the following:
(1) the stable structure corresponding to the substance that causes carp intolerance in the organism;
(2) the stable structure corresponding to the substance of the organism itself and related to carp intolerance disease;
(3) the stable structure corresponding to the substance that inhibits the structure that causes carp intolerance disease;
(4) the stable structure corresponding to the substance containing a substance of any one of (1) to (3) above.

The substance for treating and/or preventing carp intolerance disease designed according to the above-mentioned method.

The preparation method of the above-mentioned substance for treating and/or preventing carp intolerance disease, wherein the stable structure corresponding to the substance related to carp intolerance disease is prepared by a high-temperature carbonization method.

Use of the above-mentioned substance for treating and/or preventing carp intolerance disease in the preparation of medicines, health products, foods, and food additives.

A method for designing substances for the treatment and/or prevention of trout intolerance disease, wherein the stable structure corresponding to the substance related to trout intolerance disease is used as the substance for treating and/or preventing trout intolerance disease.

Preferably, the stable structure corresponding to the substance refers to the structure of said substance that exists in a solid state at a high temperature of 500 °C or higher.

Preferably, the stable structure corresponding to the substance related to trout intolerance disease is selected from one or more of the following:
(1) the stable structure corresponding to the substance that causes trout intolerance in the organism;
(2) the stable structure corresponding to the substance of the organism itself and related to trout intolerance disease;
(3) the stable structure corresponding to the substance that inhibits the structure that causes trout intolerance disease;
(4) the stable structure corresponding to the substance containing a substance of any one of (1) to (3) above.

The substance for treating and/or preventing trout intolerance disease designed according to the above-mentioned method.

The preparation method of the above-mentioned substance for treating and/or preventing trout intolerance disease, wherein the stable structure corresponding to the substance related to trout intolerance disease is prepared by a high-temperature carbonization method.

Use of the above-mentioned substance for treating and/or preventing trout intolerance disease in the preparation of medicines, health products, foods, and food additives.

A method for designing substances for the treatment and/or prevention of tuna intolerance disease, wherein the stable structure corresponding to the substance related to tuna intolerance disease is used as the substance for treating and/or preventing tuna intolerance disease.

Preferably, the stable structure corresponding to the substance refers to the structure of said substance that exists in a solid state at a high temperature of 500 °C or higher.

Preferably, the stable structure corresponding to the substance related to tuna intolerance disease is selected from one or more of the following:
(1) the stable structure corresponding to the substance that causes tuna intolerance in the organism;
(2) the stable structure corresponding to the substance of the organism itself and related to tuna intolerance disease;
(3) the stable structure corresponding to the substance that inhibits the structure that causes tuna intolerance disease;
(4) the stable structure corresponding to the substance containing a substance of any one of (1) to (3) above.

The substance for treating and/or preventing tuna intolerance disease designed according to the above-mentioned method.

The preparation method of the above-mentioned substance for treating and/or preventing tuna intolerance disease, wherein the stable structure corresponding to the substance related to tuna intolerance disease is prepared by a high-temperature carbonization method.

Use of the above-mentioned substance for treating and/or preventing tuna intolerance disease in the preparation of medicines, health products, foods, and food additives.

A method for designing substances for the treatment and/or prevention of chocolate intolerance disease, wherein the stable structure corresponding to the substance related to chocolate intolerance disease is used as the substance for treating and/or preventing chocolate intolerance disease.

Preferably, the stable structure corresponding to the substance refers to the structure of said substance that exists in a solid state at a high temperature of 500 °C or higher.

Preferably, the stable structure corresponding to the substance related to chocolate intolerance disease is selected from one or more of the following:
(1) the stable structure corresponding to the substance that causes chocolate intolerance in the organism;
(2) the stable structure corresponding to the substance of the organism itself and related to chocolate intolerance disease;
(3) the stable structure corresponding to the substance that inhibits the structure that causes chocolate intolerance disease;
(4) the stable structure corresponding to the substance containing a substance of any one of (1) to (3) above.

The substance for treating and/or preventing chocolate intolerance disease designed according to the above-mentioned method.

The preparation method of the above-mentioned substance for treating and/or preventing chocolate intolerance disease, wherein the stable structure corresponding to the substance related to chocolate intolerance disease is prepared by a high-temperature carbonization method.

Use of the above-mentioned substance for treating and/or preventing chocolate intolerance disease in the preparation of medicines, health products, foods, and food additives.

A method for designing substances for the treatment and/or prevention of coffee intolerance disease, wherein the stable structure corresponding to the substance related to coffee intolerance disease is used as the substance for treating and/or preventing coffee intolerance disease.

Preferably, the stable structure corresponding to the substance refers to the structure of said substance that exists in a solid state at a high temperature of 500 °C or higher.

Preferably, the stable structure corresponding to the substance related to coffee intolerance disease is selected from one or more of the following:
(1) the stable structure corresponding to the substance that causes coffee intolerance in the organism;
(2) the stable structure corresponding to the substance of the organism itself and related to coffee intolerance disease;
(3) the stable structure corresponding to the substance that inhibits the structure that causes coffee intolerance disease;
(4) the stable structure corresponding to the substance containing a substance of any one of (1) to (3) above.

The substance for treating and/or preventing coffee intolerance disease designed according to the above-mentioned method.

The preparation method of the above-mentioned substance for treating and/or preventing coffee intolerance disease, wherein the stable structure corresponding to the substance related to coffee intolerance disease is prepared by a high-temperature carbonization method.

Use of the above-mentioned substance for treating and/or preventing coffee intolerance disease in the preparation of medicines, health products, foods, and food additives.

A method for designing substances for the treatment and/or prevention of mopane (Colophospermum mopane) intolerance disease, wherein the stable structure corresponding to the substance related to mopane intolerance disease is used as the substance for treating and/or preventing mopane intolerance disease.

Preferably, the stable structure corresponding to the substance refers to the structure of said substance that exists in a solid state at a high temperature of 500 °C or higher.

Preferably, the stable structure corresponding to the substance related to mopane intolerance disease is selected from one or more of the following:
(1) the stable structure corresponding to the substance that causes mopane intolerance in the organism;
(2) the stable structure corresponding to the substance of the organism itself and related to mopane intolerance disease;
(3) the stable structure corresponding to the substance that inhibits the structure that causes mopane intolerance disease;
(4) the stable structure corresponding to the substance containing a substance of any one of (1) to (3) above.

The substance for treating and/or preventing mopane intolerance disease designed according to the above-mentioned method.

The preparation method of the above-mentioned substance for treating and/or preventing mopane intolerance disease, wherein the stable structure corresponding to the substance related to mopane intolerance disease is prepared by a high-temperature carbonization method.

Use of the above-mentioned substance for treating and/or preventing mopane intolerance disease in the preparation of medicines, health products, foods, and food additives.

A method for designing substances for the treatment and/or prevention of baker's yeast (*Saccharomyces cerevisiae*) intolerance disease, wherein the stable structure corresponding to the substance related to baker's yeast intolerance disease is used as the substance for treating and/or preventing baker's yeast intolerance disease.

Preferably, the stable structure corresponding to the substance refers to the structure of said substance that exists in a solid state at a high temperature of 500 °C or higher.

Preferably, the stable structure corresponding to the substance related to baker's yeast intolerance disease is selected from one or more of the following:
(1) the stable structure corresponding to the substance that causes baker's yeast intolerance in the organism;
(2) the stable structure corresponding to the substance of the organism itself and related to baker's yeast intolerance disease;
(3) the stable structure corresponding to the substance that inhibits the structure that causes baker's yeast intolerance disease;
(4) the stable structure corresponding to the substance containing a substance of any one of (1) to (3) above.

The substance for treating and/or preventing baker's yeast intolerance disease designed according to the above-mentioned method.

The preparation method of the above-mentioned substance for treating and/or preventing baker's yeast intolerance disease, wherein the stable structure corresponding to the substance related to baker's yeast intolerance disease is prepared by a high-temperature carbonization method.

Use of the above-mentioned substance for treating and/or preventing baker's yeast intolerance disease in the preparation of medicines, health products, foods, and food additives.

A method for designing substances for the treatment and/or prevention of monosaccharose intolerance disease, wherein the stable structure corresponding to the substance related to monosaccharose intolerance disease is used as the substance for treating and/or preventing monosaccharose intolerance disease.

Preferably, the stable structure corresponding to the substance refers to the structure of said substance that exists in a solid state at a high temperature of 500 °C or higher.

Preferably, the stable structure corresponding to the substance related to monosaccharose intolerance disease is selected from one or more of the following:
(1) the stable structure corresponding to the substance that causes monosaccharose intolerance in the organism;
(2) the stable structure corresponding to the substance of the organism itself and related to monosaccharose intolerance disease;
(3) the stable structure corresponding to the substance that inhibits the structure that causes monosaccharose intolerance disease;
(4) the stable structure corresponding to the substance containing a substance of any one of (1) to (3) above.

The substance for treating and/or preventing monosaccharose intolerance disease designed according to the above-mentioned method.

The preparation method of the above-mentioned substance for treating and/or preventing monosaccharose intolerance disease, wherein the stable structure corresponding to the substance related to monosaccharose intolerance disease is prepared by a high-temperature carbonization method.

Use of the above-mentioned substance for treating and/or preventing monosaccharose intolerance disease in the preparation of medicines, health products, foods, and food additives.

A method for designing substances for the treatment and/or prevention of disaccharide intolerance disease, wherein the stable structure corresponding to the substance related to disaccharide intolerance disease is used as the substance for treating and/or preventing disaccharide intolerance disease.

Preferably, the stable structure corresponding to the substance refers to the structure of said substance that exists in a solid state at a high temperature of 500 °C or higher.

Preferably, the stable structure corresponding to the substance related to disaccharide intolerance disease is selected from one or more of the following:
(1) the stable structure corresponding to the substance that causes disaccharide intolerance in the organism;
(2) the stable structure corresponding to the substance of the organism itself and related to disaccharide intolerance disease;
(3) the stable structure corresponding to the substance that inhibits the structure that causes disaccharide intolerance disease;
(4) the stable structure corresponding to the substance containing a substance of any one of (1) to (3) above.

The substance for treating and/or preventing disaccharide intolerance disease designed according to the above-mentioned method.

The preparation method of the above-mentioned substance for treating and/or preventing disaccharide intolerance disease, wherein the stable structure corresponding to the substance related to disaccharide intolerance disease is prepared by a high-temperature carbonization method.

Use of the above-mentioned substance for treating and/or preventing disaccharide intolerance disease in the preparation of medicines, health products, foods, and food additives.

A method for designing substances for the treatment and/or prevention of lima beans intolerance disease, wherein the stable structure corresponding to the substance related to lima beans intolerance disease is used as the substance for treating and/or preventing lima beans intolerance disease.

Preferably, the stable structure corresponding to the substance refers to the structure of said substance that exists in a solid state at a high temperature of 500 °C or higher.

Preferably, the stable structure corresponding to the substance related to lima beans intolerance disease is selected from one or more of the following:
(1) the stable structure corresponding to the substance that causes lima beans intolerance in the organism;
(2) the stable structure corresponding to the substance of the organism itself and related to lima beans intolerance disease;
(3) the stable structure corresponding to the substance that inhibits the structure that causes lima beans intolerance disease;
(4) the stable structure corresponding to the substance containing a substance of any one of (1) to (3) above.

The substance for treating and/or preventing lima beans intolerance disease designed according to the above-mentioned method.

The preparation method of the above-mentioned substance for treating and/or preventing lima beans intolerance disease, wherein the stable structure corresponding to the substance related to lima beans intolerance disease is prepared by a high-temperature carbonization method.

Use of the above-mentioned substance for treating and/or preventing lima beans intolerance disease in the preparation of medicines, health products, foods, and food additives.

A method for designing substances for the treatment and/or prevention of maltose intolerance disease, wherein the stable structure corresponding to the substance related to maltose intolerance disease is used as the substance for treating and/or preventing maltose intolerance disease.

Preferably, the stable structure corresponding to the substance refers to the structure of said substance that exists in a solid state at a high temperature of 500 °C or higher.

Preferably, the stable structure corresponding to the substance related to maltose intolerance disease is selected from one or more of the following:
(1) the stable structure corresponding to the substance that causes maltose intolerance in the organism;
(2) the stable structure corresponding to the substance of the organism itself and related to maltose intolerance disease;
(3) the stable structure corresponding to the substance that inhibits the structure that causes maltose intolerance disease;
(4) the stable structure corresponding to the substance containing a substance of any one of (1) to (3) above.

The substance for treating and/or preventing maltose intolerance disease designed according to the above-mentioned method.

The preparation method of the above-mentioned substance for treating and/or preventing maltose intolerance disease, wherein the stable structure corresponding to the substance related to maltose intolerance disease is prepared by a high-temperature carbonization method.

Use of the above-mentioned substance for treating and/or preventing maltose intolerance disease in the preparation of medicines, health products, foods, and food additives.

A method for designing substances for the treatment and/or prevention of polysaccharide intolerance disease, wherein the stable structure corresponding to the substance related to polysaccharide intolerance disease is used as the substance for treating and/or preventing polysaccharide intolerance disease.

Preferably, the stable structure corresponding to the substance refers to the structure of said substance that exists in a solid state at a high temperature of 500 °C or higher.

Preferably, the stable structure corresponding to the substance related to polysaccharide intolerance disease is selected from one or more of the following:
(1) the stable structure corresponding to the substance that causes polysaccharide intolerance in the organism;
(2) the stable structure corresponding to the substance of the organism itself and related to polysaccharide intolerance disease;
(3) the stable structure corresponding to the substance that inhibits the structure that causes polysaccharide intolerance disease;
(4) the stable structure corresponding to the substance containing a substance of any one of (1) to (3) above.

The substance for treating and/or preventing polysaccharide intolerance disease designed according to the above-mentioned method.

The preparation method of the above-mentioned substance for treating and/or preventing polysaccharide intolerance disease, wherein the stable structure corresponding to the substance related to polysaccharide intolerance disease is prepared by a high-temperature carbonization method.

Use of the above-mentioned substance for treating and/or preventing polysaccharide intolerance disease in the preparation of medicines, health products, foods, and food additives.

A method for designing substances for the treatment and/or prevention of starch intolerance disease, wherein the stable structure corresponding to the substance related to starch intolerance disease is used as the substance for treating and/or preventing starch intolerance disease.

Preferably, the stable structure corresponding to the substance refers to the structure of said substance that exists in a solid state at a high temperature of 500 °C or higher.

Preferably, the stable structure corresponding to the substance related to starch intolerance disease is selected from one or more of the following:
(1) the stable structure corresponding to the substance that causes starch intolerance in the organism;
(2) the stable structure corresponding to the substance of the organism itself and related to starch intolerance disease;
(3) the stable structure corresponding to the substance that inhibits the structure that causes starch intolerance disease;
(4) the stable structure corresponding to the substance containing a substance of any one of (1) to (3) above.

The substance for treating and/or preventing starch intolerance disease designed according to the above-mentioned method.

The preparation method of the above-mentioned substance for treating and/or preventing starch intolerance disease, wherein the stable structure corresponding to the substance related to starch intolerance disease is prepared by a high-temperature carbonization method.

Use of the above-mentioned substance for treating and/or preventing starch intolerance disease in the preparation of medicines, health products, foods, and food additives.

A method for designing substances for the treatment and/or prevention of sucrose intolerance disease, wherein the stable structure corresponding to the substance related to sucrose intolerance disease is used as the substance for treating and/or preventing sucrose intolerance disease.

Preferably, the stable structure corresponding to the substance refers to the structure of said substance that exists in a solid state at a high temperature of 500 °C or higher.

Preferably, the stable structure corresponding to the substance related to sucrose intolerance disease is selected from one or more of the following:
(1) the stable structure corresponding to the substance that causes sucrose intolerance in the organism;
(2) the stable structure corresponding to the substance of the organism itself and related to sucrose intolerance disease;
(3) the stable structure corresponding to the substance that inhibits the structure that causes sucrose intolerance disease;
(4) the stable structure corresponding to the substance containing a substance of any one of (1) to (3) above.

The substance for treating and/or preventing sucrose intolerance disease designed according to the above-mentioned method.

The preparation method of the above-mentioned substance for treating and/or preventing sucrose intolerance disease, wherein the stable structure corresponding to the substance related to sucrose intolerance disease is prepared by a high-temperature carbonization method.

Use of the above-mentioned substance for treating and/or preventing sucrose intolerance disease in the preparation of medicines, health products, foods, and food additives.

A method for designing substances for the treatment and/or prevention of alcohol intolerance disease, wherein the stable structure corresponding to the substance related to alcohol intolerance disease is used as the substance for treating and/or preventing alcohol intolerance disease.

Preferably, the stable structure corresponding to the substance refers to the structure of said substance that exists in a solid state at a high temperature of 500 °C or higher.

Preferably, the stable structure corresponding to the substance related to alcohol intolerance disease is selected from one or more of the following:
(1) the stable structure corresponding to the substance that causes alcohol intolerance in the organism;
(2) the stable structure corresponding to the substance of the organism itself and related to alcohol intolerance disease;
(3) the stable structure corresponding to the substance that inhibits the structure that causes alcohol intolerance disease;
(4) the stable structure corresponding to the substance containing a substance of any one of (1) to (3) above.

The substance for treating and/or preventing alcohol intolerance disease designed according to the above-mentioned method.

The preparation method of the above-mentioned substance for treating and/or preventing alcohol intolerance disease, wherein the stable structure corresponding to the substance related to alcohol intolerance disease is prepared by a high-temperature carbonization method.

Use of the above-mentioned substance for treating and/or preventing alcohol intolerance disease in the preparation of medicines, health products, foods, and food additives.

A method for designing substances for the treatment and/or prevention of biogenic amine intolerance disease, wherein the stable structure corresponding to the substance related to biogenic amine intolerance disease is used as the substance for treating and/or preventing biogenic amine intolerance disease.

Preferably, the stable structure corresponding to the substance refers to the structure of said substance that exists in a solid state at a high temperature of 500 °C or higher.

Preferably, the stable structure corresponding to the substance related to biogenic amine intolerance disease is selected from one or more of the following:
(1) the stable structure corresponding to the substance that causes biogenic amine intolerance in the organism;
(2) the stable structure corresponding to the substance of the organism itself and related to biogenic amine intolerance disease;
(3) the stable structure corresponding to the substance that inhibits the structure that causes biogenic amine intolerance disease;
(4) the stable structure corresponding to the substance containing a substance of any one of (1) to (3) above.

The substance for treating and/or preventing biogenic amine intolerance disease designed according to the above-mentioned method.

The preparation method of the above-mentioned substance for treating and/or preventing biogenic amine intolerance disease, wherein the stable structure corresponding to the substance related to biogenic amine intolerance disease is prepared by a high-temperature carbonization method.

Use of the above-mentioned substance for treating and/or preventing biogenic amine intolerance disease in the preparation of medicines, health products, foods, and food additives.

A method for designing substances for the treatment and/or prevention of salicylates intolerance disease, wherein the stable structure corresponding to the substance related to salicylates intolerance disease is used as the substance for treating and/or preventing salicylates intolerance disease.

Preferably, the stable structure corresponding to the substance refers to the structure of said substance that exists in a solid state at a high temperature of 500 °C or higher.

Preferably, the stable structure corresponding to the substance related to salicylates intolerance disease is selected from one or more of the following:
(1) the stable structure corresponding to the substance that causes salicylates intolerance in the organism;
(2) the stable structure corresponding to the substance of the organism itself and related to salicylates intolerance disease;
(3) the stable structure corresponding to the substance that inhibits the structure that causes salicylates intolerance disease;
(4) the stable structure corresponding to the substance containing a substance of any one of (1) to (3) above.

The substance for treating and/or preventing salicylates intolerance disease designed according to the above-mentioned method.

The preparation method of the above-mentioned substance for treating and/or preventing salicylates intolerance disease, wherein the stable structure corresponding to the substance related to salicylates intolerance disease is prepared by a high-temperature carbonization method.

Use of the above-mentioned substance for treating and/or preventing salicylates intolerance disease in the preparation of medicines, health products, foods, and food additives.

A method for designing substances for the treatment and/or prevention of food additives intolerance disease, wherein the stable structure corresponding to the substance related to food additives intolerance disease is used as the substance for treating and/or preventing food additives intolerance disease.

Preferably, the stable structure corresponding to the substance refers to the structure of said substance that exists in a solid state at a high temperature of 500 °C or higher.

Preferably, the stable structure corresponding to the substance related to food additives intolerance disease is selected from one or more of the following:
(1) the stable structure corresponding to the substance that causes food additives intolerance in the organism;
(2) the stable structure corresponding to the substance of the organism itself and related to food additives intolerance disease;
(3) the stable structure corresponding to the substance that inhibits the structure that causes food additives intolerance disease;
(4) the stable structure corresponding to the substance containing a substance of any one of (1) to (3) above.

The substance for treating and/or preventing food additives intolerance disease designed according to the above-mentioned method.

The preparation method of the above-mentioned substance for treating and/or preventing food additives intolerance disease, wherein the stable structure corresponding to the substance related to food additives intolerance disease is prepared by a high-temperature carbonization method.

Use of the above-mentioned substance for treating and/or preventing food additives intolerance disease in the preparation of medicines, health products, foods, and food additives.

A method for designing substances for the treatment and/or prevention of nitrites intolerance disease, wherein the stable structure corresponding to the substance related to nitrites intolerance disease is used as the substance for treating and/or preventing nitrites intolerance disease.

Preferably, the stable structure corresponding to the substance refers to the structure of said substance that exists in a solid state at a high temperature of 500 °C or higher.

Preferably, the stable structure corresponding to the substance related to nitrites intolerance disease is selected from one or more of the following:
(1) the stable structure corresponding to the substance that causes nitrites intolerance in the organism;
(2) the stable structure corresponding to the substance of the organism itself and related to nitrites intolerance disease;
(3) the stable structure corresponding to the substance that inhibits the structure that causes nitrites intolerance disease;
(4) the stable structure corresponding to the substance containing a substance of any one of (1) to (3) above.

The substance for treating and/or preventing nitrites intolerance disease designed according to the above-mentioned method.

The preparation method of the above-mentioned substance for treating and/or preventing nitrites intolerance disease, wherein the stable structure corresponding to the substance related to nitrites intolerance disease is prepared by a high-temperature carbonization method.

Use of the above-mentioned substance for treating and/or preventing nitrites intolerance disease in the preparation of medicines, health products, foods, and food additives.

A method for designing substances for the treatment and/or prevention of artificial pigment intolerance disease, wherein the stable structure corresponding to the substance related to artificial pigment intolerance disease is used as the substance for treating and/or preventing artificial pigment intolerance disease.

Preferably, the stable structure corresponding to the substance refers to the structure of said substance that exists in a solid state at a high temperature of 500 °C or higher.

Preferably, the stable structure corresponding to the substance related to artificial pigment intolerance disease is selected from one or more of the following:
(1) the stable structure corresponding to the substance that causes artificial pigment intolerance in the organism;
(2) the stable structure corresponding to the substance of the organism itself and related to artificial pigment intolerance disease;
(3) the stable structure corresponding to the substance that inhibits the structure that causes artificial pigment intolerance disease;
(4) the stable structure corresponding to the substance containing a substance of any one of (1) to (3) above.

The substance for treating and/or preventing artificial pigment intolerance disease designed according to the above-mentioned method.

The preparation method of the above-mentioned substance for treating and/or preventing artificial pigment intolerance disease, wherein the stable structure corresponding to the substance related to artificial pigment intolerance disease is prepared by a high-temperature carbonization method.

Use of the above-mentioned substance for treating and/or preventing artificial pigment intolerance disease in the preparation of medicines, health products, foods, and food additives.

A method for designing substances for the treatment and/or prevention of azo dye intolerance disease, wherein the stable structure corresponding to the substance related to azo dye intolerance disease is used as the substance for treating and/or preventing azo dye intolerance disease.

Preferably, the stable structure corresponding to the substance refers to the structure of said substance that exists in a solid state at a high temperature of 500 °C or higher.

Preferably, the stable structure corresponding to the substance related to azo dye intolerance disease is selected from one or more of the following:
(1) the stable structure corresponding to the substance that causes azo dye intolerance in the organism;
(2) the stable structure corresponding to the substance of the organism itself and related to azo dye intolerance disease;
(3) the stable structure corresponding to the substance that inhibits the structure that causes azo dye intolerance disease;
(4) the stable structure corresponding to the substance containing a substance of any one of (1) to (3) above.

The substance for treating and/or preventing azo dye intolerance disease designed according to the above-mentioned method.

The preparation method of the above-mentioned substance for treating and/or preventing azo dye intolerance disease, wherein the stable structure corresponding to the substance related to azo dye intolerance disease is prepared by a high-temperature carbonization method.

Use of the above-mentioned substance for treating and/or preventing azo dye intolerance disease in the preparation of medicines, health products, foods, and food additives.

A method for designing substances for the treatment and/or prevention of drug intolerance disease, wherein the stable structure corresponding to the substance related to drug intolerance disease is used as the substance for treating and/or preventing drug intolerance disease.

Preferably, the stable structure corresponding to the substance refers to the structure of said substance that exists in a solid state at a high temperature of 500 °C or higher.

Preferably, the stable structure corresponding to the substance related to drug intolerance disease is selected from one or more of the following:
(1) the stable structure corresponding to the substance that causes drug intolerance in the organism;
(2) the stable structure corresponding to the substance of the organism itself and related to drug intolerance disease;
(3) the stable structure corresponding to the substance that inhibits the structure that causes drug intolerance disease;
(4) the stable structure corresponding to the substance containing a substance of any one of (1) to (3) above.

The substance for treating and/or preventing drug intolerance disease designed according to the above-mentioned method.

The preparation method of the above-mentioned substance for treating and/or preventing drug intolerance disease, wherein the stable structure corresponding to the substance related to drug intolerance disease is prepared by a high-temperature carbonization method.

Use of the above-mentioned substance for treating and/or preventing drug intolerance disease in the preparation of medicines, health products, foods, and food additives.

A method for designing substances for the treatment and/or prevention of anti-infectives intolerance disease, wherein the stable structure corresponding to the substance related to anti-infectives intolerance disease is used as the substance for treating and/or preventing anti-infectives intolerance disease.

Preferably, the stable structure corresponding to the substance refers to the structure of said substance that exists in a solid state at a high temperature of 500 °C or higher.

Preferably, the stable structure corresponding to the substance related to anti-infectives intolerance disease is selected from one or more of the following:
(1) the stable structure corresponding to the substance that causes anti-infectives intolerance in the organism;
(2) the stable structure corresponding to the substance of the organism itself and related to anti-infectives intolerance disease;
(3) the stable structure corresponding to the substance that inhibits the structure that causes anti-infectives intolerance disease;
(4) the stable structure corresponding to the substance containing a substance of any one of (1) to (3) above.

The substance for treating and/or preventing anti-infectives intolerance disease designed according to the above-mentioned method.

The preparation method of the above-mentioned substance for treating and/or preventing anti-infectives intolerance disease, wherein the stable structure corresponding to the substance related to anti-infectives intolerance disease is prepared by a high-temperature carbonization method.

Use of the above-mentioned substance for treating and/or preventing anti-infectives intolerance disease in the preparation of medicines, health products, foods, and food additives.

A method for designing substances for the treatment and/or prevention of anesthetics and auxiliary drugs intolerance disease, wherein the stable structure corresponding to the substance related to anesthetics and auxiliary drugs intolerance disease is used as the substance for treating and/or preventing anesthetics and auxiliary drugs intolerance disease.

Preferably, the stable structure corresponding to the substance refers to the structure of said substance that exists in a solid state at a high temperature of 500 °C or higher.

Preferably, the stable structure corresponding to the substance related to anesthetics and auxiliary drugs intolerance disease is selected from one or more of the following:
(1) the stable structure corresponding to the substance that causes anesthetics and auxiliary drugs intolerance in the organism;
(2) the stable structure corresponding to the substance of the organism itself and related to anesthetics and auxiliary drugs intolerance disease;
(3) the stable structure corresponding to the substance that inhibits the structure that causes anesthetics and auxiliary drugs intolerance disease;
(4) the stable structure corresponding to the substance containing a substance of any one of (1) to (3) above.

The substance for treating and/or preventing anesthetics and auxiliary drugs intolerance disease designed according to the above-mentioned method.

The preparation method of the above-mentioned substance for treating and/or preventing anesthetics and auxiliary drugs intolerance disease, wherein the stable structure corresponding to the substance related to anesthetics and auxiliary drugs intolerance disease is prepared by a high-temperature carbonization method.

Use of the above-mentioned substance for treating and/or preventing anesthetics and auxiliary drugs intolerance disease in the preparation of medicines, health products, foods, and food additives.

A method for designing substances for the treatment and/or prevention of cholinergic drugs intolerance disease, wherein the stable structure corresponding to the substance related to cholinergic drugs intolerance disease is used as the substance for treating and/or preventing cholinergic drugs intolerance disease.

Preferably, the stable structure corresponding to the substance refers to the structure of said substance that exists in a solid state at a high temperature of 500 °C or higher.

Preferably, the stable structure corresponding to the substance related to cholinergic drugs intolerance disease is selected from one or more of the following:
(1) the stable structure corresponding to the substance that causes cholinergic drugs intolerance in the organism;
(2) the stable structure corresponding to the substance of the organism itself and related to cholinergic drugs intolerance disease;
(3) the stable structure corresponding to the substance that inhibits the structure that causes cholinergic drugs intolerance disease;
(4) the stable structure corresponding to the substance containing a substance of any one of (1) to (3) above.

The substance for treating and/or preventing cholinergic drugs intolerance disease designed according to the above-mentioned method.

The preparation method of the above-mentioned substance for treating and/or preventing cholinergic drugs intolerance disease, wherein the stable structure corresponding to the substance related to cholinergic drugs intolerance disease is prepared by a high-temperature carbonization method.

Use of the above-mentioned substance for treating and/or preventing cholinergic drugs intolerance disease in the preparation of medicines, health products, foods, and food additives.

A method for designing substances for the treatment and/or prevention of psychotropic drugs intolerance disease, wherein the stable structure corresponding to the substance related to psychotropic drugs intolerance disease is used as the substance for treating and/or preventing psychotropic drugs intolerance disease.

Preferably, the stable structure corresponding to the substance refers to the structure of said substance that exists in a solid state at a high temperature of 500 °C or higher.

Preferably, the stable structure corresponding to the substance related to psychotropic drugs intolerance disease is selected from one or more of the following:
(1) the stable structure corresponding to the substance that causes psychotropic drugs intolerance in the organism;
(2) the stable structure corresponding to the substance of the organism itself and related to psychotropic drugs intolerance disease;
(3) the stable structure corresponding to the substance that inhibits the structure that causes psychotropic drugs intolerance disease;
(4) the stable structure corresponding to the substance containing a substance of any one of (1) to (3) above.

The substance for treating and/or preventing psychotropic drugs intolerance disease designed according to the above-mentioned method.

The preparation method of the above-mentioned substance for treating and/or preventing psychotropic drugs intolerance disease, wherein the stable structure corresponding to the substance related to psychotropic drugs intolerance disease is prepared by a high-temperature carbonization method.

Use of the above-mentioned substance for treating and/or preventing psychotropic drugs intolerance disease in the preparation of medicines, health products, foods, and food additives.

A method for designing substances for the treatment and/or prevention of antitumor drugs intolerance disease, wherein the stable structure corresponding to the substance related to antitumor drugs intolerance disease is used as the substance for treating and/or preventing antitumor drugs intolerance disease.

Preferably, the stable structure corresponding to the substance refers to the structure of said substance that exists in a solid state at a high temperature of 500 °C or higher.

Preferably, the stable structure corresponding to the substance related to antitumor drugs intolerance disease is selected from one or more of the following:
(1) the stable structure corresponding to the substance that causes antitumor drugs intolerance in the organism;
(2) the stable structure corresponding to the substance of the organism itself and related to antitumor drugs intolerance disease;
(3) the stable structure corresponding to the substance that inhibits the structure that causes antitumor drugs intolerance disease;
(4) the stable structure corresponding to the substance containing a substance of any one of (1) to (3) above.

The substance for treating and/or preventing antitumor drugs intolerance disease designed according to the above-mentioned method.

The preparation method of the above-mentioned substance for treating and/or preventing antitumor drugs intolerance disease, wherein the stable structure corresponding to the substance related to antitumor drugs intolerance disease is prepared by a high-temperature carbonization method.

Use of the above-mentioned substance for treating and/or preventing antitumor drugs intolerance disease in the preparation of medicines, health products, foods, and food additives.

A method for designing substances for the treatment and/or prevention of cardiovascular drugs intolerance disease, wherein the stable structure corresponding to the substance related to cardiovascular drugs intolerance disease is used as the substance for treating and/or preventing cardiovascular drugs intolerance disease.

Preferably, the stable structure corresponding to the substance refers to the structure of said substance that exists in a solid state at a high temperature of 500 °C or higher.

Preferably, the stable structure corresponding to the substance related to cardiovascular drugs intolerance disease is selected from one or more of the following:
(1) the stable structure corresponding to the substance that causes cardiovascular drugs intolerance in the organism;
(2) the stable structure corresponding to the substance of the organism itself and related to cardiovascular drugs intolerance disease;
(3) the stable structure corresponding to the substance that inhibits the structure that causes cardiovascular drugs intolerance disease;
(4) the stable structure corresponding to the substance containing a substance of any one of (1) to (3) above.

The substance for treating and/or preventing cardiovascular drugs intolerance disease designed according to the above-mentioned method.

The preparation method of the above-mentioned substance for treating and/or preventing cardiovascular drugs intolerance disease, wherein the stable structure corresponding to the substance related to cardiovascular drugs intolerance disease is prepared by a high-temperature carbonization method.

Use of the above-mentioned substance for treating and/or preventing cardiovascular drugs intolerance disease in the preparation of medicines, health products, foods, and food additives.

A method for designing substances for the treatment and/or prevention of statins intolerance disease, wherein the stable structure corresponding to the substance related to statins intolerance disease is used as the substance for treating and/or preventing statins intolerance disease.

Preferably, the stable structure corresponding to the substance refers to the structure of said substance that exists in a solid state at a high temperature of 500 °C or higher.

Preferably, the stable structure corresponding to the substance related to statins intolerance disease is selected from one or more of the following:
(1) the stable structure corresponding to the substance that causes statins intolerance in the organism;
(2) the stable structure corresponding to the substance of the organism itself and related to statins intolerance disease;
(3) the stable structure corresponding to the substance that inhibits the structure that causes statins intolerance disease;
(4) the stable structure corresponding to the substance containing a substance of any one of (1) to (3) above.

The substance for treating and/or preventing statins intolerance disease designed according to the above-mentioned method.

The preparation method of the above-mentioned substance for treating and/or preventing statins intolerance disease, wherein the stable structure corresponding to the substance related to statins intolerance disease is prepared by a high-temperature carbonization method.

Use of the above-mentioned substance for treating and/or preventing statins intolerance disease in the preparation of medicines, health products, foods, and food additives.

A method for designing substances for the treatment and/or prevention of nitrates intolerance disease, wherein the stable structure corresponding to the substance related to nitrates intolerance disease is used as the substance for treating and/or preventing nitrates intolerance disease.

Preferably, the stable structure corresponding to the substance refers to the structure of said substance that exists in a solid state at a high temperature of 500 °C or higher.

Preferably, the stable structure corresponding to the substance related to nitrates intolerance disease is selected from one or more of the following:
(1) the stable structure corresponding to the substance that causes nitrates intolerance in the organism;
(2) the stable structure corresponding to the substance of the organism itself and related to nitrates intolerance disease;
(3) the stable structure corresponding to the substance that inhibits the structure that causes nitrates intolerance disease;
(4) the stable structure corresponding to the substance containing a substance of any one of (1) to (3) above.

The substance for treating and/or preventing nitrates intolerance disease designed according to the above-mentioned method.

The preparation method of the above-mentioned substance for treating and/or preventing nitrates intolerance disease, wherein the stable structure corresponding to the substance related to nitrates intolerance disease is prepared by a high-temperature carbonization method.

Use of the above-mentioned substance for treating and/or preventing nitrates intolerance disease in the preparation of medicines, health products, foods, and food additives.

A method for designing substances for the treatment and/or prevention of respiratory drugs intolerance disease, wherein the stable structure corresponding to the substance related to respiratory drugs intolerance disease is used as the substance for treating and/or preventing respiratory drugs intolerance disease.

Preferably, the stable structure corresponding to the substance refers to the structure of said substance that exists in a solid state at a high temperature of 500 °C or higher.

Preferably, the stable structure corresponding to the substance related to respiratory drugs intolerance disease is selected from one or more of the following:
(1) the stable structure corresponding to the substance that causes respiratory drugs intolerance in the organism;
(2) the stable structure corresponding to the substance of the organism itself and related to respiratory drugs intolerance disease;
(3) the stable structure corresponding to the substance that inhibits the structure that causes respiratory drugs intolerance disease;
(4) the stable structure corresponding to the substance containing a substance of any one of (1) to (3) above.

The substance for treating and/or preventing respiratory drugs intolerance disease designed according to the above-mentioned method.

The preparation method of the above-mentioned substance for treating and/or preventing respiratory drugs intolerance disease, wherein the stable structure corresponding to the substance related to respiratory drugs intolerance disease is prepared by a high-temperature carbonization method.

Use of the above-mentioned substance for treating and/or preventing respiratory drugs intolerance disease in the preparation of medicines, health products, foods, and food additives.

A method for designing substances for the treatment and/or prevention of digestive drugs intolerance disease, wherein the stable structure corresponding to the substance related to digestive drugs intolerance disease is used as the substance for treating and/or preventing digestive drugs intolerance disease.

Preferably, the stable structure corresponding to the substance refers to the structure of said substance that exists in a solid state at a high temperature of 500 °C or higher.

Preferably, the stable structure corresponding to the substance related to digestive drugs intolerance disease is selected from one or more of the following:
(1) the stable structure corresponding to the substance that causes digestive drugs intolerance in the organism;
(2) the stable structure corresponding to the substance of the organism itself and related to digestive drugs intolerance disease;
(3) the stable structure corresponding to the substance that inhibits the structure that causes digestive drugs intolerance disease;
(4) the stable structure corresponding to the substance containing a substance of any one of (1) to (3) above.

The substance for treating and/or preventing digestive drugs intolerance disease designed according to the above-mentioned method.

The preparation method of the above-mentioned substance for treating and/or preventing digestive drugs intolerance disease, wherein the stable structure corresponding to the substance related to digestive drugs intolerance disease is prepared by a high-temperature carbonization method.

Use of the above-mentioned substance for treating and/or preventing digestive drugs intolerance disease in the preparation of medicines, health products, foods, and food additives.

A method for designing substances for the treatment and/or prevention of urinary system drugs intolerance disease, wherein the stable structure corresponding to the substance related to urinary system drugs intolerance disease is used as the substance for treating and/or preventing urinary system drugs intolerance disease.

Preferably, the stable structure corresponding to the substance refers to the structure of said substance that exists in a solid state at a high temperature of 500 °C or higher.

Preferably, the stable structure corresponding to the substance related to urinary system drugs intolerance disease is selected from one or more of the following:
(1) the stable structure corresponding to the substance that causes urinary system drugs intolerance in the organism;
(2) the stable structure corresponding to the substance of the organism itself and related to urinary system drugs intolerance disease;
(3) the stable structure corresponding to the substance that inhibits the structure that causes urinary system drugs intolerance disease;
(4) the stable structure corresponding to the substance containing a substance of any one of (1) to (3) above.

The substance for treating and/or preventing urinary system drugs intolerance disease designed according to the above-mentioned method.

The preparation method of the above-mentioned substance for treating and/or preventing urinary system drugs intolerance disease, wherein the stable structure corresponding to the substance related to urinary system drugs intolerance disease is prepared by a high-temperature carbonization method.

Use of the above-mentioned substance for treating and/or preventing urinary system drugs intolerance disease in the preparation of medicines, health products, foods, and food additives.

A method for designing substances for the treatment and/or prevention of hormones and related drugs intolerance disease, wherein the stable structure corresponding to the substance related to hormones and related drugs intolerance disease is used as the substance for treating and/or preventing hormones and related drugs intolerance disease.

Preferably, the stable structure corresponding to the substance refers to the structure of said substance that exists in a solid state at a high temperature of 500 °C or higher.

Preferably, the stable structure corresponding to the substance related to hormones and related drugs intolerance disease is selected from one or more of the following:
(1) the stable structure corresponding to the substance that causes hormones and related drugs intolerance in the organism;
(2) the stable structure corresponding to the substance of the organism itself and related to hormones and related drugs intolerance disease;
(3) the stable structure corresponding to the substance that inhibits the structure that causes hormones and related drugs intolerance disease;
(4) the stable structure corresponding to the substance containing a substance of any one of (1) to (3) above.

The substance for treating and/or preventing hormones and related drugs intolerance disease designed according to the above-mentioned method.

The preparation method of the above-mentioned substance for treating and/or preventing hormones and related drugs intolerance disease, wherein the stable structure corresponding to the substance related to hormones and related drugs intolerance disease is prepared by a high-temperature carbonization method.

Use of the above-mentioned substance for treating and/or preventing hormones and related drugs intolerance disease in the preparation of medicines, health products, foods, and food additives.

A method for designing substances for the treatment and/or prevention of antithroid drugs intolerance disease, wherein the stable structure corresponding to the substance related to antithroid drugs intolerance disease is used as the substance for treating and/or preventing antithroid drugs intolerance disease.

Preferably, the stable structure corresponding to the substance refers to the structure of said substance that exists in a solid state at a high temperature of 500 °C or higher.

Preferably, the stable structure corresponding to the substance related to antithroid drugs intolerance disease is selected from one or more of the following:
(1) the stable structure corresponding to the substance that causes antithroid drugs intolerance in the organism;
(2) the stable structure corresponding to the substance of the organism itself and related to antithroid drugs intolerance disease;
(3) the stable structure corresponding to the substance that inhibits the structure that causes antithroid drugs intolerance disease;
(4) the stable structure corresponding to the substance containing a substance of any one of (1) to (3) above.

The substance for treating and/or preventing antithroid drugs intolerance disease designed according to the above-mentioned method.

The preparation method of the above-mentioned substance for treating and/or preventing antithroid drugs intolerance disease, wherein the stable structure corresponding to the substance related to antithroid drugs intolerance disease is prepared by a high-temperature carbonization method.

Use of the above-mentioned substance for treating and/or preventing antithroid drugs intolerance disease in the preparation of medicines, health products, foods, and food additives.

A method for designing substances for the treatment and/or prevention of blood sugar regulating drugs intolerance disease, wherein the stable structure corresponding to the substance related to blood sugar regulating drugs intolerance disease is used as the substance for treating and/or preventing blood sugar regulating drugs intolerance disease.

Preferably, the stable structure corresponding to the substance refers to the structure of said substance that exists in a solid state at a high temperature of 500 °C or higher.

Preferably, the stable structure corresponding to the substance related to blood sugar regulating drugs intolerance disease is selected from one or more of the following:
(1) the stable structure corresponding to the substance that causes blood sugar regulating drugs intolerance in the organism;
(2) the stable structure corresponding to the substance of the organism itself and related to blood sugar regulating drugs intolerance disease;
(3) the stable structure corresponding to the substance that inhibits the structure that causes blood sugar regulating drugs intolerance disease;
(4) the stable structure corresponding to the substance containing a substance of any one of (1) to (3) above.

The substance for treating and/or preventing blood sugar regulating drugs intolerance disease designed according to the above-mentioned method.

The preparation method of the above-mentioned substance for treating and/or preventing blood sugar regulating drugs intolerance disease, wherein the stable structure corresponding to the substance related to blood sugar regulating drugs intolerance disease is prepared by a high-temperature carbonization method.

Use of the above-mentioned substance for treating and/or preventing blood sugar regulating drugs intolerance disease in the preparation of medicines, health products, foods, and food additives.

A method for designing substances for the treatment and/or prevention of skin intolerance disease, wherein the stable structure corresponding to the substance related to skin intolerance disease is used as the substance for treating and/or preventing skin intolerance disease.

Preferably, the stable structure corresponding to the substance refers to the structure of said substance that exists in a solid state at a high temperature of 500 °C or higher.

Preferably, the stable structure corresponding to the substance related to skin intolerance disease is selected from one or more of the following:
(1) the stable structure corresponding to the substance that causes skin intolerance in the organism;
(2) the stable structure corresponding to the substance of the organism itself and related to skin intolerance disease;
(3) the stable structure corresponding to the substance that inhibits the structure that causes skin intolerance disease;
(4) the stable structure corresponding to the substance containing a substance of any one of (1) to (3) above.

The substance for treating and/or preventing skin intolerance disease designed according to the above-mentioned method.

The preparation method of the above-mentioned substance for treating and/or preventing skin intolerance disease, wherein the stable structure corresponding to the substance related to skin intolerance disease is prepared by a high-temperature carbonization method.

Use of the above-mentioned substance for treating and/or preventing skin intolerance disease in the preparation of medicines, health products, foods, and food additives.

A method for designing substances for the treatment and/or prevention of skin care products intolerance disease, wherein the stable structure corresponding to the substance related to skin care products intolerance disease is used as the substance for treating and/or preventing skin care products intolerance disease.

Preferably, the stable structure corresponding to the substance refers to the structure of said substance that exists in a solid state at a high temperature of 500 °C or higher.

Preferably, the stable structure corresponding to the substance related to skin care products intolerance disease is selected from one or more of the following:
(1) the stable structure corresponding to the substance that causes skin care products intolerance in the organism;
(2) the stable structure corresponding to the substance of the organism itself and related to skin care products intolerance disease;
(3) the stable structure corresponding to the substance that inhibits the structure that causes skin care products intolerance disease;
(4) the stable structure corresponding to the substance containing a substance of any one of (1) to (3) above.

The substance for treating and/or preventing skin care products intolerance disease designed according to the above-mentioned method.

The preparation method of the above-mentioned substance for treating and/or preventing skin care products intolerance disease, wherein the stable structure corresponding to the substance related to skin care products intolerance disease is prepared by a high-temperature carbonization method.

Use of the above-mentioned substance for treating and/or preventing skin care products intolerance disease in the preparation of medicines, health products, foods, and food additives.

A method for designing substances for the treatment and/or prevention of cosmetics intolerance disease, wherein the stable structure corresponding to the substance related to cosmetics intolerance disease is used as the substance for treating and/or preventing cosmetics intolerance disease.

Preferably, the stable structure corresponding to the substance refers to the structure of said substance that exists in a solid state at a high temperature of 500 °C or higher.

Preferably, the stable structure corresponding to the substance related to cosmetics intolerance disease is selected from one or more of the following:
(1) the stable structure corresponding to the substance that causes cosmetics intolerance in the organism;
(2) the stable structure corresponding to the substance of the organism itself and related to cosmetics intolerance disease;
(3) the stable structure corresponding to the substance that inhibits the structure that causes cosmetics intolerance disease;
(4) the stable structure corresponding to the substance containing a substance of any one of (1) to (3) above.

The substance for treating and/or preventing cosmetics intolerance disease designed according to the above-mentioned method.

The preparation method of the above-mentioned substance for treating and/or preventing cosmetics intolerance disease, wherein the stable structure corresponding to the substance related to cosmetics intolerance disease is prepared by a high-temperature carbonization method.

Use of the above-mentioned substance for treating and/or preventing cosmetics intolerance disease in the preparation of medicines, health products, foods, and food additives.

The organisms in the content of the present invention can use the same or similar species, preferably pigs, cattle, sheep, and primates.

The essence of health is the dynamic balance of the structural system, which is the result of the interactions between the structures in the structural system. The most fundamental interactions are promotion and restriction. In a biological structural system, if a structure lacks a structure that promotes it, this structure will be lacking, and if it lacks a structure that restricts it, this structure will be excessive, both of these situations will cause an imbalance in the structural system. The essence of disease is the imbalance of biological structure system. As can be seen from the above technical solutions, the method for treating or preventing diseases disclosed in the present invention uses the stable structure of the substance related to diseases as an external structure to act on the biological structural system (e.g. via oral administration), and the biological structural system regulates the gene or gene expression by recognizing and transmitting the structural information of the external structure and using its own self-adaptation, self-organization and other functions, so as to treat or prevent the diseases related to the substance.

It can be seen from the above technical solutions that the beneficial effects of the substances for treating and/or preventing diseases and their design methods and preparation methods disclosed in the present invention include:
1. The design method and the preparation method of the substance for treating and/or preventing diseases according to the present invention are not based on the concrete structure of the substance causing diseases or the specific pathogenic mechanism, but are characterized in that a stable structure corresponding to the substance which is the same as or similar to an external substance or a substance in the body that causes diseases is used as an external structure to act on a biological structural system, and said external structure enable organisms to eliminate or alleviate diseases or prevent the occurrence of diseases according to structural self-adaptation and self-organization. Therefore, the method of the present invention is versatile.
2. The present invention combines the concrete structure of Western medicine research and the macroscopic theory established by TCM, treats and/or prevents diseases from the perspective of a biological system. Relatively speaking, the system is limited, and the disease is unlimited, because different lesions of the same organ will cause different diseases, and the symptoms of the same disease in different individuals are not exactly the same. Whether in accordance with Western medicine or TCM, from a systematic point of view, people are the same, and even humans, pigs, cows, sheep, and primates are basically the same. Therefore, when designing substance for treating and/or preventing diseases based on the system, for diseases caused by internal diseases of organisms, only the specific diseased cells, tissues or organs need to be known, and there is no need to explore the pathogenic mechanism and specific symptoms of the disease. You only need to know the system where the disease-related substances are located or the cells, tissues or organs belonging to the system, and use the corresponding stable structure as an external structure to act on the biological structural system. The structural system of the organism enable the organism to establish the balance with the structure of the external substance or restore the structural imbalance in the biological structural system according to structural self-adaptation and self-organization, thereby eliminating, relieving the diseases or preventing the diseases.
3. The substance for treating and/or preventing diseases and design method and preparation method thereof according to the present invention avoid the research on the disease mechanism and greatly save the investment of time, manpower, material resources and financial resources. It greatly shortens the cycle of drug research and development and reduces the cost of disease treatment, and greatly simplifies drug research and development, disease diagnosis, and treatment.

### Detailed Description

The present invention discloses substances for treating and/or preventing intolerance diseases, the design method and the preparation method thereof. Those skilled in the art can learn from the content of this article, use different materials, improve the process or adopt other similar processes for preparation, all of which are considered to be included in the present invention. In particular, it should be pointed out that all such substitutions and modifications will be apparent to those skilled in the art and are intended to be included herein. While the methods and products of the present invention have been described in terms of preferred embodiments, it will be apparent to those of ordinary skill in the art that variations and modifications of the methods described herein, as well as appropriate variations and combinations, may be made to implement and use the techniques of the present invention without departing from the spirit and scope of the invention.

In order to further understand the present invention, the technical solutions in the embodiments of the present invention will be clearly and completely described below with reference to the examples of the present invention, and it is obvious that the described examples are only a part of the embodiments of the present invention, and not all of the embodiments. All other embodiments, which can be obtained by a person skilled in the art without making any creative effort based on the embodiments in the present invention, belong to the protection scope of the present invention.

The specific embodiments are as follows:

### Example 1: Substance for treating and/or preventing egg intolerance and preparation method thereof

Since eggs contain substances causing egg intolerance and are easily available, it is preferable to use the stable structure corresponding to egg as the substance for treating and/or preventing egg intolerance. If other organs or tissues also contain the substance related to egg intolerance disease, it is also preferable to use the stable structure corresponding to the organ or tissue containing the substance related to egg intolerane disease as the substance for treating and/or preventing egg intolerane, so that the complicated problems can be simplified, and the substance for treating and/or preventing egg intolerane can be designed and prepared without knowing the specific structure of the intolerant substance and pathogenic mechanism.

The egg is carbonized at high temperature to obtain the substance for treating and/or preventing egg intolerane.

### Example 2: Substance for treating and/or preventing wheat intolerance and preparation method thereof

Since wheats contain substances causing wheat intolerance and are easily available, it is preferable to use the stable structure corresponding to wheat as the substance for treating and/or preventing wheat intolerance. If other organs or tissues also contain the substance related to wheat intolerance disease, it is also preferable to use the stable structure corresponding to the organ or tissue containing the substance related to wheat intolerane disease as the substance for treating and/or preventing wheat intolerane, so that the complicated problems can be simplified, and the substance for treating and/or preventing wheat intolerane can be designed and prepared without knowing the specific structure of the intolerant substance and pathogenic mechanism.

The wheat is carbonized at high temperature to obtain the substance for treating and/or preventing wheat intolerane.

Using the same method, the present invention prepared a series of substances for treating and/or preventing intolerane diseases caused by different intolerant substances.

### Example 3: Clinical application test of the substances prepared in Example 1 and Example 2 and various substances for treating intolerance diseases prepared in the same manner in the treatment of intolerance diseases

1. **Clinical data:** A total of 60 outpatients were recruited, of which 28 were males and 32 were females, aged 4 to 68 years old, including 28 cases of gastrointestinal discomfort (symptoms include various degrees of abdominal pain, constipation or diarrhea, abdominal fullness, bad breath, etc.), 25 cases of skin reactions (various dermatitis, urticaria, erythema, etc.), 13 cases of migraine and sleep disorders, and other diseases such as asthma, rhinitis, joint pain, etc.
2. **Diagnostic criteria:** A double-blind placebo-controlled drug challenge test was performed based on the patient's medical history as the diagnostic criteria for testing drug intolerance.
3. **Treatment method:** Patients were orally administered the substance for treating intolerance diseases prepared in Example 1 and Example 2, once a day, 3 g/time, for 3 days continuously.
4. **Criteria for judging the treatment effect:** According to the changes in the clinical symptoms of patients after treatment.
5. **Efficacy results:** After treatment, up to 55 patients were cured and 4 patients were markedly improved, with a total effective rate of 98.33%. After treatment, these patients did not show any symptoms after repeated administration of drugs that caused intolerance. At the same time, there were no side effects and drug dependence during the observation period.

## Claims

1. A method for designing substances for treating and/or preventing intolerance diseases, **characterized in that**:
the stable structure corresponding to the substance related to the intolerance disease is used as the substance for treating and/or preventing said intolerance disease; and
the stable structure corresponding to the substance is **characterized by** the structure of said substance that exists in a solid state at a high temperature of 500 °C or higher.

2. The method according to claim 1, **characterized in that** said stable structure corresponding to the substance related to the intolerance disease is selected from one or more of the following:
(1) the stable structure corresponding to the substance that causes intolerance disease in the organism;
(2) the stable structure corresponding to the substance of the organism itself and related to intolerance disease;
(3) the stable structure corresponding to the substance that inhibits the structure that causes intolerance disease;
(4) the stable structure corresponding to the substance containing a substance of any one of (1) to (3) above.

3. A substance for treating and/or preventing said diseases designed according to the method of any one of claims 1-2.

4. The preparation method of the substance for treating and/or preventing intolerance disease according to claim 3, **characterized in that** the stable structure corresponding to the substance is prepared by a high-temperature carbonization method.

5. A method for designing substances for treating and/or preventing food intolerance diseases, **characterized in that**:
the stable structure corresponding to the substance related to the food intolerance disease is used as the substance for treating and/or preventing said food intolerance disease; and
the stable structure corresponding to the substance is **characterized by** the structure of said substance that exists in a solid state at a high temperature of 500 °C or higher.

6. The method according to claim 1, **characterized in that** said stable structure corresponding to the substance related to the intolerance disease is selected from one or more of the following:
(1) the stable structure corresponding to the substance that causes food intolerance disease in the organism;
(2) the stable structure corresponding to the substance of the organism itself and related to food intolerance disease;
(3) the stable structure corresponding to the substance that inhibits the structure that causes food intolerance disease;
(4) the stable structure corresponding to the substance containing a substance of any one of (1) to (3) above.

7. A substance for treating and/or preventing food intolerance disease designed according to the method of any one of claims 5-6.

8. The preparation method of the substance for treating and/or preventing food intolerance disease according to claim 7, **characterized in that** the stable structure corresponding to the substance is prepared by a high-temperature carbonization method.

9. A method for designing substances for treating and/or preventing barley intolerance, **characterized in that**:
the stable structure corresponding to the substance related to the barley intolerance is used as the substance for treating and/or preventing said barley intolerance; and
the stable structure corresponding to the substance is **characterized by** the structure of said substance that exists in a solid state at a high temperature of 500 °C or higher.

10. The method according to claim 9, **characterized in that** said stable structure corresponding to the substance related to the intolerance disease is selected from one or more of the following:
(1) the stable structure corresponding to the substance that causes barley intolerance in the organism;
(2) the stable structure corresponding to the substance of the organism itself and related to barley intolerance;
(3) the stable structure corresponding to the substance that inhibits the structure that causes barley intolerance;
(4) the stable structure corresponding to the substance containing a substance of any one of (1) to (3) above.

11. A substance for treating and/or preventing barley intolerance designed according to the method of any one of claims 9-10.

12. The preparation method of the substance for treating and/or preventing barley intolerance according to claim 11, **characterized in that** the stable structure corresponding to the substance is prepared by a high-temperature carbonization method.

13. A method for designing substances for treating and/or preventing corn intolerance, **characterized in that**:
the stable structure corresponding to the substance related to the corn intolerance is used as the substance for treating and/or preventing said corn intolerance; and
the stable structure corresponding to the substance is **characterized by** the structure of said substance that exists in a solid state at a high temperature of 500 °C or higher.

14. The method according to claim 13, **characterized in that** said stable structure corresponding to the substance related to the intolerance disease is selected from one or more of the following:
(1) the stable structure corresponding to the substance that causes corn intolerance in the organism;
(2) the stable structure corresponding to the substance of the organism itself and related to corn intolerance;
(3) the stable structure corresponding to the substance that inhibits the structure that causes corn intolerance;
(4) the stable structure corresponding to the substance containing a substance of any one of (1) to (3) above.

15. A substance for treating and/or preventing corn intolerance designed according to the method of any one of claims 13-14.

16. The preparation method of the substance for treating and/or preventing corn intolerance according to claim 15, **characterized in that** the stable structure corresponding to the substance is prepared by a high-temperature carbonization method.

17. A method for designing substances for treating and/or preventing millet intolerance, **characterized in that**:
the stable structure corresponding to the substance related to the millet intolerance is used as the substance for treating and/or preventing said millet intolerance; and
the stable structure corresponding to the substance is **characterized by** the structure of said substance that exists in a solid state at a high temperature of 500 °C or higher.

18. The method according to claim 17, **characterized in that** said stable structure corresponding to the substance related to the intolerance disease is selected from one or more of the following:
(1) the stable structure corresponding to the substance that causes millet intolerance in the organism;
(2) the stable structure corresponding to the substance of the organism itself and related to millet intolerance;
(3) the stable structure corresponding to the substance that inhibits the structure that causes millet intolerance;
(4) the stable structure corresponding to the substance containing a substance of any one of (1) to (3) above.

19. A substance for treating and/or preventing millet intolerance designed according to the method of any one of claims 17-18.

20. The preparation method of the substance for treating and/or preventing millet intolerance according to claim 19, **characterized in that** the stable structure corresponding to the substance is prepared by a high-temperature carbonization method.

21. A method for designing substances for treating and/or preventing apple intolerance, **characterized in that**:
the stable structure corresponding to the substance related to the apple intolerance is used as the substance for treating and/or preventing said apple intolerance; and
the stable structure corresponding to the substance is **characterized by** the structure of said substance that exists in a solid state at a high temperature of 500 °C or higher.

22. The method according to claim 21, **characterized in that** said stable structure corresponding to the substance related to the intolerance disease is selected from one or more of the following:
(1) the stable structure corresponding to the substance that causes apple intolerance in the organism;
(2) the stable structure corresponding to the substance of the organism itself and related to apple intolerance;
(3) the stable structure corresponding to the substance that inhibits the structure that causes apple intolerance;
(4) the stable structure corresponding to the substance containing a substance of any one of (1) to (3) above.

23. A substance for treating and/or preventing apple intolerance designed according to the method of any one of claims 20-21.

24. The preparation method of the substance for treating and/or preventing apple intolerance according to claim 23, **characterized in that** the stable structure corresponding to the substance is prepared by a high-temperature carbonization method.

25. A method for designing substances for treating and/or preventing banana intolerance, **characterized in that**:
the stable structure corresponding to the substance related to the banana intolerance is used as the substance for treating and/or preventing said banana intolerance; and
the stable structure corresponding to the substance is **characterized by** the structure of said substance that exists in a solid state at a high temperature of 500 °C or higher.

26. The method according to claim 25, **characterized in that** said stable structure corresponding to the substance related to the intolerance disease is selected from one or more of the following:
(1) the stable structure corresponding to the substance that causes banana intolerance in the organism;
(2) the stable structure corresponding to the substance of the organism itself and related to banana intolerance;
(3) the stable structure corresponding to the substance that inhibits the structure that causes banana intolerance;
(4) the stable structure corresponding to the substance containing a substance of any one of (1) to (3) above.

27. A substance for treating and/or preventing banana intolerance designed according to the method of any one of claims 25-26.

28. The preparation method of the substance for treating and/or preventing banana intolerance according to claim 27, **characterized in that** the stable structure corresponding to the substance is prepared by a high-temperature carbonization method.

29. A method for designing substances for treating and/or preventing grape intolerance, **characterized in that**:
the stable structure corresponding to the substance related to the grape intolerance is used as the substance for treating and/or preventing said grape intolerance; and
the stable structure corresponding to the substance is **characterized by** the structure of said substance that exists in a solid state at a high temperature of 500 °C or higher.

30. The method according to claim 29, **characterized in that** said stable structure corresponding to the substance related to the intolerance disease is selected from one or more of the following:
(1) the stable structure corresponding to the substance that causes grape intolerance in the organism;
(2) the stable structure corresponding to the substance of the organism itself and related to grape intolerance;
(3) the stable structure corresponding to the substance that inhibits the structure that causes grape intolerance;
(4) the stable structure corresponding to the substance containing a substance of any one of (1) to (3) above.

31. A substance for treating and/or preventing grape intolerance designed according to the method of any one of claims 29-30.

32. The preparation method of the substance for treating and/or preventing grape intolerance according to claim 31, **characterized in that** the stable structure corresponding to the substance is prepared by a high-temperature carbonization method.

33. A method for designing substances for treating and/or preventing beef intolerance, **characterized in that**:
the stable structure corresponding to the substance related to the beef intolerance is used as the substance for treating and/or preventing said beef intolerance; and
the stable structure corresponding to the substance is **characterized by** the structure of said substance that exists in a solid state at a high temperature of 500 °C or higher.

34. The method according to claim 33, **characterized in that** said stable structure corresponding to the substance related to the intolerance disease is selected from one or more of the following:
(1) the stable structure corresponding to the substance that causes beef intolerance in the organism;
(2) the stable structure corresponding to the substance of the organism itself and related to beef intolerance;
(3) the stable structure corresponding to the substance that inhibits the structure that causes beef intolerance;
(4) the stable structure corresponding to the substance containing a substance of any one of (1) to (3) above.

35. A substance for treating and/or preventing beef intolerance designed according to the method of any one of claims 33-34.

36. The preparation method of the substance for treating and/or preventing beef intolerance according to claim 35, **characterized in that** the stable structure corresponding to the substance is prepared by a high-temperature carbonization method.

37. A method for designing substances for treating and/or preventing chiken intolerance, **characterized in that**:
the stable structure corresponding to the substance related to the chiken intolerance is used as the substance for treating and/or preventing said chiken intolerance; and
the stable structure corresponding to the substance is **characterized by** the structure of said substance that exists in a solid state at a high temperature of 500 °C or higher.

38. The method according to claim 37, **characterized in that** said stable structure corresponding to the substance related to the intolerance disease is selected from one or more of the following:
(1) the stable structure corresponding to the substance that causes chiken intolerance in the organism;
(2) the stable structure corresponding to the substance of the organism itself and related to chiken intolerance;
(3) the stable structure corresponding to the substance that inhibits the structure that causes chiken intolerance;
(4) the stable structure corresponding to the substance containing a substance of any one of (1) to (3) above.

39. A substance for treating and/or preventing chiken intolerance designed according to the method of any one of claims 37-38.

40. The preparation method of the substance for treating and/or preventing chiken intolerance according to claim 39, **characterized in that** the stable structure corresponding to the substance is prepared by a high-temperature carbonization method.

41. A method for designing substances for treating and/or preventing carrot intolerance, **characterized in that**:
the stable structure corresponding to the substance related to the carrot intolerance is used as the substance for treating and/or preventing said carrot intolerance; and
the stable structure corresponding to the substance is **characterized by** the structure of said substance that exists in a solid state at a high temperature of 500 °C or higher.

42. The method according to claim 41, **characterized in that** said stable structure corresponding to the substance related to the intolerance disease is selected from one or more of the following:
(1) the stable structure corresponding to the substance that causes carrot intolerance in the organism;
(2) the stable structure corresponding to the substance of the organism itself and related to carrot intolerance;
(3) the stable structure corresponding to the substance that inhibits the structure that causes carrot intolerance;
(4) the stable structure corresponding to the substance containing a substance of any one of (1) to (3) above.

43. A substance for treating and/or preventing carrot intolerance designed according to the method of any one of claims 41-42.

44. The preparation method of the substance for treating and/or preventing carrot intolerance according to claim 43, **characterized in that** the stable structure corresponding to the substance is prepared by a high-temperature carbonization method.

45. A method for designing substances for treating and/or preventing cucumber intolerance, **characterized in that**:
the stable structure corresponding to the substance related to the cucumber intolerance is used as the substance for treating and/or preventing said cucumber intolerance; and
the stable structure corresponding to the substance is **characterized by** the structure of said substance that exists in a solid state at a high temperature of 500 °C or higher.

46. The method according to claim 45, **characterized in that** said stable structure corresponding to the substance related to the intolerance disease is selected from one or more of the following:
(1) the stable structure corresponding to the substance that causes cucumber intolerance in the organism;
(2) the stable structure corresponding to the substance of the organism itself and related to cucumber intolerance;
(3) the stable structure corresponding to the substance that inhibits the structure that causes cucumber intolerance;
(4) the stable structure corresponding to the substance containing a substance of any one of (1) to (3) above.

47. A substance for treating and/or preventing cucumber intolerance designed according to the method of any one of claims 45-46.

48. The preparation method of the substance for treating and/or preventing cucumber intolerance according to claim 47, **characterized in that** the stable structure corresponding to the substance is prepared by a high-temperature carbonization method.

49. A method for designing substances for treating and/or preventing eggplant intolerance, **characterized in that**:
the stable structure corresponding to the substance related to the eggplant intolerance is used as the substance for treating and/or preventing said eggplant intolerance; and
the stable structure corresponding to the substance is **characterized by** the structure of said substance that exists in a solid state at a high temperature of 500 °C or higher.

50. The method according to claim 49, **characterized in that** said stable structure corresponding to the substance related to the intolerance disease is selected from one or more of the following:
(1) the stable structure corresponding to the substance that causes eggplant intolerance in the organism;
(2) the stable structure corresponding to the substance of the organism itself and related to eggplant intolerance;
(3) the stable structure corresponding to the substance that inhibits the structure that causes eggplant intolerance;
(4) the stable structure corresponding to the substance containing a substance of any one of (1) to (3) above.

51. A substance for treating and/or preventing eggplant intolerance designed according to the method of any one of claims 49-50.

52. The preparation method of the substance for treating and/or preventing eggplant intolerance according to claim 51, **characterized in that** the stable structure corresponding to the substance is prepared by a high-temperature carbonization method.

53. A method for designing substances for treating and/or preventing celery intolerance, **characterized in that**:
the stable structure corresponding to the substance related to the celery intolerance is used as the substance for treating and/or preventing said celery intolerance; and
the stable structure corresponding to the substance is **characterized by** the structure of said substance that exists in a solid state at a high temperature of 500 °C or higher.

54. The method according to claim 53, **characterized in that** said stable structure corresponding to the substance related to the intolerance disease is selected from one or more of the following:
(1) the stable structure corresponding to the substance that causes celery intolerance in the organism;
(2) the stable structure corresponding to the substance of the organism itself and related to celery intolerance;
(3) the stable structure corresponding to the substance that inhibits the structure that causes celery intolerance;
(4) the stable structure corresponding to the substance containing a substance of any one of (1) to (3) above.

55. A substance for treating and/or preventing celery intolerance designed according to the method of any one of claims 53-54.

56. The preparation method of the substance for treating and/or preventing celery intolerance according to claim 55, **characterized in that** the stable structure corresponding to the substance is prepared by a high-temperature carbonization method.

57. A method for designing substances for treating and/or preventing butter intolerance, **characterized in that**:
the stable structure corresponding to the substance related to the butter intolerance is used as the substance for treating and/or preventing said butter intolerance; and
the stable structure corresponding to the substance is **characterized by** the structure of said substance that exists in a solid state at a high temperature of 500 °C or higher.

58. The method according to claim 57, **characterized in that** said stable structure corresponding to the substance related to the intolerance disease is selected from one or more of the following:
(1) the stable structure corresponding to the substance that causes butter intolerance in the organism;
(2) the stable structure corresponding to the substance of the organism itself and related to butter intolerance;
(3) the stable structure corresponding to the substance that inhibits the structure that causes butter intolerance;
(4) the stable structure corresponding to the substance containing a substance of any one of (1) to (3) above.

59. A substance for treating and/or preventing butter intolerance designed according to the method of any one of claims 57-58.

60. The preparation method of the substance for treating and/or preventing butter intolerance according to claim 59, **characterized in that** the stable structure corresponding to the substance is prepared by a high-temperature carbonization method.

61. A method for designing substances for treating and/or preventing milk intolerance, **characterized in that**:
the stable structure corresponding to the substance related to the milk intolerance is used as the substance for treating and/or preventing said milk intolerance; and
the stable structure corresponding to the substance is **characterized by** the structure of said substance that exists in a solid state at a high temperature of 500 °C or higher.

62. The method according to claim 61, **characterized in that** said stable structure corresponding to the substance related to the intolerance disease is selected from one or more of the following:
(1) the stable structure corresponding to the substance that causes milk intolerance in the organism;
(2) the stable structure corresponding to the substance of the organism itself and related to milk intolerance;
(3) the stable structure corresponding to the substance that inhibits the structure that causes milk intolerance;
(4) the stable structure corresponding to the substance containing a substance of any one of (1) to (3) above.

63. A substance for treating and/or preventing milk intolerance designed according to the method of any one of claims 61-62.

64. The preparation method of the substance for treating and/or preventing milk intolerance according to claim 63, **characterized in that** the stable structure corresponding to the substance is prepared by a high-temperature carbonization method.

65. A method for designing substances for treating and/or preventing lima beans intolerance, **characterized in that**:
the stable structure corresponding to the substance related to the lima beans intolerance is used as the substance for treating and/or preventing said lima beans intolerance; and
the stable structure corresponding to the substance is **characterized by** the structure of said substance that exists in a solid state at a high temperature of 500 °C or higher.

66. The method according to claim 65, **characterized in that** said stable structure corresponding to the substance related to the intolerance disease is selected from one or more of the following:
(1) the stable structure corresponding to the substance that causes lima beans intolerance in the organism;
(2) the stable structure corresponding to the substance of the organism itself and related to lima beans intolerance;
(3) the stable structure corresponding to the substance that inhibits the structure that causes lima beans intolerance;
(4) the stable structure corresponding to the substance containing a substance of any one of (1) to (3) above.

67. A substance for treating and/or preventing lima beans intolerance designed according to the method of any one of claims 65-66.

68. The preparation method of the substance for treating and/or preventing lima beans intolerance according to claim 67, **characterized in that** the stable structure corresponding to the substance is prepared by a high-temperature carbonization method.

69. A method for designing substances for treating and/or preventing honey intolerance, **characterized in that**:
the stable structure corresponding to the substance related to the honey intolerance is used as the substance for treating and/or preventing said honey intolerance; and
the stable structure corresponding to the substance is **characterized by** the structure of said substance that exists in a solid state at a high temperature of 500 °C or higher.

70. The method according to claim 69, **characterized in that** said stable structure corresponding to the substance related to the intolerance disease is selected from one or more of the following:
(1) the stable structure corresponding to the substance that causes honey intolerance in the organism;
(2) the stable structure corresponding to the substance of the organism itself and related to honey intolerance;
(3) the stable structure corresponding to the substance that inhibits the structure that causes honey intolerance;
(4) the stable structure corresponding to the substance containing a substance of any one of (1) to (3) above.

71. A substance for treating and/or preventing honey intolerance designed according to the method of any one of claims 69-70.

72. The preparation method of the substance for treating and/or preventing honey intolerance according to claim 71, **characterized in that** the stable structure corresponding to the substance is prepared by a high-temperature carbonization method.

73. A method for designing substances for treating and/or preventing cashew intolerance, **characterized in that**:
the stable structure corresponding to the substance related to the cashew intolerance is used as the substance for treating and/or preventing said cashew intolerance; and
the stable structure corresponding to the substance is **characterized by** the structure of said substance that exists in a solid state at a high temperature of 500 °C or higher.

74. The method according to claim 73, **characterized in that** said stable structure corresponding to the substance related to the intolerance disease is selected from one or more of the following:
(1) the stable structure corresponding to the substance that causes cashew intolerance in the organism;
(2) the stable structure corresponding to the substance of the organism itself and related to cashew intolerance;
(3) the stable structure corresponding to the substance that inhibits the structure that causes cashew intolerance;
(4) the stable structure corresponding to the substance containing a substance of any one of (1) to (3) above.

75. A substance for treating and/or preventing cashew intolerance designed according to the method of any one of claims 73-74.

76. The preparation method of the substance for treating and/or preventing cashew intolerance according to claim 75, **characterized in that** the stable structure corresponding to the substance is prepared by a high-temperature carbonization method.

77. A method for designing substances for treating and/or preventing peanut intolerance, **characterized in that**:
the stable structure corresponding to the substance related to the peanut intolerance is used as the substance for treating and/or preventing said peanut intolerance; and
the stable structure corresponding to the substance is **characterized by** the structure of said substance that exists in a solid state at a high temperature of 500 °C or higher.

78. The method according to claim 77, **characterized in that** said stable structure corresponding to the substance related to the intolerance disease is selected from one or more of the following:
(1) the stable structure corresponding to the substance that causes peanut intolerance in the organism;
(2) the stable structure corresponding to the substance of the organism itself and related to peanut intolerance;
(3) the stable structure corresponding to the substance that inhibits the structure that causes peanut intolerance;
(4) the stable structure corresponding to the substance containing a substance of any one of (1) to (3) above.

79. A substance for treating and/or preventing peanut intolerance designed according to the method of any one of claims 77-78.

80. The preparation method of the substance for treating and/or preventing peanut intolerance according to claim 79, **characterized in that** the stable structure corresponding to the substance is prepared by a high-temperature carbonization method.

81. A method for designing substances for treating and/or preventing soybean intolerance, **characterized in that**:
the stable structure corresponding to the substance related to the soybean intolerance is used as the substance for treating and/or preventing said soybean intolerance; and
the stable structure corresponding to the substance is **characterized by** the structure of said substance that exists in a solid state at a high temperature of 500 °C or higher.

82. The method according to claim 81, **characterized in that** said stable structure corresponding to the substance related to the intolerance disease is selected from one or more of the following:
(1) the stable structure corresponding to the substance that causes soybean intolerance in the organism;
(2) the stable structure corresponding to the substance of the organism itself and related to soybean intolerance;
(3) the stable structure corresponding to the substance that inhibits the structure that causes soybean intolerance;
(4) the stable structure corresponding to the substance containing a substance of any one of (1) to (3) above.

83. A substance for treating and/or preventing soybean intolerance designed according to the method of any one of claims 81-82.

84. The preparation method of the substance for treating and/or preventing soybean intolerance according to claim 83, **characterized in that** the stable structure corresponding to the substance is prepared by a high-temperature carbonization method.

85. A method for designing substances for treating and/or preventing crab intolerance, **characterized in that**:
the stable structure corresponding to the substance related to the crab intolerance is used as the substance for treating and/or preventing said crab intolerance; and
the stable structure corresponding to the substance is **characterized by** the structure of said substance that exists in a solid state at a high temperature of 500 °C or higher.

86. The method according to claim 85, **characterized in that** said stable structure corresponding to the substance related to the intolerance disease is selected from one or more of the following:
(1) the stable structure corresponding to the substance that causes crab intolerance in the organism;
(2) the stable structure corresponding to the substance of the organism itself and related to crab intolerance;
(3) the stable structure corresponding to the substance that inhibits the structure that causes crab intolerance;
(4) the stable structure corresponding to the substance containing a substance of any one of (1) to (3) above.

87. A substance for treating and/or preventing crab intolerance designed according to the method of any one of claims 85-86.

88. The preparation method of the substance for treating and/or preventing crab intolerance according to claim 87, **characterized in that** the stable structure corresponding to the substance is prepared by a high-temperature carbonization method.

89. A method for designing substances for treating and/or preventing shrimp intolerance, **characterized in that**:
the stable structure corresponding to the substance related to the shrimp intolerance is used as the substance for treating and/or preventing said shrimp intolerance; and
the stable structure corresponding to the substance is **characterized by** the structure of said substance that exists in a solid state at a high temperature of 500 °C or higher.

90. The method according to claim 89, **characterized in that** said stable structure corresponding to the substance related to the intolerance disease is selected from one or more of the following:
(1) the stable structure corresponding to the substance that causes shrimp intolerance in the organism;
(2) the stable structure corresponding to the substance of the organism itself and related to shrimp intolerance;
(3) the stable structure corresponding to the substance that inhibits the structure that causes shrimp intolerance;
(4) the stable structure corresponding to the substance containing a substance of any one of (1) to (3) above.

91. A substance for treating and/or preventing shrimp intolerance designed according to the method of any one of claims 89-90.

92. The preparation method of the substance for treating and/or preventing shrimp intolerance according to claim 91, **characterized in that** the stable structure corresponding to the substance is prepared by a high-temperature carbonization method.

93. A method for designing substances for treating and/or preventing coffee intolerance, **characterized in that**:
the stable structure corresponding to the substance related to the coffee intolerance is used as the substance for treating and/or preventing said coffee intolerance; and
the stable structure corresponding to the substance is **characterized by** the structure of said substance that exists in a solid state at a high temperature of 500 °C or higher.

94. The method according to claim 93, **characterized in that** said stable structure corresponding to the substance related to the intolerance disease is selected from one or more of the following:
(1) the stable structure corresponding to the substance that causes coffee intolerance in the organism;
(2) the stable structure corresponding to the substance of the organism itself and related to coffee intolerance;
(3) the stable structure corresponding to the substance that inhibits the structure that causes coffee intolerance;
(4) the stable structure corresponding to the substance containing a substance of any one of (1) to (3) above.

95. A substance for treating and/or preventing coffee intolerance designed according to the method of any one of claims 93-94.

96. The preparation method of the substance for treating and/or preventing coffee intolerance according to claim 95, **characterized in that** the stable structure corresponding to the substance is prepared by a high-temperature carbonization method.

97. A method for designing substances for treating and/or preventing polysaccharide intolerance, **characterized in that**:
the stable structure corresponding to the substance related to the polysaccharide intolerance is used as the substance for treating and/or preventing said polysaccharide intolerance; and
the stable structure corresponding to the substance is **characterized by** the structure of said substance that exists in a solid state at a high temperature of 500 °C or higher.

98. The method according to claim 97, **characterized in that** said stable structure corresponding to the substance related to the intolerance disease is selected from one or more of the following:
(1) the stable structure corresponding to the substance that causes polysaccharide intolerance in the organism;
(2) the stable structure corresponding to the substance of the organism itself and related to polysaccharide intolerance;
(3) the stable structure corresponding to the substance that inhibits the structure that causes polysaccharide intolerance;
(4) the stable structure corresponding to the substance containing a substance of any one of (1) to (3) above.

99. A substance for treating and/or preventing polysaccharide intolerance designed according to the method of any one of claims 97-98.

100. The preparation method of the substance for treating and/or preventing polysaccharide intolerance according to claim 99, **characterized in that** the stable structure corresponding to the substance is prepared by a high-temperature carbonization method.

101. A method for designing substances for treating and/or preventing biogenic amine intolerance, **characterized in that**:
the stable structure corresponding to the substance related to the biogenic amine intolerance is used as the substance for treating and/or preventing said biogenic amine intolerance; and
the stable structure corresponding to the substance is **characterized by** the structure of said substance that exists in a solid state at a high temperature of 500 °C or higher.

102. The method according to claim 101, **characterized in that** said stable structure corresponding to the substance related to the intolerance disease is selected from one or more of the following:
(1) the stable structure corresponding to the substance that causes biogenic amine intolerance in the organism;
(2) the stable structure corresponding to the substance of the organism itself and related to biogenic amine intolerance;
(3) the stable structure corresponding to the substance that inhibits the structure that causes biogenic amine intolerance;
(4) the stable structure corresponding to the substance containing a substance of any one of (1) to (3) above.

103. A substance for treating and/or preventing biogenic amine intolerance designed according to the method of any one of claims 101-102.

104. The preparation method of the substance for treating and/or preventing biogenic amine intolerance according to claim 103, **characterized in that** the stable structure corresponding to the substance is prepared by a high-temperature carbonization method.

105. A method for designing substances for treating and/or preventing food additives intolerance, **characterized in that**:
the stable structure corresponding to the substance related to the food additives intolerance is used as the substance for treating and/or preventing said food additives intolerance; and
the stable structure corresponding to the substance is **characterized by** the structure of said substance that exists in a solid state at a high temperature of 500 °C or higher.

106. The method according to claim 105, **characterized in that** said stable structure corresponding to the substance related to the intolerance disease is selected from one or more of the following:
(1) the stable structure corresponding to the substance that causes food additives intolerance in the organism;
(2) the stable structure corresponding to the substance of the organism itself and related to food additives intolerance;
(3) the stable structure corresponding to the substance that inhibits the structure that causes food additives intolerance;
(4) the stable structure corresponding to the substance containing a substance of any one of (1) to (3) above.

107. A substance for treating and/or preventing food additives intolerance designed according to the method of any one of claims 105-106.

108. The preparation method of the substance for treating and/or preventing food additives intolerance according to claim 107, **characterized in that** the stable structure corresponding to the substance is prepared by a high-temperature carbonization method.

109. A method for designing substances for treating and/or preventing alcohol intolerance, **characterized in that**:
the stable structure corresponding to the substance related to the alcohol intolerance is used as the substance for treating and/or preventing said alcohol intolerance; and
the stable structure corresponding to the substance is **characterized by** the structure of said substance that exists in a solid state at a high temperature of 500 °C or higher.

110. The method according to claim 109, **characterized in that** said stable structure corresponding to the substance related to the intolerance disease is selected from one or more of the following:
(1) the stable structure corresponding to the substance that causes alcohol intolerance in the organism;
(2) the stable structure corresponding to the substance of the organism itself and related to alcohol intolerance;
(3) the stable structure corresponding to the substance that inhibits the structure that causes alcohol intolerance;
(4) the stable structure corresponding to the substance containing a substance of any one of (1) to (3) above.

111. A substance for treating and/or preventing alcohol intolerance designed according to the method of any one of claims 109-110.

112. The preparation method of the substance for treating and/or preventing alcohol intolerance according to claim 111, **characterized in that** the stable structure corresponding to the substance is prepared by a high-temperature carbonization method.

113. A method for designing substances for treating and/or preventing drug intolerance, **characterized in that**:
the stable structure corresponding to the substance related to the drug intolerance is used as the substance for treating and/or preventing said drug intolerance; and
the stable structure corresponding to the substance is **characterized by** the structure of said substance that exists in a solid state at a high temperature of 500 °C or higher.

114. The method according to claim 113, **characterized in that** said stable structure corresponding to the substance related to the intolerance disease is selected from one or more of the following:
(1) the stable structure corresponding to the substance that causes drug intolerance in the organism;
(2) the stable structure corresponding to the substance of the organism itself and related to drug intolerance;
(3) the stable structure corresponding to the substance that inhibits the structure that causes drug intolerance;
(4) the stable structure corresponding to the substance containing a substance of any one of (1) to (3) above.

115. A substance for treating and/or preventing drug intolerance designed according to the method of any one of claims 113-114.

116. The preparation method of the substance for treating and/or preventing drug intolerance according to claim 115, **characterized in that** the stable structure corresponding to the substance is prepared by a high-temperature carbonization method.

117. A method for designing substances for treating and/or preventing anti-infectives intolerance, **characterized in that**:
the stable structure corresponding to the substance related to the anti-infectives intolerance is used as the substance for treating and/or preventing said anti-infectives intolerance; and
the stable structure corresponding to the substance is **characterized by** the structure of said substance that exists in a solid state at a high temperature of 500 °C or higher.

118. The method according to claim 117, **characterized in that** said stable structure corresponding to the substance related to the intolerance disease is selected from one or more of the following:
(1) the stable structure corresponding to the substance that causes anti-infectives intolerance in the organism;
(2) the stable structure corresponding to the substance of the organism itself and related to anti-infectives intolerance;
(3) the stable structure corresponding to the substance that inhibits the structure that causes anti-infectives intolerance;
(4) the stable structure corresponding to the substance containing a substance of any one of (1) to (3) above.

119. A substance for treating and/or preventing anti-infectives intolerance designed according to the method of any one of claims 117-118.

120. The preparation method of the substance for treating and/or preventing anti-infectives intolerance according to claim 119, **characterized in that** the stable structure corresponding to the substance is prepared by a high-temperature carbonization method.

121. A method for designing substances for treating and/or preventing cardiovascular drugs intolerance, **characterized in that**:
the stable structure corresponding to the substance related to the cardiovascular drugs intolerance is used as the substance for treating and/or preventing said cardiovascular drugs intolerance; and
the stable structure corresponding to the substance is **characterized by** the structure of said substance that exists in a solid state at a high temperature of 500 °C or higher.

122. The method according to claim 121, **characterized in that** said stable structure corresponding to the substance related to the intolerance disease is selected from one or more of the following:
(1) the stable structure corresponding to the substance that causes cardiovascular drugs intolerance in the organism;
(2) the stable structure corresponding to the substance of the organism itself and related to cardiovascular drugs intolerance;
(3) the stable structure corresponding to the substance that inhibits the structure that causes cardiovascular drugs intolerance;
(4) the stable structure corresponding to the substance containing a substance of any one of (1) to (3) above.

123. A substance for treating and/or preventing cardiovascular drugs intolerance designed according to the method of any one of claims 121-122.

124. The preparation method of the substance for treating and/or preventing cardiovascular drugs intolerance according to claim 123, **characterized in that** the stable structure corresponding to the substance is prepared by a high-temperature carbonization method.

125. A method for designing substances for treating and/or preventing skin intolerance, **characterized in that**:
the stable structure corresponding to the substance related to the skin intolerance is used as the substance for treating and/or preventing said skin intolerance; and
the stable structure corresponding to the substance is **characterized by** the structure of said substance that exists in a solid state at a high temperature of 500 °C or higher.

126. The method according to claim 125, **characterized in that** said stable structure corresponding to the substance related to the intolerance disease is selected from one or more of the following:
(1) the stable structure corresponding to the substance that causes skin intolerance in the organism;
(2) the stable structure corresponding to the substance of the organism itself and related to skin intolerance;
(3) the stable structure corresponding to the substance that inhibits the structure that causes skin intolerance;
(4) the stable structure corresponding to the substance containing a substance of any one of (1) to (3) above.

127. A substance for treating and/or preventing skin intolerance designed according to the method of any one of claims 125-126.

128. The preparation method of the substance for treating and/or preventing skin intolerance according to claim 127, **characterized in that** the stable structure corresponding to the substance is prepared by a high-temperature carbonization method.

129. Use of the substance according to any one of claims 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123 and 127 in the preparation of medicines, health products, foods, and food additives.
